(19) Europäisches Patentamt
European Patent Office
Office européen des brevets

(11) EP 3 054 010 B1

(12) **EUROPEAN PATENT SPECIFICATION**

(45) Date of publication and mention of the grant of the patent:
**22.07.2020 Bulletin 2020/30**

(21) Application number: **14850688.4**

(22) Date of filing: **03.10.2014**

(51) Int Cl.:
*A61K 39/00* (2006.01) *A61K 35/17* (2015.01)
*C07K 7/06* (2006.01) *C07K 7/08* (2006.01)
*A61K 38/08* (2019.01) *A61P 35/00* (2006.01)

(86) International application number:
**PCT/JP2014/076625**

(87) International publication number:
**WO 2015/050259 (09.04.2015 Gazette 2015/14)**

(54) **TUMOR ANTIGEN PEPTIDE**

TUMORANTIGENPEPTID

PEPTIDE D'ANTIGÈNE TUMORAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2013 JP 2013208645**

(43) Date of publication of application:
**10.08.2016 Bulletin 2016/32**

(73) Proprietor: **Sapporo Medical University**
**Sapporo-shi, Hokkaido 060-8556 (JP)**

(72) Inventors:
- **SATO, Noriyuki**
  **Sapporo-shi**
  **Hokkaido 060-8556 (JP)**
- **TORIGOE, Toshihiko**
  **Sapporo-shi**
  **Hokkaido 060-8556 (JP)**
- **HIROHASHI, Yoshihiko**
  **Sapporo-shi**
  **Hokkaido 060-8556 (JP)**
- **KANASEKI, Takayuki**
  **Sapporo-shi**
  **Hokkaido 060-8556 (JP)**
- **KOCHIN, Vitaly**
  **Sapporo-shi**
  **Hokkaido 060-8556 (JP)**
- **TAKAYA, Akari**
  **Sapporo-shi**
  **Hokkaido 060-8556 (JP)**
- **GOTO, Masashi**
  **Osaka-shi**
  **Osaka 554-0022 (JP)**

(74) Representative: **Zech, Stefan Markus**
**Meissner Bolte Patentanwälte**
**Rechtsanwälte Partnerschaft mbB**
**Postfach 86 06 24**
**81633 München (DE)**

(56) References cited:
**WO-A1-2010/050190**
**WO-A2-01/57278**
**WO-A2-03/087768**
**JP-A- H07 509 319**
**JP-A- 2006 511 196**
**US-A1- 2003 219 741**

- **RENA MORITA ET AL.: 'Daichogan ni Okeru Gan Kansaibo to Shinki Gan Kogen no Bunshi Byorigakuteki Kaiseki' NIPPON BYORI GAKKAI KAISHI vol. 99, no. 1, 2010, page 246, XP008183299**
- **RENA MORITA ET AL.: 'Daichogan Kansaibo to Shinki Gan Kogen OR7C1 no Bunshi Byorigakuteki Kaiseki' NIPPON BYORI GAKKAI KAISHI vol. 101, no. 1, 2012, page 349, XP008183265**
- **YOSHIHIKO HIROHASHI ET AL.: 'Gan Kansaibo o Hyoteki to shita Men'eki Ryoho no Kisoteki Kento' NIPPON BYORI GAKKAI KAISHI vol. 99, no. 1, 2010, page 187, XP008183264**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 054 010 B1

- **ROCKY CIPRIANO ET AL.: 'FAM83B-mediated activation of PI3K/AKT and MAPK signaling cooperates to promote epithelial cell transformation and resistance to targeted therapies' ONCOTARGET vol. 4, no. 5, May 2013, pages 728 - 738, XP055331765**
- **AKARI TAKAHASHI ET AL.: 'Identification of the naturally processed peptide derived from FAM83B that is specifically expressd in human cancer initiating cells' PROCEEDINGS OF THE JAPANESE SOCIETY FOR IMMUNOLOGY vol. 42, 18 November 2013, page 74, XP008183597**

## Description

[Technical Field]

The present disclosure relates to a detecting agent for detecting a cancer stem cell utilizing a gene that is specifically expressed in a cancer stem cell, a tumor antigen peptide derived from the gene that is useful as an agent for the prevention and/or treatment of a cancer, and the use thereof. Furthermore, the present disclosure relates to a method for screening such a tumor antigen peptide. Moreover, the present disclosure also relates to a method for identifying a natural peptide that is subjected to antigen presentation on a cell.

[Background Art]

The therapeutic effect of anti cancer agents that have been developed so far is not sufficient and the probability of curing a cancer is very low. As a cause thereof, the inability of conventional therapeutic agents to selectively target cells that form the basis of cancer tissue can be cited. In recent years, as such 'cells forming the basis of cancer tissue' the presence of cancer stem cells has been reported. Cancer stem cells are thought to be causal cells involved in the occurrence, recurrence, and metastasis of a cancer and, therefore, if cancer stem cells can be targeted, it can be expected that the possibility of suppressing effectively the proliferation, recurrence, and metastasis of a cancer will be high. That is, the development of a technique for detecting cancer stem cells and a novel therapeutic agent that targets cancer stem cells are important issues in cancer medicine.

On the other hand, in the elimination of tumor cells, virus-infected cells, etc. in a living body, cell-mediated immunity, in particular involving cytotoxic T cells (CTLs), plays an important role. In the case of the elimination of tumor cells, a CTL recognizes a complex of an antigen peptide (tumor antigen peptide) and a major histocompatibility complex (MHC: Major Histocompatibility Complex) class I antigen (called an HLA class I antigen in the case of humans) on a tumor cell and attacks and destroys the tumor cell. That is, a tumor antigen peptide is produced by intracellular degradation by a protease of a tumor-specific protein, that is, a tumor antigen protein, after it has been synthesized in the cell. The tumor antigen peptide thus produced binds to an MHC class I antigen (HLA class I antigen) in the endoplasmic reticulum to form a complex, which is transported to the cell surface and is presented as an antigen. A tumor-specific CTL recognizes the complex involved in this antigen presentation, and an anti-tumor effect is exhibited via cytotoxic action, lymphokine production, etc. Accompanying the elucidation of such a series of actions, therapies in which a tumor antigen protein or a tumor antigen peptide is utilized as a so-called cancer immunotherapy agent (cancer vaccine) to thus enhance cancer-specific CTLs in the body of a cancer patient are in the process of being developed.

Among them, the development of a novel cancer vaccine that can immunologically eliminate cancer stem cells has been particularly desired (e.g. Patent Document 1).

It is known that the expression of FAM83B (family with sequence similarity 83, member B) is greatly increased in a cancerous part such as a breast cancer, cervical cancer, lung cancer, thyroid cancer, colon cancer, testicular tumor, or ovarian cancer compared with each of the non-cancerous parts. A human mammary-derived epithelial cell into which FAM83B has been artificially introduced forms a cancer in an immunodeficient mouse. Furthermore, when the expression of FAM83B is suppressed in breast cancer cells, cell proliferation is suppressed both in vitro and in vivo. From the above, it has been pointed out that FAM83B is an oncogene that is intimately involved in the occurrence, formation, and proliferation of a cancer in breast cancer, cervical cancer, lung cancer, thyroid cancer, colon cancer, testicular tumor, ovarian cancer, etc. (e.g. Non-Patent Documents 1 to 5).

[Prior Art Documents]

[Patent Documents]

[Patent Document 1] International Patent Application WO2010/050268

[Non-Patent Documents]

[Non-Patent Document 1] R. Cipriano, et al., J. Clin. Invest. 2012, 122 (9), 3197-3210
[Non-Patent Document 2] M. Hilger, et al., J. Proteome Res. 2012, 11, 982-994
[Non-Patent Document 3] R. Cipriano, et al., Oncotarget 2013, 4, 728-738
[Non-Patent Document 4] R. Cipriano, et al., Oncogene 2013, 1-9
[Non-Patent Document 5] S. Grant, et al., J. Clin. Invest. 2012, 122 (9), 3048-3051

[Summary of the Invention]

While searching for a peptide that is specifically subjected to antigen presentation on a tumor cell, and in particular a cancer stem cell, even if a plurality of epitope regions that are predicted to bind to an HLA exist in the sequence of a protein specifically expressed in the cancer stem cell, it is not easy to identify which portion of the protein actually binds to an HLA in a living body and is subjected to antigen presentation on the cell surface. Therefore, in order to solve such problems, a method is hereby disclosed for directly identifying a peptide that is actually presented as an antigen on a cancer stem cell (natural peptide) and have identified several natural peptides. It has been found that among such peptides, a peptide that is specifically presented as an antigen only on a cancer stem cell is a peptide derived from a FAM83B protein, and as a result of further intensive investigation the present invention has been accomplished.

The invention is defined by the appended claims. That is, the present invention relates to the following:

[1] A FAM83B peptide consisting of amino acid sequences SEQ ID Nos: 3, 4, 6, 9, 12, 14, 15, 20, 23, 24, 29-33, 35, 37, 38, 47, 57, 60, 65-68, 71-75 or 77-80.

[2] An HLA multimer comprising an HLA and any one of the FAM83B peptides according to [1].

[3] A T cell receptor-like antibody that recognizes a complex of an HLA and any one of the FAM83B peptides according to [1].

[4] A chimeric antigen receptor that recognizes a complex of an HLA and any one of the FAM83B peptides according to [1].

[5] A genetically modified T cell characterized in that the genetic modification is due to the introduction of the chimeric antigen receptor according to [4] into a T cell.

[6] An artificial CTL comprising a T cell receptor that recognizes a complex of an HLA and any one of the FAM83B peptides according to [1].

[7] A polynucleotide encoding at least one of the FAM83B peptides according to [1].

[8] A pharmaceutical composition for use in the prevention and/or treatment of a cancer comprising as an active ingredient any of (a) to (e) below:

(a) at least one of the FAM83B peptides according to [1],
(b) the T-cell receptor-like antibody according to [3],
(c) the genetically modified T cell according to [5],
(d) the artificial CTL according to [6], and
(e) the polynucleotide according to [7].

[9] The pharmaceutical composition for use according to [8], the pharmaceutical composition comprising as the active ingredient any of (a) to (e), and further comprising an adjuvant.

[Brief Description of Drawings]

[FIG. 1] FIG. 1 shows the result of flow cytometry of a human colon cancer cell line (SW480) stained with Hoechst 33342/PI in the presence or absence of verapamil (Verapamil).

[FIG. 2-1] FIG. 2-1 shows the result of flow cytometry of cultures of single cell isolated from a SW480-SP clone cell line and a SW480-MP clone cell line stained with Hoechst 33342/PI.

[FIG. 2-2] FIG. 2-2 shows a confocal microscopy image of the SW480-SP clone cell line and the SW480-MP clone cell line.

[FIG. 3] FIG. 3 shows the result of evaluation of a tumor formed when each of the SW480-SP clone cell line and the SW480-MP clone cell line was transplanted into a mouse.

[FIG. 4] FIG. 4 shows the result of sequence analysis using mass spectrometry of a peptide isolated from a complex of an HLA and the peptide immunoprecipitated using an anti-HLA-A24 antibody from a lysate of the SW480-SP clone cell line and the SW480-MP clone cell line.

[FIG. 5] FIG. 5 shows a photograph of electrophoresis when extracting mRNA of the SW480-SP and the SW480-MP and examining gene expression by means of RT-PCR. FAM83B gene was confirmed as a gene specific to the SW480-SP clone cell line.

[FIG. 6] FIG. 6 shows the result of RT-PCR using mRNA of FAM83B derived from human adult normal tissue.

[FIG. 7] FIG. 7 shows the result of RT-PCR using mRNA of FAM83B derived from various cancer cell lines.

[FIG. 8] FIG. 8 shows the result of an ELISPOT assay of T2-A24 cells pulsed with various peptides using an ELISPOT plate coated with IFN-$\gamma$.

[FIG. 9] FIG. 9 shows the result of evaluation of cytotoxicity toward T2-A24 cells pulsed with various peptides of effector cells (CTL) induced from PBMC. The effector cell used was one induced by means of the FAM83B peptide represented by SEQ ID No: 3.

[FIG. 10] FIG. 10 shows the results of evaluation of in vitro CTL inducibility of the peptide represented by SEQ ID No: 4 (F23_10) by means of an interferon-γ ELISPOT assay. The ordinate denotes the number of spots per given number of seeded cells. A denotes the result of a test using an HLA-A*02:01 transgenic mouse, and B denotes the result of a test using an HLA-A*24:02 transgenic mouse. The black bar ('w/Peptide') and the white bar ('w/o') show the results of restimulation culturing of peptide-treated mouse-derived spleen cells in the presence or absence of administered peptide respectively. That is, the differences in the figures between the black bar and the white bar denote the number of peptide-specific CTLs induced in the mouse living body by administration of each of the peptides.

[FIG. 11] FIG. 11 shows the results of evaluation of in vitro CTL inducibility of the peptide represented by SEQ ID No: 65 (F62_10) by means of an interferon-γ ELISPOT assay. The ordinate, A, B, black bar and white bar are the same as those in FIG. 10.

[FIG. 12] FIG. 12 shows the result of evaluation of in vitro CTL inducibility of the peptide represented by SEQ ID No: 66 (F148_9) by means of an interferon-γ ELISPOT assay. The ordinate, A, B, black bar and white bar are the same as those in FIG. 10.

[FIG. 13] FIG. 13 shows the result of evaluation of in vitro CTL inducibility of the peptide represented by SEQ ID No: 67 (F239_12) by means of an interferon-γ ELISPOT assay. The ordinate, A, B, black bar and white bar are the same as those in FIG. 10.

[FIG. 14] FIG. 14 shows the result of evaluation of in vitro CTL inducibility of the peptide represented by SEQ ID No: 68 (F305_12) by means of an interferon-γ ELISPOT assay. The ordinate, A, B, black bar and white bar are the same as those in FIG. 10.

[FIG. 15] FIG. 15 shows the result of evaluation of in vitro CTL inducibility of the peptide represented by SEQ ID No: 20 (F475_10) by means of an interferon-γ ELISPOT assay. The ordinate, A, B, black bar and white bar are the same as those in FIG. 10.

[FIG. 16] FIG. 16 shows the result of evaluation of in vitro CTL inducibility of the peptide represented by SEQ ID No: 47 (F476_9) by means of an interferon-γ ELISPOT assay. The ordinate, A, B, black bar and white bar are the same as those in FIG. 10.

[FIG. 17] FIG. 17 shows the result of evaluation of in vitro CTL inducibility of the peptide represented by SEQ ID No: 35 (F131_9) and the peptide represented by SEQ ID No: 12 (F245_11) by means of an interferon-γ ELISPOT assay. The ordinate, black bar and white bar are the same as those in FIG. 10. A2 denotes the test result using an HLA-A*02:01 transgenic mouse, and A24 denotes the test result using an HLA-A*24:02 transgenic mouse.

[FIG. 18] FIG. 18 shows the result of evaluation of in vitro CTL inducibility of a peptide that exhibited high affinity toward an HLA-A02 by means of an interferon-γ ELISPOT assay using an HLA-A*02:01 transgenic mouse. The ordinate, black bar and white bar are the same as those in FIG. 10.

[FIG. 19] FIG. 18 shows the result of evaluation of in vitro CTL inducibility of a peptide that exhibited high affinity toward an HLA-A24 by means of an interferon-γ ELISPOT assay using an HLA-A*24:02 transgenic mouse. The ordinate, black bar and white bar are the same as those in FIG. 10.

[FIG. 20] FIG. 20 shows the result of an HLA-A24 tetramer assay. FIG. 20-1 shows the result of patient A, and FIG. 20-2 shows the result of patient B. A) mainly shows a lymphocyte gate, and B) shows a CD8 positive T cell gate in A). C) is a diagram showing the result of a tetramer assay and shows the result of tetramer staining in B).

[Modes for Carrying Out the Invention]

The present invention is explained in detail below.

The 'epitope peptide' referred to in the present invention means a peptide that binds to an MHC (an HLA for humans) and is subjected to antigen presentation on the cell surface and has antigenicity (can be recognized by a T cell). The epitope peptide includes a CTL epitope peptide that binds to an MHC class I, is subjected to antigen presentation, and is recognized by a CD8-positive T cell, and a helper epitope peptide that binds to an MHC class II, is subjected to antigen presentation, and is recognized by a CD4-positive T cell.

Among epitope peptides, a protein-derived peptide that is specifically or over expressed in a tumor cell is in particular called a tumor antigen peptide. The antigen presentation referred to a phenomenon in which a peptide present within a cell binds to an MHC and this MHC/antigen peptide complex is localized on the cell surface. As described above, it is known that an antigen presented on a cell surface is recognized by a T cell, etc. and then activates cell-mediated immunity or humoral immunity; since an antigen presented by an MHC class I activates cell-mediated immunity and is also recognized by a T cell receptor of a naive T cell to thus induce the naive T cell to become a CTL having cytotoxic activity, a tumor antigen peptide used in immunotherapy is preferably a peptide that binds to an MHC class I and is subjected to antigen presentation.

In the present invention, a 'tumor' includes a benign tumor and a malignant tumor (cancer, malignant neoplasm). A cancer includes a hematopoietic tumor, an epithelial malignant tumor (carcinoma), and a nonepithelial malignant tumor (sarcoma). In the present invention, a 'cancer stem cell' means a cell, among cells present in cancerous tissue, that exhibits stem cell-like properties, and is a cell that is thought to be a causal cell involved in the occurrence, recurrence, and metastasis of a cancer. In general, since only a small amount of 'cancer stem cells' are present in cancerous tissue, it is difficult to distinguish them from other cells, but in the present technical field methods for isolating/concentrating cancer stem cells are known, examples thereof including an SP fractionation method. Therefore, in the present invention, a 'cancer stem cell' can mean a cell population that has been isolated/concentrated by a known cancer stem cell isolation/concentration method.

(1) Method for identifying natural peptide

One aspect of the present disclosure relates to a method for isolating/identifying a natural peptide that is actually subjected to antigen presentation on a cell surface.

In the present invention, a 'natural peptide' means a peptide that is actually subjected to antigen presentation on a cell surface. Furthermore, a 'natural antigen peptide' is a natural peptide that is confirmed to have antigenicity. By isolating this natural antigen peptide from a cancer cell and determining the sequence and the origin thereof, it is possible to obtain useful findings for the targeted therapy of a cancer using CTLs.

This method includes a step of lysing a cell presenting a natural peptide and isolating a complex of an MHC and the natural peptide from the lysate.

A cell used in this method is not particularly limited as long as it is a cell that presents a natural peptide, that is, a cell that expresses an MHC, but is preferably a tumor cell, more preferably a cancer tissue-derived tumor cell, and yet more preferably a cancer stem cell. In particular, when a cancer stem cell is used, since a natural antigen peptide that is actually subjected to antigen presentation on a cancer stem cell can be identified, it becomes possible to reliably obtain a tumor antigen peptide that can induce a CTL targeting the cancer stem cell, which is very useful.

A method for isolating a complex of an MHC and a natural peptide is not particularly limited, and a protein isolation method known in the present technical field may be used. Specifically, although not limited thereto, since it is necessary to select only a peptide that binds to an MHC among the abundance of peptides present in the cell, a method of extracting a peptide/MHC complex by an immunoprecipitation method using an antibody specific to the MHC is preferable.

An appropriate anti-MHC antibody can vary according to the cell used, and a person skilled in the art can select the optimal one according to the purpose and experimental conditions. Specifically, for example, when a human cell is used, an anti-HLA antibody is used as an anti-MHC antibody. Examples of the anti-HLA antibody include, but are not limited to, an antibody against an HLA class I such as an anti-HLA-A02 antibody or an anti-HLA-A24 antibody.

This method further includes a step of isolating a natural peptide from the complex of an MHC and the natural peptide. A method of separating a complex into an MHC molecule and a natural peptide is not particularly limited as long as it does not change the structure of the natural peptide, and a person skilled in the art can select an appropriate method. Specific examples include, but are not limited to, peptide isolation using a weak acid.

Furthermore, the method includes a step of analyzing the sequence of the isolated natural peptide. Methods of analyzing the sequence of a short-chain peptide are known in the present technical field, and a person skilled in the art can select an appropriate method. Specific examples include, but are not limited to, a peptide sequence analysis method in which liquid chromatography and tandem mass spectrometry are combined.

A natural peptide that is actually subjected to antigen presentation on a cell surface can be identified by the above steps.

One further embodiment further includes a step of confirming the antigenicity of a natural peptide. Methods of confirming the antigenicity of a peptide are known in the present technical field, and a person skilled in the art can select an appropriate method. Specific examples include, but are not limited to, a cytotoxic test, an ELISPOT assay, and an assay using a TCR-like antibody. Confirmation of the antigenicity of a peptide is preferably carried out after the sequence of a natural peptide has been identified but may be carried out before identification or may be carried out independently from identification of a natural peptide. By further confirming by such a step the antigenicity of a natural peptide that has been identified by the method, the natural peptide can be identified as being a natural antigen peptide.

The present inventors have analyzed a natural antigen peptide that is subjected to antigen presentation on a human cancer stem cell by the above method. As a result, a FAM83B protein-derived peptide (SEQ ID No: 3) has been identified as a natural antigen peptide that is subjected to antigen presentation on a cancer stem cell. As a result of further progressing research based on such a finding, it has been found that the FAM83B gene is highly expressed specifically in cancer stem cells and is a useful candidate gene for molecularly targeted therapy of cancer stem cells. The finding that FAM83B is a tumor antigen and, furthermore, the finding that a FAM83B-derived peptide binds to an HLA class I antigen to form a complex on a tumor cell surface and is transported to the cell surface and subjected to antigen presentation are new findings that were hitherto completely unknown.

(2) The peptide of the present invention

In the present specification, a ’human FAM83B protein’ means a known protein reported in J Proteome Res. 2012; 11: 982-94 and J Clin Invest. 2012; 122: 3197-210, and it specifically means a protein having an amino acid sequence described in SEQ ID No: 2 (Genbank Accession No: NP_001010872.1) and a homolog thereof. Examples of the homolog include a splicing variant and a variant such as an SNP based on individual difference. Specific examples include (1) a protein with an amino acid sequence that has a homology of at least 90% with the amino acid sequence represented by SEQ ID No: 2, preferably at least 95%, and more preferably at least 98% and (2) a protein with an amino acid sequence for which one or more amino acids, preferably one to several, and more preferably 1 to 10, 1 to 5, 1 to 3, or 1 or 2 amino acids have been replaced, deleted, added, or inserted in the amino acid sequence described in SEQ ID No: 2. Examples of such a variant include a protein of Genbank Accession No.: BC112275 with an amino acid sequence for which the 640th amino acid in SEQ ID No: 2 has been replaced by threonine (T) and the 907th amino acid has been replaced by asparagine (N). When simply ’FAM83B protein’ is referred to in the present specification, it means a human FAM83B protein unless otherwise specified.

Preferred examples of the human FAM83B protein include a protein including with the amino acid sequence described in SEQ ID No: 2 and a protein with an amino acid sequence for which 1 to 3, and preferably 1 or 2 amino acids have been replaced in the protein. A protein with the amino acid sequence described in SEQ ID No: 2 can be cited as a yet more preferred example.

The present invention concerns specific human FAM83B protein partial peptides as specified in claim 1, the peptides binding to an MHC, and in particular to an HLA; it is preferably a peptide that is subjected to antigen presentation by means of an MHC, in particular an HLA, and more preferably a peptide that is subjected to antigen presentation by means of an MHC, in particular an HLA, and can induce a CTL. There are several types of HLA; the peptide of the present invention preferably can bind to an HLA class I, more preferably can bind to HLA-A02 or HLA-A24, and yet more preferably can bind to both HLA-A02 and HLA-A24. The peptide of the present invention may be subjected to a treatment such as processing prior to binding to an MHC, and a peptide that forms an epitope peptide as a result of such a treatment is also included in the peptide of the present invention. Therefore, the amino acid length of the peptide of the present invention is not particularly limited as long as it is a sequence including an amino acid sequence according to SEQ ID Nos: 3, 4, 6, 9, 12, 14, 15, 20, 23, 24, 29-33, 35, 37, 38, 47, 57, 60, 65-68, 71-75 and 77-80.. However, it is preferable that the peptide of the present invention itself is an epitope peptide, and therefore the amino acid length is preferably on the order of 9 to 11 amino acids.

An epitope peptide that binds to an HLA class I, which is a human MHC class I, has a length of about 8 to 14 amino acids, and preferably a length of about 9 to 11 amino acids, and is known to have an HLA-specific binding motif in the sequence. For example, a peptide binding to HLA-A02 has a binding motif in which the second amino acid from the N terminal is leucine, isoleucine, or methionine and/or the amino acid at the C terminal is valine, leucine, or isoleucine, and a peptide binding to HLA-A24 has a binding motif in which the second amino acid from the N terminal is tyrosine, phenylalanine, methionine, or tryptophan and/or the amino acid at the C terminal is leucine, isoleucine, or phenylalanine.

Therefore, the peptide of the present invention includes an epitope peptide that is a partial peptide of the FAM83B protein with 8 to 13 consecutive amino acids in the amino acid sequence of said protein as indicated in claim 1, the second amino acid from the N terminal being leucine, isoleucine, or methionine and/or the amino acid at the C terminal being valine, leucine, or isoleucine, and more preferably is the epitope peptide itself. Among them, an epitope peptide with an amino acid sequence represented by any of SEQ ID Nos: 29 to 33, 35, 37, 38, 47, 57, 60, and 71 is particularly preferable.

Furthermore, in another preferred embodiment, the partial peptide includes an epitope peptide as indicated in claim 1 having the second amino acid from the N terminal replaced by leucine, isoleucine, or methionine and/or the amino acid at the C terminal replaced by valine, leucine, or isoleucine, and more preferably is the epitope peptide itself. Among them, an epitope peptide with an amino acid sequence represented by any of SEQ ID Nos: 29 to 33, 35, 37, 38, 47, 57, 60 and 71, the second amino acid from the N terminal being replaced by leucine, isoleucine, or methionine and/or the amino acid at the C terminal being replaced by valine, leucine, or isoleucine is particularly preferable.

In another preferred embodiment, the peptide of the present invention includes an epitope peptide that is a partial peptide of the FAM83B protein with 8 to 14 consecutive amino acids in the amino acid sequence of said protein as indicated in claim 1, the second amino acid from the N terminal being tyrosine, phenylalanine, methionine, or tryptophan and/or the amino acid at the C terminal being leucine, isoleucine, or phenylalanine, and more preferably is the epitope peptide itself. Among them, an epitope peptide with an amino acid sequence represented by any of SEQ ID Nos: 3, 4, 6, 9, 12, 14, 15, 20, 23, 24, 67, and 78 is particularly preferable.

Furthermore, in another preferred embodiment, the partial peptide includes an epitope peptide as indicated in claim 1, the second amino acid from the N terminal being replaced by tyrosine, phenylalanine, methionine, or tryptophan and/or the amino acid at the C terminal being replaced by leucine, isoleucine, or phenylalanine, and more preferably is the epitope peptide itself. Among them, an epitope peptide with an amino acid sequence represented by any of SEQ ID Nos: 3, 4, 6, 9, 12, 14, 15, 20, 23, 24, 67, and 78, the second amino acid from the N terminal being replaced by tyrosine, phenylalanine, methionine, or tryptophan and/or the amino acid at the C terminal being replaced by leucine, isoleucine, or phenylalanine is particularly preferable.

With regard to the peptide of the present invention, in a preferred embodiment, the peptide has the same amino acid sequence as the amino acid sequence described in any of SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 71, SEQ ID No: 72, SEQ ID No: 73, SEQ ID No: 74, SEQ ID No: 75, SEQ ID No: 77, SEQ ID No: 78, and SEQ ID No: 80. In this embodiment, the peptides of the present invention are partial peptides of the FAM83B protein. In such a more preferred embodiment, examples of the peptide of the present invention include a peptide with the same amino acid sequence as the amino acid sequence described in any of SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 67, SEQ ID No: 68, SEQ ID No: 71, SEQ ID No: 72, SEQ ID No: 73, SEQ ID No: 74, SEQ ID No: 75, SEQ ID No: 77, SEQ ID No: 78, SEQ ID No: 79, and SEQ ID No: 80.

The peptides represented by SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 67, SEQ ID No: 68, SEQ ID No: 71, SEQ ID No: 72, SEQ ID No: 73, SEQ ID No: 74, SEQ ID No: 75, SEQ ID No: 77, SEQ ID No: 78, SEQ ID No: 79, and SEQ ID No: 80 are peptides with the 10 amino acids at amino acid positions 23 to 32 of the above FAM83B, the 10 amino acids at 107 to 116, the 10 amino acids at 176 to 185, the 11 amino acids at 245 to 255, the 11 amino acids at 276 to 286, the 9 amino acids at 305 to 313, the 10 amino acids at 475 to 484, the 9 amino acids at 939 to 947, the 9 amino acids at 971 to 979, the 11 amino acids at 55 to 65, the 9 amino acids at 63 to 71, the 10 amino acids at 86 to 95, the 9 amino acids at 103 to 111, the 11 amino acids at 114 to 124, the 9 amino acids at 131 to 139, the 10 amino acids at 151 to 160, the 11 amino acids at 179 to 189, the 9 amino acids at 476 to 484, the 10 amino acids at 799 to 808, the 11 amino acids at 256 to 266, the 10 amino acids at 62 to 71, the 9 amino acids at 148 to 156, the 12 amino acids at 239 to 250, the 12 amino acids at 305 to 316, the 10 amino acids at 55 to 64, the 10 amino acids at 231 to 240, the 10 amino acids at 257 to 266, the 10 amino acids at 18 to 27, the 10 amino acids at 47 to 56, the 10 amino acids at 149 to 158, the 11 amino acids at 153 to 163, the 13 amino acids at 238 to 250, and the 10 amino acids at 239 to 248 respectively, and the present inventors have found that all of the peptides being capable of binding to HLA-A02 and/or HLA-A24 and having CTL inducibility.

In a yet more preferred embodiment, the peptide of the present invention has the same amino acid sequence as the amino acid sequence described in SEQ ID No: 4, SEQ ID No: 15, SEQ ID No: 20, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 71, SEQ ID No: 72, SEQ ID No: 73, and SEQ ID No: 80. In this embodiment, the peptides of the present invention are partial peptides of the FAM83B protein. In such a more preferred embodiment, examples of the peptide of the present invention include a peptide with the same amino acid sequence as the amino acid sequence described in SEQ ID No: 4, SEQ ID No: 20, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 67, SEQ ID No: 68, SEQ ID No: 71, SEQ ID No: 72, and SEQ ID No: 73.

The peptides represented by SEQ ID No: 4, SEQ ID No: 20, SEQ ID No: 29, SEQ ID No: 30, SEQ ID No: 31, SEQ ID No: 32, SEQ ID No: 33, SEQ ID No: 35, SEQ ID No: 37, SEQ ID No: 38, SEQ ID No: 47, SEQ ID No: 57, SEQ ID No: 60, SEQ ID No: 65, SEQ ID No: 66, SEQ ID No: 67, SEQ ID No: 68, SEQ ID No: 71, SEQ ID No: 72, and SEQ ID No: 73 are peptides with the 10 amino acids at amino acid positions 23 to 32 of the above FAM83B, the 10 amino acids at 475 to 484, the 11 amino acids at 55 to 65, the 9 amino acids at 63 to 71, the 10 amino acids at 86 to 95, the 9 amino acids at 103 to 111, the 11 amino acids at 114 to 124, the 9 amino acids at 131 to 139, the 10 amino acids at 151 to 160, the 11 amino acids at 179 to 189, the 9 amino acids at 476 to 484, the 10 amino acids at 799 to 808, the 11 amino acids at 256 to 266, the 10 amino acids at 62 to 71, the 9 amino acids at 148 to 156, the 12 amino acids at 239 to 250, the 12 amino acids at 305 to 316, the 10 amino acids at 55 to 64, the 10 amino acids at 231 to 240, and the 10 amino acids at 257 to 266 respectively, and the present inventors have found that all of the peptides being capable of binding to HLA-A02 and having CTL inducibility.

In another yet more preferred embodiment, the peptide of the present invention has the same amino acid sequence as the amino acid sequence described in SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 47, SEQ ID No: 65, SEQ ID No: 66, SEQ ID No: 74, SEQ ID No: 75, SEQ ID No: 77, SEQ ID No: 78, and SEQ ID No: 80. In this embodiment, all of the peptides of the present invention are partial peptides of the FAM83B protein. In such a more preferred embodiment, examples of the peptide of the present invention includes peptides with the same amino acid sequence as the amino acid sequence described in SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 47, SEQ ID No: 65, SEQ ID No: 66, SEQ ID No: 67, SEQ ID No: 68, SEQ ID No: 74, SEQ ID No: 75, SEQ ID No: 77, SEQ ID No: 78, SEQ ID No: 79, and SEQ ID No: 80.

The peptides represented by SEQ ID No: 4, SEQ ID No: 6, SEQ ID No: 9, SEQ ID No: 12, SEQ ID No: 14, SEQ ID No: 15, SEQ ID No: 20, SEQ ID No: 23, SEQ ID No: 24, SEQ ID No: 47, SEQ ID No: 65, SEQ ID No: 66, SEQ ID No: 67, SEQ ID No: 68, SEQ ID No: 74, SEQ ID No: 75, SEQ ID No: 77, SEQ ID No: 78, SEQ ID No: 79 and SEQ ID No: 80 are peptides with the 10 amino acids at amino acid positions 23 to 32 of the above FAM83B, the 10 amino acids at 107 to 116, the 10 amino acids at 176 to 185, the 11 amino acids at 245 to 255, the 11 amino acids at 276 to 286, the 9 amino acids at 305 to 313, the 10 amino acids at 475 to 484, the 9 amino acids at 939 to 947, the 9 amino acids at 971 to 979, the 9 amino acids at 476 to 484, the 10 amino acids at 62 to 71, the 9 amino acids at 148 to 156, the 12 amino acids at 239 to 250, the 12 amino acids at 305 to 316, the 10 amino acids at 18 to 27, the 10 amino acids at 47 to 56, the 10 amino acids at 149 to 158, the 11 amino acids at 153 to 163, the 13 amino acids at 238 to 250, and the 10 amino acids at 239 to 248 respectively, and the present inventors have found that all of the peptides being capable of binding to HLA-A024 and having CTL inducibility.

Furthermore, in another preferred embodiment, the inventive peptide is a peptide with the same amino acid sequence as the amino acid sequence described in SEQ ID No: 4, SEQ ID No: 15, SEQ ID No: 20, SEQ ID No: 47, SEQ ID No: 65, SEQ ID No: 66, and SEQ ID No: 80. In this embodiment, it is more preferable that all of the peptides of the present invention are partial peptides of the FAM83B protein. In such a more preferred embodiment, examples of the peptide of the present invention include peptides with the same amino acid sequence as the amino acid sequence described in SEQ ID No: 4, SEQ ID No: 20, SEQ ID No: 47, SEQ ID No: 65, SEQ ID No: 66, SEQ ID No: 67, and SEQ ID No: 68.

The peptides represented by SEQ ID No: 4, SEQ ID No: 20, SEQ ID No: 47, SEQ ID No: 65, SEQ ID No: 66, SEQ ID No: 67, and SEQ ID No: 68 are peptides with the 10 amino acids at amino acid positions 23 to 32 of the above FAM83B, the 10 amino acids at 475 to 484, the 9 amino acids at 476 to 484, the 10 amino acids at 62 to 71, the 9 amino acids at 148 to 156, the 12 amino acids at 239 to 250, and the 12 amino acids at 305 to 316 respectively, and the present inventors have found that all of the peptides being capable of binding to both HLA-A02 and HLA-A24 and having CTL inducibility.

Synthesis of the peptide of the present invention may be carried out in accordance with known methods used in normal peptide chemistry. Such known methods includes methods described in the literature (Peptide Synthesis, Interscience, New York, 1966; The Proteins, Vol. 2, Academic Press Inc., New York, 1976; Peptide Synthesis, Maruzen Co., Ltd., 1975; Basics and Experiments of Peptide Synthesis, Maruzen Co., Ltd., 1985; Development of Pharmaceuticals Seq. Vol. 14 Peptide Synthesis, Hirokawa Shoten Co., 1991, these publications forming part of the present application by reference), etc.

With regard to the peptide of the present invention, in vivo activity can be confirmed by subjecting it to a CTL induction method, which is described later, an assay using an animal model for human (WO02/47474, Int J. Cancer: 100, 565-570 (2002)), etc.

Disclosed is further a peptide in which a plurality of epitope peptides including at least one of the peptides of the present invention are linked (polyepitope peptide). Specific examples of this polyepitope peptide have CTL-inducing activity.

The polyepitope peptide may specifically be defined as

(i) a peptide in which the peptide of the present invention (epitope peptide) and any one or more CTL epitope peptides other than the peptide of the present invention are linked directly or via a spacer as appropriate,
(ii) a peptide in which the peptide of the present invention and any one or more helper epitope peptides are linked directly or via a spacer as appropriate, or
(iii) a peptide in which a polyepitope peptide described in (i) above and further one or more helper epitope peptides are linked directly or via a spacer as appropriate,
the peptide being subjected to processing within an antigen-presenting cell, and the epitope peptide thus formed being presented on the antigen-presenting cell, thus leading to CTL-inducing activity.

The CTL epitope peptide other than the peptide of the present invention in (i) is not particularly limited; specific examples include another human FAM83B-derived epitope peptide that is not included in the present invention and a human OR7C1- or human DNAJB8-derived epitope peptide (for example, a peptide described in International Patent Application No. WO2010/050190).

The spacer is not particularly limited as long as it does not adversely affect processing within an antigen-presenting cell, and is preferably a linker that is linked to each epitope peptide via a peptide bond, examples including a peptide linker in which several amino acids are linked and a linker having an amino group and a carboxyl group at each end. Specific examples include a glycine linker or a PEG (polyethylene glycol) linker; examples of the glycine linker include polyglycine (for example a peptide consisting of six glycines; Cancer Sci, Vol. 103, p. 150-153), and examples of the PEG linker include a linker derived from a compound having an amino group and a carboxy group at each end of PEG (for example, $H_2N-(CH_2)_2-(OCH_2CH_2)_3-COOH$; Angew. Chem. Int. Ed. 2008, 47, 7551-7556).

With regard to the epitope peptide of the present invention contained in the polyepitope peptide, one or more types may be selected. That is, a plurality of identical epitope peptides may be linked, or a plurality of different epitope peptides may be linked. Naturally, even when two or more types of epitope peptides are selected, a plurality of one or more types of selected epitope peptides may be linked. Similarly, with regard to the epitope peptide other than the peptide of the present invention, a plurality of types and/or a plurality of epitope peptides may be linked. The polyepitope peptide may be one in which 2 to 12 epitope peptides are linked, is preferably one in which 2, 3, 4, 5, 6, 7, 8, 9, 10, 11, or 12 epitope peptides are linked, and is most preferably one in which 2 epitope peptides are linked.

When the epitope peptide that is linked to the peptide of the present invention is a helper epitope peptide, examples of the helper epitope peptide used include hepatitis B virus-derived HBVc128-140 and tetanus toxin-derived TT947-967. The length of the helper epitope peptide is on the order of 13 to 30 amino acids, and preferably on the order of 13 to 17 amino acids.

Such a peptide in which a plurality of epitope peptides are linked (polyepitope peptide) may also be produced by a standard peptide synthesis method as described above. Furthermore, based on information regarding the sequence of a polynucleotide encoding such a polyepitope peptide in which a plurality of epitope peptides are linked, it may be produced using standard DNA synthesis and genetic engineering methods.

That is, said polynucleotide is inserted into a known expression vector, a host cell is transformed by means of the recombinant expression vector thus obtained to give a transformant, the transformant is cultured, and the target polyepitope peptide in which a plurality of epitopes are linked can be produced by recovery from the culture. These methods may be carried out in accordance with methods described in the literature as described above (Molecular Cloning, T. Maniatis et al., CSH Laboratory (1983), DNA Cloning, D M. Glover, IRL PRESS (1985)).

The polyepitope peptide thus produced in which a plurality of epitope peptides are linked is subjected to the above in vitro assay or an in vivo assay using an animal model for human described in WO02/47474 and Int J. Cancer: 100, 565-570 (2002) (these publications forming part of the present application by reference), etc., thus enabling CTL-inducing activity to be confirmed.

The peptide of the present invention is useful for the prevention and/or treatment of a cancer, etc. as described in the present specification, and may be an active ingredient of a pharmaceutical composition. Furthermore, the peptide of the present invention may be for the prevention and/or treatment of a cancer. Moreover, the present invention also relates to use of the peptide of the present invention in the production of a medicament for the prevention and/or treatment of a cancer.

(3) Polynucleotide of the present invention

The polynucleotide of the present invention includes a polynucleotide that encodes at least one of the FAM83B peptides of the present invention. The polynucleotide of the present invention may be any of cDNA, mRNA, cRNA, or synthetic DNA. It may have either a single-strand or a double-strand configuration. Specific examples include, but are not limited to, a polynucleotide with a nucleotide sequence encoding an amino acid sequence described in SEQ ID Nos: 3, 4, 6, 9, 12, 14, 15, 20, 23, 24, 29-33, 35, 37, 38, 47, 57, 60, 65-68, 71-75 and 77-80.

The polynucleotide of the present invention may take on either a single strand or a double strand configuration. When the polynucleotide of the present invention is a double strand, a recombinant expression vector expressing the peptide of the present invention may be produced by inserting the polynucleotide of the present invention into an expression vector. That is, the scope of the polynucleotide of the present invention includes a recombinant expression vector produced by inserting the double strand polynucleotide of the present invention into an expression vector.

The polynucleotide of the present invention is useful for the prevention and/or treatment of a cancer, etc. as described in the present specification, and may be an active ingredient of a pharmaceutical composition. Furthermore, the polynucleotide of the present invention may be for the prevention and/or treatment of a cancer. Moreover, the present invention also relates to use of the polynucleotide of the present invention in the production of a medicament for the prevention and/or treatment of a cancer.

With regard to the expression vector used in the present invention, various types may be used according to the host used, the intended application, etc., and a person skilled in the art may select it as appropriate. Examples of expression vectors that can be used in the present invention include a plasmid, a phage vector, and a virus vector. For example, when the host is Escherichia coli, examples of the vector include plasmid vectors such as pUC118, pUC119, pBR322, and pCR3 and phage vectors such as λZAPII and λgt11. When the host is a yeast, examples of the vector include pYES2 and pYEUra3. When the host is an insect cell, examples include pAcSGHisNT-A. When the host is an animal cell, examples include plasmid vectors such as pCEP4, pKCR, pCDM8, pGL2, pcDNA3.1, pRc/RSV, and pRc/CMV and virus vectors such as a retrovirus vector, an adenovirus vector, and an adeno-associated virus vector.

The vector may have as appropriate a factor such as a promoter capable of inducing expression, a gene encoding a signal sequence, a selection marker gene, or a terminator. Furthermore, in order to make isolation and purification easy, a sequence for expression as a fusion protein with thioredoxin, a His tag, GST (glutathione S-transferase), etc. may be added. In this case, a GST fusion protein vector (pGEX4T, etc.) having an appropriate promoter (lac, tac, trc, trp, CMV, SV40 early promoter, etc.) that functions within a host cell, a vector having a tag sequence such as Myc or His (pcDNA3.1/Myc-His, etc.) and, furthermore, a vector expressing a fusion protein with thioredoxin and a His tag (pET32a), etc. may be used.

Transforming a host with the expression vector prepared as above enables a transformed cell containing the expression vector to be prepared. Therefore, the present disclosure includes a gene transfer composition including the expression vector.

The host used for transformation may be any cell as long as the function of the polypeptide of the present invention is not impaired, and examples include an Escherichia coli, a yeast, an insect cell, and an animal cell. Examples of the Escherichia coli include E.coli K-12 strain HB101, C600, JM109, DH5α, and AD494 (DE3). Examples of the yeast include Saccharomyces cerevisiae. Examples of the animal cell include L929 cells, BALB/c3T3 cells, C127 cells, CHO cells, COS cells, Vero cells, Hela cells, and 293-EBNA cells. Examples of the insect cell include sf9.

As a method for introducing an expression vector into a host cell, a standard introduction method suitable for the host cell may be used. Specific examples include a calcium phosphate method, a DEAE-dextran method, an electroporation method, and a method using a lipid for gene transfer (Lipofectamine, Lipofectin; Gibco-BRL). After introduction, culturing is carried out in a standard medium containing a selection marker, thus enabling a transformed cell in which the expression vector has been introduced into the host cell to be selected.

Continuing culturing the transformed cell thus obtained under suitable conditions enables the peptide of the present invention to be produced. The peptide thus obtained may be further isolated and purified by usual biochemical purification means. Examples of purification means include salting out, ionexchange chromatography, adsorption chromatography, affinity chromatography, and gel filtration chromatography. When the peptide of the present invention is expressed as a fusion protein with a thioredoxin, a His tag, a GST, etc. as described above, isolation and purification may be carried out by a purification method utilizing the properties of the fusion protein or the tag.

The polynucleotide encoding the peptide of the present invention may have a DNA configuration or an RNA configuration. These polynucleotides of the present invention may be easily produced by standard methods known in the present technical field based on amino acid sequence information of the peptide of the present invention and DNA sequence information encoded thereby. Specifically, it may be produced by standard DNA synthesis, amplification by means of PCR, etc.

The polynucleotide encoding the peptide of the present invention includes a polynucleotide encoding the epitope peptide.

(4) CTL inducer/pharmaceutical composition comprising a peptide of the present invention as active ingredient,

The peptide of the present invention has CTL-inducing activity and can be a CTL inducer as a tumor antigen peptide. Furthermore, as described above, the present inventors have found for the first time that the FAM83B protein is a tumor antigen and a FAM83B protein-derived peptide binds to an HLA class I antigen, forms a complex on the tumor cell surface, is transported to the cell surface, and is subjected to antigen presentation. Therefore, the FAM83B protein itself can become a CTL inducer.

That is, peripheral blood lymphocytes are isolated from a person who is positive for an HLA-A02 antigen or an HLA-A24 antigen, they are stimulated in vitro by adding the peptide of the present invention and/or FAM83B protein, and CTLs that specifically recognize an HLA-A02 antigen-positive cell or an HLA-A24 antigen-positive cell that have been pulsed with the peptide can be induced (J. Immunol., 154, p. 2257, 1995). The presence or absence of CTL induction may be confirmed by measuring for example the amount of various cytokines (for example IFN-γ) produced by CTLs when reacting with an antigen peptide-presenting cell, by means of for example an ELISA method, etc. It may also be confirmed by a method for measuring CTL toxicity toward an antigen peptide-presenting cell labeled with $^{51}Cr$ ($^{51}Cr$ release assay, Int. J. Cancer, 58: p317, 1994).

Furthermore, a CTL clone may be established by a method described in Int. J. Cancer, 39, 390-396, 1987, N. Eng. J. Med, 333, 1038-1044, 1995, etc.

A CTL induced by the peptide of the present invention and/or FAM83B protein has a cytotoxic action toward a cell presenting the peptide of the present invention and/or another FAM83B protein-derived epitope peptide as an antigen and the ability to produce a lymphokine. Since the peptide of the present invention is a tumor antigen peptide as described above, and the FAM83B protein is decomposed within a cell to thus form a tumor antigen peptide, it can exhibit an anti-tumor action, and preferably an anti-cancer action, via the above functions. Therefore, the peptide of the present invention and/or FAM83B protein and a CTL induced thereby can be an active ingredient of a medicament or a pharmaceutical composition for the prevention and/or treatment of a cancer.

When a CTL inducer containing the peptide of the present invention and/or FAM83B protein as an active ingredient is administered to a cancer patient, the peptide of the present invention and/or the FAM83B protein-derived epitope peptide is presented on an HLA-A02 antigen or HLA-A24 antigen of an antigen-presenting cell, a CTL that is specific to a complex of the HLA-A02 antigen or HLA-A24 antigen and the presented peptide proliferates and destroys the cancer cells, and as a result, the cancer can be prevented and/or treated. Therefore, a CTL inducer containing the peptide of the present invention and/or FAM83B protein as an active ingredient can preferably be used for a subject who is positive for an HLA-A02 antigen or HLA-A24 antigen and who has a FAM83B-positive cancer. Examples of FAM83B-positive cancers include cancers (tumors) such as colon cancer, lung cancer, breast cancer, oral cancer, cervical cancer, thyroid cancer, testicular tumor, and ovarian cancer, and the CTL inducer of the present invention may be used for the prevention and/or treatment of such cancers.

The 'prevention' of a cancer includes not only preventing a patient from having a cancer but also prevention of recurrence in a patient who has been subjected to surgery to remove a primary tumor and prevention of metastasis of a tumor that could not be completely removed by a cancer treatment such as surgery, radiotherapy, drug therapy, etc. Furthermore, the 'treatment' of a cancer includes not only curing and improvement of the symptoms of a cancer that reduces the size of the cancer but also prevention of cancer cell proliferation or tumor enlargement, or suppression of metastasis of cancer cells from a primary focus.

A CTL inducer containing the peptide of the present invention and/or FAM83B protein as an active ingredient is for example particularly effective for an HLA-A02- or HLA-A24-positive cancer patient who has a cancer positive for the FAM83B described in SEQ ID No: 2. Specifically, it may be used for the prevention or treatment of a cancer (tumor) such as for example colon cancer, lung cancer, or ovarian cancer. Therefore, a pharmaceutical composition containing the peptide of the present invention as an active ingredient is also included in the present invention. Such a pharmaceutical composition is preferably a composition for the prevention and/or treatment of a cancer, that is, an agent for the prevention and/or treatment of a cancer. Furthermore, since the pharmaceutical composition of the present invention prevents and/or treats a cancer by inducing a CTL that is specific to a cancer cell (preferably a cancer stem cell), that is, activating cell-mediated immunity that is specific to a cancer cell, it is preferably a vaccine for the prevention and/or treatment of a cancer.

A pharmaceutical composition containing the peptide of the present invention as an active ingredient may be one that contains a single CTL epitope (the peptide of the present invention) as an active ingredient or one that contains as an active ingredient a polyepitope peptide having another peptide (CTL epitope or helper epitope) linked thereto. In recent years, it has been shown that a polyepitope peptide having a plurality of linked CTL epitopes (antigen peptides) has activity in efficiently inducing CTLs in vivo. For example, Journal of Immunology 1998, 161: 3186-3194 (this publication forms part of the present application by reference) describes the induction in vivo of a CTL that is specific to each CTL epitope by means of an approximately 30mer polyepitope peptide in which cancer antigen protein PSA-derived HLA-A2, -A3, -All, and -B53-restricted CTL epitopes (antigen peptides) are linked. It is also shown that a polyepitope peptide in which a CTL epitope and a helper epitope are linked efficiently induces a CTL. When administered in the configuration of such a polyepitope peptide, the polyepitope peptide is incorporated into an antigen-presenting cell, and after that, individual antigen peptides that have been formed by intracellular degradation bind to an HLA antigen to thus form a complex, this complex is presented on the antigen-presenting cell surface at high density, a CTL specific to this complex proliferates efficiently in the body, and cancer cells are destroyed. In this way, the treatment or prevention of a cancer is promoted.

A pharmaceutical composition containing the peptide of the present invention and/or FAM83B protein as an active ingredient may be administered as a mixture with a pharmaceutically acceptable carrier, for example an appropriate adjuvant, or in combination therewith, so as to establish cell-mediated immunity effectively.

As the adjuvant, an adjuvant known in the present technical field such as one described in the literature (for example, Clin Infect Dis.: S266-70, 2000) may be applied, and specific examples include a gel type such as aluminum hydroxide, aluminum phosphate, or calcium phosphate, a bacterial type such as CpG, monophosphoryl lipid A (monophosphoryl lipid A; MPL), cholera toxin, Escherichia coli heat-labile toxin, pertussis toxin, or muramyl dipeptide (Muramyl dipeptide; MDP), an oil emulsion type (emulsion preparation) such as Freund's incomplete adjuvant, MF59, or SAF, a macromolecular nanoparticle type such as an immunostimulatory complex (Immunostimulatory complex; ISCOMs), a liposome, biodegradable microspheres (Biodegradable microsphere), or saponin-derived QS-21, a synthetic type such as a nonionic block copolymer, a muramyl peptide analog (Muramyl peptide analogue), a polyphosphazene, or a synthetic polynucleotide, and a cytokine type such as IFN-$\gamma$, IL-2, or IL-12.

Furthermore, the dosage form of a CTL inducer/pharmaceutical composition containing the peptide of the present invention and/or FAM83B protein as an active ingredient is not particularly limited, and examples include an oil emulsion (emulsion formulation), macromolecular nanoparticles, a liposome formulation, a particulate formulation bonded to beads having a diameter of a few μm, a lipid-bonded formulation, a microsphere formulation, and a microcapsule formulation.

Examples of an administration method include any known administration method such as intradermal administration, subcutaneous administration, intramuscular administration, or intravenous administration. The dose of the peptide of the present invention in a preparation may be adjusted as appropriate according to the target disease to be treated, the age and body weight of the patient, etc., but it is usually 0.0001 mg to 1000 mg, preferably 0.001 mg to 1000 mg, and more preferably 0.1 mg to 10 mg, this being preferably administered once in a few days to a few months.

As a method for making the peptide of the present invention actually act as a medicament, there is an in vivo method in which the peptide is directly introduced into the body as well as an ex vivo method in which a specific type of cells are collected from a person, the peptide of the present invention is made to act thereon in vitro, and the cells are returned into the body (Nikkei Science, April, 1994, pp. 20-45, Gekkan Yakuji, 36 (1), 23-48 (1994), Experimental Medicine Special Edition, 12 (15), (1994), references quoted therein, etc., these publications forming part of the present application by reference), and a person skilled in the art can select a cell, an administration method, an administration configuration, and a dose appropriate for such a method.

(5) CTL inducer/pharmaceutical composition containing the polynucleotide of the present invention as active ingredient

Since a cell in which the polynucleotide of the present invention and/or FAM83B protein-encoding polynucleotide invention is expressed becomes a cell that presents the peptide of the present invention and/or another FAM83B protein-derived epitope peptide as an antigen, it has the feature that it is recognized by a T cell via a T cell receptor. Therefore, the polynucleotide of the present invention and/or FAM83B protein-encoding polynucleotide can also become a CTL inducer. An induced CTL can exhibit, in the same way as for a CTL induced by the peptide of the present invention and/or FAM83B protein, an anti-tumor action via a cytotoxic action or the production of a lymphokine, and preferably an anti-cancer action. Therefore, the polynucleotide of the present invention and/or FAM83B protein-encoding polynucleotide can be an active ingredient of a medicament or a pharmaceutical composition for the treatment or prevention of a cancer. A CTL inducer containing the polynucleotide of the present invention and/or FAM83B protein-encoding polynucleotide as an active ingredient can treat and/or prevent a cancer by for example administering the polynucleotide of the present invention and/or FAM83B protein-encoding polynucleotide to a cancer patient and expressing them in the cancer patient.

For example, when the polynucleotide of the present invention and/or FAM83B protein-encoding polynucleotide incorporated into an expression vector is administered to a cancer patient by the method below, a tumor antigen peptide is highly expressed within antigen-presenting cells. The tumor antigen peptide thus produced subsequently binds to an HLA-A02 antigen or an HLA-A24 antigen to form a complex, this complex is presented at high density on the antigen-presenting cell surface, cancer-specific CTLs proliferate efficiently in the body, and the cancer cells are destroyed. As described above, the treatment or prevention of a cancer is achieved. Therefore, a pharmaceutical composition containing the polynucleotide of the present invention is also included in the present invention. Such a pharmaceutical composition is preferably a composition for the prevention and/or treatment of a cancer, that is, an agent for the prevention and/or treatment of a cancer. Furthermore, since the pharmaceutical composition of the present invention prevents and/or treats a cancer by inducing a CTL that is specific to a cancer cell (preferably a cancer stem cell), that is, activating cell-mediated immunity that is specific to a cancer cell, it is preferably a vaccine for the prevention and/or treatment of a cancer.

The CTL inducer/pharmaceutical composition containing the polynucleotide of the present invention as an active ingredient may preferably be used for an HLA-A02 antigen- or HLA-A24 antigen-positive subject who has a FAM83B-positive cancer. Examples of the FAM83B-positive cancer include cancers (tumors) such as colon cancer, lung cancer, breast cancer, oral cancer, cervical cancer, thyroid cancer, testicular tumor, and ovarian cancer, and the CTL inducer may be used for the prevention or treatment of these cancers.

As a method for administering the polynucleotide of the present invention and/or FAM83B protein-encoding polynucleotide and incorporating it into a cell, any method such as a method involving a virus vector and other methods (Nikkei Science, 1994, April, pp. 20-45, Gekkan Yakuji, 36 (1), 23-48 (1994), Experimental Medicine Special Edition, 12 (15), (1994), references quoted therein, etc., these publications forming part of the present application by reference) may be employed. Therefore, in an embodiment of the pharmaceutical composition of the present invention, a vector containing the polynucleotide of the present invention and/or the FAM83B protein-encoding polynucleotide is contained as an active ingredient.

Examples of a method involving a virus vector include a method in which the DNA of the present invention is integrated into for example a DNA virus or RNA virus such as a retrovirus, adenovirus, adeno-associated virus, herpes virus, vaccinia virus, poxvirus, poliovirus, or sindbis virus, and incorporation is carried out. Among them, a method involving a retrovirus, adenovirus, adeno-associated virus, vaccinia virus, etc. is particularly preferable.

Examples of other methods include a method in which an expression plasmid is directly administered intramuscularly (DNA vaccine method), a liposome method, a lipofectin method, a microinjection method, a calcium phosphate method, and an electroporation method, and a DNA vaccine method and a liposome method are particularly preferable.

In order to make the polynucleotide of the present invention and/or FAM83B protein-encoding polynucleotide actually act as a medicament, there are an in vivo method in which the polynucleotide is directly introduced into the body and an ex vivo method in which a specific type of cells are collected from a person, the polynucleotide of the present invention is incorporated into the cells in vitro, and the cells are returned into the body (Nikkei Science, 1994, April, pp. 20-45, Gekkan Yakuji, 36 (1), 23-48 (1994), Experimental Medicine Special Edition, 12 (15), (1994), references quoted therein, etc., these publications forming part of the present application by reference). An in vivo method is more preferable.

When the polynucleotide of the present invention and/or FAM83B protein-encoding polynucleotide is administered by an in vivo method, administration may be carried out by selecting as appropriate an administration route and an administration form according to the target disease to be treated, the symptoms, etc. For example, administration may be carried out in a form that can be injected into a vein, an artery, subcutaneously, intradermally, intramuscularly, etc. When administration is carried out by an in vivo method, for example, a formulation form such as a liquid may be employed, but it is usually made into an injection, etc. containing the polynucleotide of the present invention, which is an active ingredient, and a pharmaceutically acceptable carrier (carrier) may be added as necessary. With regard to a liposome or a membrane fusion liposome (Sendai virus (HVJ)-liposome, etc.) containing the polynucleotide of the present invention, a liposome preparation such as a suspension, a frozen agent, or a centrifugation-concentrated frozen agent may be employed.

The content of the polynucleotide of the present invention in a formulation may be adjusted as appropriate according to the target disease to be treated, the age and body weight of the patient, etc.; it is usually 0.0001 mg to 100 mg as a polynucleotide content, and preferably 0.001 mg to 10 mg of the polynucleotide of the present invention, it preferably being administered once in a few days to a few months.

A person skilled in the art can appropriately select a suitable cell, vector, administration method, administration form, and dose.

Furthermore, in recent years, it has been shown that a polynucleotide encoding a polyepitope peptide having a plurality of linked CTL epitopes (tumor antigen peptides) and a polynucleotide encoding a polyepitope peptide having a CTL epitope and a helper epitope that are linked have activity in efficiently inducing CTLs in vivo. For example, Journal of Immunology 1999, 162: 3915-3925 (this publication forms part of the present application by reference) reports that DNA encoding an epitope peptide (minigene) having six types of HBV-derived HLA-A02-restricted antigen peptides, three types of HLA-A11-restricted antigen peptides, and a helper epitope that are linked has induced CTLs for each epitope in vivo effectively.

Therefore, a CTL inducer active ingredient can be made by incorporating into an appropriate expression vector a polynucleotide prepared by linking one or more types of polynucleotide encoding the peptide of the present invention, and in some cases also linking a polynucleotide encoding another peptide. Such a CTL inducer can also employ the same administration method and administration form as described above.

(6) Antigen-presenting cell

The peptide and polynucleotide of the present invention described above may be utilized for example in vitro as follows. That is, either of the peptide and polynucleotide of the present invention and cells having antigen-presenting ability are brought into contact with each other in vitro, thus enabling antigen-presenting cells to be prepared. Therefore, the following disclosure concerns an antigen-presenting cell that presents on the cell surface a complex of an HLA-A02 antigen or an HLA-A24 antigen and the peptide of the present invention, and a method for producing same. As described above, the peptide and polynucleotide of the present invention can be utilized for the prevention and/or treatment of a cancer. Therefore, the antigen-presenting cell or the production method therefor preferably utilizes an isolated cell that is derived from a cancer patient. Specifically, an antigen-presenting cell presenting a complex of an HLA-A02 antigen or an HLA-A24 antigen and the peptide of the present invention on the cell surface of a cancer patient-derived isolated cell having antigen-presenting ability is produced by bringing the cell into contact with either the peptide or the polynucleotide of the present invention in vitro.

The ’cell having antigen-presenting ability’ is not particularly limited as long as it is a cell expressing on the cell surface an MHC, preferably an HLA, and more preferably an HLA-A02 antigen or an HLA-A24 antigen, that can present the peptide of the present invention, and among them it is preferably a professional antigen-presenting cell, and particularly preferably a dendritic cell, which is considered to have high antigen-presenting ability.

Furthermore, with regard to a substance that is added in order to prepare the antigen-presenting cell of from the cell having an antigen-presenting ability, it may be either the peptide or the polynucleotide of the present invention.

The antigen-presenting cell is obtained by for example isolating cells having antigen-presenting ability from a cancer patient, and pulsing the cells with the peptide of the present invention in vitro so as to make them present a complex of an HLA-A02 antigen or an HLA-A24 antigen and the peptide of the present invention (Cancer Immunol.

Immunother., 46: 82, 1998, J. Immunol., 158, p. 1796, 1997, Cancer Res., 59, p. 1184, 1999). When dendritic cells are used, for example, lymphocytes are separated from the peripheral blood of a cancer patient by the Ficoll method, non-adherent cells are then removed, adherent cells are cultured in the presence of GM-CSF and IL-4 to thus induce dendritic cells, and the dendritic cells are cultured and pulsed together with the peptide of the present invention, thus enabling the antigen-presenting cell to be prepared.

Furthermore, when the antigen-presenting cell is prepared by transfecting the cell having an antigen-presenting ability with the polynucleotide of the present invention, the polynucleotide may be in the form of a DNA or the form of an RNA. Specifically, in the case of a DNA, Cancer Res., 56: p. 5672, 1996 or J. Immunol., 161: p. 5607, 1998 (these publications forming part of the present application by reference) may be referred to, and in the case of an RNA, J. Exp. Med., 184: p. 465, 1996 (this publication forming part of the present application by reference) may be referred to.

The antigen-presenting cell can be an active ingredient of a CTL inducer. The CTL inducer containing the antigen-presenting cell as an active ingredient preferably contains physiological saline, phosphate buffered physiological saline (PBS), a medium, etc. in order to maintain the antigen-presenting cell stably. Examples of an administration method include intravenous administration, subcutaneous administration, and intradermal administration. Returning a CTL inducer containing such an antigen-presenting cell as an active ingredient to the body of the patient enables a CTL that is specific to a cancer cell presenting the peptide of the present invention as an antigen to be efficiently induced in the body of a patient having a FAM83B-positive cancer, and as a result a FAM83B-positive cancer that subjects the peptide of the present invention to antigen presentation can be treated.

(7) Cytotoxic T cell (CTL)

The peptide and polynucleotide of the present invention may be utilized in vitro for example as follows. That is, a CTL may be induced by bringing either the peptide or the polynucleotide of the present invention into contact with peripheral blood lymphocytes in vitro. Therefore, the following disclosure concerns a CTL that specifically damages a cell that subjects the peptide of the present invention to antigen presentation, and a method for inducing same. As described above, the peptide and polynucleotide of the present invention can be utilized for preventing and/or treating a cancer. Therefore, the CTL and the induction method therefor preferably utilize peripheral blood lymphocytes derived from a cancer patient. Specifically, a CTL that specifically damages a cell subjecting the peptide of the present invention to antigen presentation is induced by bringing either the peptide or the polynucleotide of the present invention into contact in vitro with peripheral blood lymphocytes derived from a cancer patient.

In a melanoma for example, it has been confirmed that an adoptive immunotherapy in which a large number of intratumoral infiltrating T cells from the patient in question are cultured in vitro and returned to the patient has a therapeutic effect (J. Natl. Cancer. Inst., 86: 1159, 1994). Furthermore, in a mouse melanoma it has been confirmed that metastasis is suppressed by stimulating spleen cells in vitro with TRP-2 tumor antigen peptide so as to make CTLs specific to the tumor antigen peptide proliferate and administering the CTLs to a melanoma transplanted mouse (J. Exp. Med., 185: 453, 1997). This is based on the result that CTLs that specifically recognize a complex of a tumor antigen peptide and an MHC of an antigen-presenting cell proliferate in vitro. It is therefore considered that a therapy in which peripheral blood lymphocytes of a patient are stimulated in vitro using the peptide or the polynucleotide of the present invention to thus increase tumor-specific CTLs and the CTLs are subsequently returned to the patient will be useful.

The CTLs may be an active ingredient of a therapeutic agent or a preventive agent for a cancer. The therapeutic agent or the preventive agent preferably contains physiological saline, phosphate buffered physiological saline (PBS), a medium, etc. in order to maintain the CTLs stably. Examples of an administration method include intravenous administration, subcutaneous administration, and intradermal administration. Returning the cancer treatment or preventive agent containing such CTLs as an active ingredient to the body of a patient enables the cytotoxicity of the CTLs to cancer cells in the body of a patient having the FAM83B-positive cancer to be promoted, and the cancer to be treated by destroying the cancer cells.

The CTL can exhibit cytotoxicity with, as a target, a complex of an HLA and the peptide of the present invention that is subjected to antigen presentation on a tumor cell. That is, a T cell receptor (TCR) of the CTL recognizes a complex of an HLA and the peptide of the present invention. In recent years, an adoptive immunotherapy has been devised in which a gene of a TCR that recognizes a specific peptide-HLA complex expressed in a CTL is cloned, this TCR gene is transferred to a CD8+ T cell harvested from a cancer patient to thus artificially produce a CTL, it is cultured on a large scale, and it is then returned to the body of the patient (e.g. Ochi et al., Blood. 2011 Aug 11; 118 (6): 1495-503, etc.). In the present invention, when an 'artificial CTL' is referred to, it means a CTL that is formed by transferring a gene encoding a TCR that recognizes a complex of a peptide and an HLA to a T cell as described above, and this can also be used in the treatment of a cancer in the same way as for the above natural CTL. Therefore, such an artificial CTL is included in the present invention. In such an embodiment, a TCR that recognizes a complex of the peptide of the present invention and an HLA and that is genetically transferred to an artificial CTL may be modified as appropriate in order to increase the binding affinity toward the complex or the cytotoxicity. Therefore, the 'artificial CTL' includes a CTL that is formed by appropriately genetically modifying a gene encoding a TCR that recognizes a complex of the peptide of the present invention and an HLA and then transferring the gene to a patient-derived T cell. Preparation of an artificial CTL may employ a method known in the present technical field.

(8) Tumor-specific CTL-detecting agent using the peptide of the present invention

The peptide of the present invention, in particular a peptide with an amino acid sequence described in SEQ ID Nos: 3, 4, 6, 9, 12, 14, 15, 20, 23, 24, 29-33, 35, 37, 38, 47, 57, 60, 65-68, 71-75 and 77-80, is recognized by a tumor-specific CTL, and is therefore useful as a component of a tumor-specific CTL-detecting agent. Therefore, the following disclosure relates to a tumor-specific CTL-detecting agent containing the peptide of the present invention. In one embodiment, the tumor-specific CTL-detecting agent contains an HLA multimer (monomer, dimer, tetramer, pentamer, or Dextramer) containing HLA-A02 or HLA-A24 and the peptide of the present invention.
For example, the HLA tetramer means a tetramer formed by biotinylating a complex (HLA monomer) in which the $\alpha$ chain and the $\beta 2$ microglobulin of the HLA are associated with a peptide (epitope peptide) and binding it to avidin (Science 279: 2103-2106 (1998), Science 274: 94-96 (1996)). At present HLA tetramers containing various types of antigen peptides are commercially available (e.g. from Medical & Biological Laboratories Co., Ltd.), and an HLA tetramer containing the peptide of the present invention and HLA-A02 or HLA-A24 can be easily prepared. Furthermore, an HLA dimer and an HLA pentamer are also based on the same principle, the HLA monomer being formed into the dimer and the pentamer respectively. An HLA multimer containing the peptide of the present invention and HLA-A02 or HLA-A24 is also one embodiment of the present invention.
Specific examples include an HLA tetramer containing a peptide with an amino acid sequence described in SEQ ID Nos: 3-85 and HLA-A02 or HLA-A24. The HLA tetramer is preferably fluorescently-labeled so that bound CTLs can be easily screened or detected by known detection means such as flow cytometry or a fluorescence microscope. Specific examples include HLA tetramers labeled with phycoerythrin (PE), fluorescein isothiocyanate (FITC), peridinin chlorophyll protein (PerCP), etc.
Examples of methods for producing an HLA tetramer include those described in the literature, such as Science 279: 2103-2106 (1998) and Science 274: 94-96 (1996), which are described in brief below.
First, Escherichia coli or mammalian cells that can express a protein are transfected with an HLA-A24 or HLA-A02 $\alpha$ chain expression vector and a $\beta 2$ microglobulin expression vector and expression is carried out. In this embodiment, it is preferable to use Escherichia coli (for example, BL21). The monomer HLA-A24 or HLA-A02 complex thus obtained and the peptide of the present invention are mixed to thus form a soluble HLA-peptide complex. Subsequently, the C terminal site sequence of the $\alpha$ chain of HLA-A02 or HLA-A24 in the HLA-peptide complex is biotinylated with BirA enzyme. This biotinylated HLA-peptide complex and fluorescently-labeled avidin are mixed at a molar ratio of 4:1, thus preparing an HLA tetramer. In each of the above steps, it is preferable to carry out protein purification by means of gel filtration, etc.

(9) Cancer stem cell-detecting agent

As described above, the present inventors have found for the first time that FAM83B is highly expressed specifically in a cancer stem cell. That is, it has been found for the first time by the present inventors that FAM83B is a gene whose expression is not observed in a cancer cell except a cancer stem cell or a normal somatic cell, but that is highly expressed in a cancer stem cell. It has been found from such a finding that FAM83B can be utilized as a marker for identifying a cancer cell, and in particular a cancer stem cell. Therefore, one aspect of the present disclosure relates to a cancer stem cell-detecting agent that contains a FAM83B-detecting agent for detecting an expression product of FAM83B.
In the present disclosure, when just 'FAM83B' is used, it means a FAM83B gene unless otherwise specified. It preferably means a human FAM83B gene but it may be a homolog thereof.
In the present disclosure, 'gene expression' means a series of biological reactions initiated by gene transcription, and an 'expression product' is a molecule produced by this series of biological reactions, such as an mRNA or an endogenous polypeptide. An endogenous polypeptide, which is a gene expression product, is preferably a protein that is the final product of gene expression.
In the present disclosure, a 'FAM83B-detecting agent' means an agent for qualitatively and/or quantitatively detecting a FAM83B gene or an expression product thereof.
The cancer stem cell-detecting agent contains a FAM83B-detecting agent for detecting a FAM83B expression product. When a FAM83B expression product is detected in a detection target, it can be determined that the detection target has a cancer stem cell, i.e., a cancer stem cell has been detected. The cancer stem cell-detecting agent can be used in vivo or in vitro, but it is preferably used in vitro for a cell population derived from a biological sample (detection target) harvested from a biological individual (test subject). In this case, detection of a cancer stem cell in a detection target which is a cell population derived from a biological sample means that a cancer stem cell has been detected in a test subject, i.e., biological individual from which a biological sample has been harvested, that is, the biological individual has a cancer stem cell. Therefore, as described herein below, a method for detecting a cancer stem cell in a test subject using the cancer stem cell-detecting agent is also included in the present disclosure.

The biological individual as a test subject may be any biological individual as long as it is a biological individual that can have a tumor but is preferably a human or a non-human mammal individual (e.g. a rodent such as a mouse, a rat, a guinea pig, or a hamster, a primate such as a chimpanzee, an artiodactyl such as a cow, a goat, or a sheep, a perissodactyl such as a horse, a rabbit, a dog, a cat, etc.), and more preferably a human individual.

The cell population as a detection target can be any biological sample-derived cell population obtained from the test subject but is preferably a cell population derived from a biological sample obtained from a human, and more preferably a cell population containing a cell derived from one or more biological samples selected from the group consisting of heart, brain, placenta, lung, liver, skeletal muscle, kidney, pancreas, spleen, thymus, prostate, testis, ovary, small intestine, large intestine, and blood, which is confirmed that almost no FAM83B is expressed.

The FAM83B-detecting agent contained in the cancer stem cell-detecting agent can be changed depending on the expression product that is to be detected, and a person skilled in the art can select the most suitable one as appropriate. Specifically, for example, when the expression product is an mRNA, any mRNA detection method known in the present technical field may be used, and examples include, but are not limited to, an RT-PCR method, an in situ hybridization method, a Northern blotting method, and real time RT-PCR and, among them, a RT-PCR method is preferable from the viewpoint of high detection sensitivity and ease of experimental technique. For example, when the expression product is an endogenous polypeptide (preferably a FAM83B protein), examples include, but are not limited to, a Western blotting method and immunohistochemical staining. The FAM83B-detecting agent used can be changed depending on the expression product that is to be detected and the detection method employed, and a person skilled in the art can select the most suitable one as appropriate. Specifically, for example, when an endogenous polypeptide is to be detected, a FAM83B-specific antibody (preferably a monoclonal antibody), etc. can be cited, and when an mRNA is to be detected, a probe and/or a primer that have a base sequence complementary to SEQ ID No: 1 can be cited, but examples are not limited to the above. Moreover, the expression product that is to be detected may be a single expression product or a combination of a plurality of expression products.

(10) T cell receptor-like antibody that recognizes complex of the peptide of the present invention and an MHC

As described above, the peptide of the present invention is presented as a CTL epitope peptide on a cancer cell, and in particular a cancer stem cell. In this process, since it is presented on a cell surface by forming a complex with an MHC, it is possible to utilize the peptide of the present invention as a tumor marker by the use of an antibody that recognizes the complex. Examples of such an antibody include a TCR (T cell receptor)-like antibody that recognizes a complex of the peptide of the present invention and an HLA, preferably HLA-A24 or HLA-A02. Therefore, the present invention also relates to a T cell receptor-like antibody that recognizes a complex of the peptide of the present invention and an MHC.

In the present invention, the 'TCR-like antibody' is a molecule having binding ability (antigen-recognizing ability) similar to TCR to a complex of a fragmented antigen-derived peptide and a major histocompatibility complex (MHC) molecule (pMHC). For example, as reported in Eur J Immunol. 2004; 34: 2919-29, etc., a TCR-like antibody that recognizes a complex of a tumor antigen-derived peptide and an MHC can recognize a cancer cell that is presenting a tumor antigen peptide that can be targeted by a CTL, a dendritic cell that has phagocytized a cancer cell and is presenting a tumor antigen peptide on an MHC class I, etc.

Furthermore, the TCR-like antibody that recognizes a complex of an MHC and a peptide derived from a virus, etc. can quantitatively and chronologically analyze what kind of presentation kinetics, CTL response, etc. a presented antigen will show on an infected cell.

The TCR-like antibody may be prepared by a method described in Eur J Immunol. 2004; 34: 2919-29, etc. For example, immunizing an animal such as a mouse with an MHC-peptide complex enables an antibody that is specific to the complex to be obtained. It is also possible to obtain a complex-specific antibody by utilizing a phage display method.

As described above, recognizing an MHC complex presenting the peptide of the present invention enables a tumor cell that presents the MHC complex on the cell surface to be detected. Therefore, the present disclosure also relates to a tumor-detecting agent containing the TCR-like antibody. Furthermore, since the peptide of the present invention is similarly presented on an antigen-presenting cell, preferably a professional antigen-presenting cell such as a dendritic cell, in addition to a tumor cell, the TCR-like antibody is also useful for detection of an antigen-presenting cell, etc. presenting the peptide of the present invention.

Moreover, as described above, since the peptide of the present invention is presented as a CTL epitope peptide by a cancer cell, and in particular a cancer stem cell, a TCR-like antibody that recognizes a complex of the peptide of the present invention and an HLA, and preferably HLA-A24 or HLA-A02, is useful as an agent for the prevention and/or treatment of a cancer in a subject. Therefore, the present invention also relates to an agent containing the TCR-like antibody of the present invention for the prevention and/or treatment of a cancer.

Furthermore, in recent years, a new immune cell therapy has been devised, which includes, forming a chimeric antigen receptor (CAR) by genetically engineering and modifying a part of a monoclonal antibody specific to tumor antigen, genetically transferring it to a patient-derived T cell, culturing and amplifying this genetically modified T cell ex vivo, and injecting the genetically modified T cells into the patient (Nat Rev Immunol. 2012; 12: 269-81). Specifically, peripheral blood mononuclear cells harvested from a patient are cultured in the presence of an anti-CD3 antibody and IL-2, etc. to thus activate T cells, and a gene encoding CAR is introduced into the T cells by the use of a transfection vector such as a retrovirus vector or a lentivirus vector to thus prepare genetically modified T cells.

In the present invention, the 'chimeric antigen receptor' is a chimeric protein molecule that has been designed so as to have at the N terminal a single chain antibody (scFv) which a light chain and a heavy chain of an antibody variable region of an antibody that recognizes a molecule present on the cell surface of a cancer cell is tandemly linked, and have at the C terminal a CD3ζ chain among molecules constituting a T cell receptor (TCR)/CD3 complex. This chimeric antigen receptor recognizes a specific antigen via the scFv region, then causing activation of a T cell via the CD3ζ chain. In order to enhance the activation of a T cell, one or more costimulators (e.g. CD28, 4-1BB, ICOS, etc.) may be incorporated between the scFv and the ζ chain. In the present invention, as the scFv, a CAR may be prepared using the TCR-like antibody of the present embodiment (including an antibody molecule designed from the TCR-like antibody or a fragment thereof). Since a CAR that recognizes a complex of a tumor antigen-derived peptide and an MHC can recognize a cancer cell that is presenting a tumor antigen peptide that can be targeted by a CTL, a dendritic cell that has phagocytized a cancer cell and is presenting a tumor antigen peptide on an MHC class I, etc., the genetically modified T cell into which the CAR has been introduced is useful as an agent for the prevention and/or treatment of a cancer that is specific to the tumor antigen, in the same way as for the artificial CTL. Therefore, the present invention also relates to an agent for the prevention and/or treatment of a cancer containing a genetically modified T cell or an artificial CTL into which has been introduced a CAR that recognizes a complex of the tumor antigen-derived peptide of the present invention and an MHC.

(11) Tumor detection method (test method, diagnostic method)

The present disclosure provides a tumor detection method (test method, diagnostic method) utilizing the CTL-detecting agent, the cancer stem cell-detecting agent, or the tumor-detecting agent, which are described above.

The detection method (diagnostic method) using the CTL-detecting agent typically involves harvesting blood from a test subject or harvesting part of the test tissue for which a tumor is suspected by means of a biopsy, etc., and detecting/measuring the amount of CTLs that recognize a complex of an HLA antigen and a FAM83B-derived tumor antigen peptide contained therein by means of the CTL-detecting agent, thus detecting, testing, or diagnosing the presence or absence or the extent of a FAM83B-positive cancer (tumor) such as colon cancer, lung cancer, breast cancer, oral cancer, cervical cancer, thyroid cancer, testicular tumor, or ovarian cancer.

The detection method (test method, diagnostic method) using the cancer stem cell-detecting agent typically involves detecting, testing, or diagnosing the presence or absence or the extent of a FAM83B-positive cancer (tumor) such as colon cancer, lung cancer, breast cancer, oral cancer, cervical cancer, thyroid cancer, testicular tumor, or ovarian cancer by harvesting blood from a test subject or harvesting by means of biopsy, etc. part of the test tissue for which a tumor is suspected, and detecting/measuring the amount of FAM83B expression product contained therein using the cancer stem cell-detecting agent.

The detection method (test method, diagnostic method) using the tumor-detecting agent typically involves harvesting blood from a test subject or harvesting part of the test tissue for which a tumor is suspected by means of a biopsy, etc., and detecting/measuring the amount of cells presenting a complex of an HLA antigen and a FAM83B-derived tumor antigen peptide contained therein by means of the tumor-detecting agent, thus detecting, testing, or diagnosing the presence or absence or the extent of a FAM83B-positive cancer (tumor) such as colon cancer, lung cancer, breast cancer, oral cancer, cervical cancer, thyroid cancer, testicular tumor, or ovarian cancer.

For example, the detection (test, diagnostic) method can detect (test, diagnose) the presence or absence or the extent of improvement of a tumor when a therapeutic drug is administered to a patient having a tumor in order to improve the tumor. Furthermore, the detection (test, diagnostic) method may be applied to the screening of a patient to be treated to whom a medicament containing the peptide or the polynucleotide of the present invention as an active ingredient can be applied effectively, and to the prediction, assessment, etc. of the therapeutic effect of the medicament. Moreover, in an embodiment in which the tumor-detecting agent is used, it is possible to detect a cancer cell presenting a tumor antigen peptide that can be actually targeted by a CTL induced within the living body of a patient by administering a cancer vaccine containing the peptide of the present invention as an active ingredient.

A specific embodiment of the detection (test) method using the CTL-detecting agent includes steps (a) and (b), and optionally step (c), as follows:

(a) a step of bringing a biological sample obtained from a test subject into contact with the CTL-detecting agent,
(b) a step of measuring the amount of CTLs that recognize a complex of an HLA antigen and a FAM83B-derived tumor antigen peptide in the biological sample using the amount of cells to which the CTL-detecting agent binds as an indicator, and
(c) a step of determining the presence of a cancer based on the result of (b).

A specific embodiment of the diagnostic method using the CTL-detecting agent steps (a), (b), and (c) above.
A specific embodiment of the detection (test) method using the cancer stem cell-detecting agent includes steps (d) and (e), and optionally step (f), as follows:

(d) a step of bringing a biological sample obtained from a test subject into contact with the cancer stem cell-detecting agent,
(e) a step of measuring the amount of FAM83B expression product in the biological sample, and
(f) a step of determining the presence of a cancer based on the result of (e).

A specific embodiment of the diagnostic method using the cancer stem cell-detecting agent includes steps (d), (e), and (f) above.
An embodiment of the method for detecting a cancer stem cell using the cancer stem cell-detecting agent includes steps (d) and (e) and step (f') below instead of (f):
(f') a step of determining the presence or absence of a cancer stem cell in a biological sample based on the result of (e).
Examples of the biological sample used here include a sample prepared from biological tissue (a tissue for which the presence of cancer cells is suspected, surrounding tissue thereof or blood etc.) of a test subject. Specific examples include a sample containing tissue cells harvested from the tissue.
A specific embodiment of the detection (test) method using the tumor-detecting agent includes steps (g) and (h), and optionally step (i), as follows:

(g) a step of bringing a biological sample obtained from a test subject into contact with the tumor-detecting agent,
(h) a step of measuring the amount of cells that present a complex of an HLA antigen and a FAM83B-derived tumor antigen peptide in the biological sample using the amount of cells to which the tumor-detecting agent binds as an indicator, and
(i) a step of determining the presence of a cancer based on the result of (h).

A specific embodiment of the diagnostic method using the tumor-detecting agent includes steps (g), (h), and (i) above.
Examples of the biological sample used here include a sample prepared from biological tissue (a tissue for which the presence of cancer cells is suspected, surrounding tissue thereof or blood etc.) of a test subject. Specific examples include a sample containing tissue cells harvested from the tissue.
One embodiment of the detection method (test method, diagnostic method) using the CTL-detecting agent is carried out by detecting a CTL specific to the peptide of the present invention in a biological sample and measuring the amount thereof. Specifically, a tetramer (HLA tetramer) of a complex of a fluorescently-labeled HLA antigen and the peptide of the present invention is prepared in accordance with a method described in the literature (Science, 274: p. 94, 1996, this publication forming part of the present application by reference), and this can be used for quantitatively determining by means of a flow cytometer the amount of antigen peptide-specific CTLs in peripheral blood lymphocytes of a patient for whom a cancer is suspected.
The prediction, assessment, determination, or diagnosis of the presence or absence of a tumor may be carried out by, for example, measuring the amount of CTLs specific to the peptide of the present invention in the blood or test tissue for which a tumor is suspected of a test subject or the amount of cells presenting the peptide of the present invention. In this process, in some cases, the level of FAM83B gene expression, the level of the peptide of the present invention, or the level of CTLs, etc. in the corresponding normal tissue may be used as a reference value, and this reference value may be compared with the level in the sample obtained from the test subject, the difference between the two being assessed.
The comparison of the levels between the test tissue of the test subject and the corresponding normal tissue may be carried out in parallel with measurement of the biological sample of the test subject and a biological sample of a healthy subject. When it is not carried out in parallel, the average value or the statistical median of the amounts of CTLs specific to the peptide of the present invention or the amounts of cells presenting the peptide of the present invention obtained using a plurality (at least two, preferably at least three, and more preferably at least five) of normal tissue pieces under uniform measurement conditions may be used in the comparison as the value for a healthy subject, that is, a reference value.

A determination of whether or not a test subject has a cancer may be carried out using as an indicator, for example, the amount of CTLs specific to the peptide of the present invention in tissue of the test subject or the cells presenting the peptide of the present invention being for example at least twice the level thereof in a healthy subject, and preferably at least three times.

Furthermore, in a test subject to which the peptide or the polynucleotide of the present invention is administered, it is also possible by measuring the amount of CTLs specific to the peptide of the present invention to assess whether or not CTLs have actually been induced. For example, it is possible to assess whether the treatment with the peptide or the polynucleotide of the present invention is effective by using as an indicator the amount of CTLs specific to the peptide of the present invention in the tissue of the test subject being for example at least twice the level thereof of a healthy subject, and preferably at least three times. Since it is considered that the peptide or the polynucleotide of the present invention mainly acts as a vaccine and induces CTLs to thus exhibit an anti-tumor action, it is possible to use the induction of CTLs as a POM (Proof of Mechanism) marker for confirming whether or not the peptide or the polynucleotide of the present invention administered acts as a vaccine. Therefore, by detecting CTLs as a POM marker, the CTL- detecting agent can be used as a diagnostic agent for confirming whether or not a peptide or polynucleotide administered to a subject is acting as a vaccine.

(12) Preventive and/or therapeutic method for cancer

The present diclosure also relates to a method for preventing and/or treating a cancer in a subject, the method including a step of administering an effective amount of an active ingredient selected from the group consisting of the peptide, the polynucleotide, the CTL, the antigen-presenting cell, the TCR-like antibody, the artificial CTL, and the genetically modified T cell of the present invention to a subject requiring same.

The 'subject' may be any biological individual as long as it is a biological individual who can suffer from a cancer, but is preferably a human or a non-human mammal individual (e.g. a rodent such as a mouse, a rat, a guinea pig, or a hamster, a primate such as a chimpanzee, an artiodactyl such as a cow, a goat, or a sheep, a perissodactyl such as a horse, a rabbit, a dog, a cat, etc.), and more preferably a human individual. The subject may be healthy or may have any disease, but when the prevention and/or treatment of a cancer is intended, it typically means a subject having a cancer or having a risk thereof. In one embodiment, the subject is HLA-A02-positive or HLA-A24-positive. In one embodiment, the subject has a FAM83B-positive cancer or has a risk thereof. In one embodiment, the subject is HLA-A02-positive or HLA-A24-positive and has a FAM83B-positive cancer or has a risk thereof.

With regard to the peptide, the polynucleotide, the TCR-like antibody, the artificial CTL, and the genetically modified T cell of the present invention used in the preventive/therapeutic method, any one described in the present specification can be cited. The effective amount referred to in the present invention is an amount that for example reduces the symptoms of a cancer or delays or halts the progress thereof, and is preferably an amount that suppresses or cures a cancer. Furthermore, it is preferably an amount that does not cause an adverse effect that exceeds the benefit obtained by administration. Such an amount may be determined as appropriate by means of an in vitro test using cultured cells, etc. or a test in a model animal such as a mouse or a rat, and such test methods are well known to a person skilled in the art. The specific dose of an active ingredient may be determined while taking into consideration various conditions related to a subject requiring same, for example, the seriousness of symptoms, the general health state, age, and body weight of the subject, the sex of the subject, diet, timing and frequency of administration, concomitant medication, response to treatment, dosage form, compliance with treatment, etc.

In the case of for example the peptide of the present invention, the specific dose is usually 0.0001 mg to 1000 mg, preferably 0.001 mg to 1000 mg, and more preferably 0.1 mg to 10 mg, and this is preferably administered once in a few days to a few months. Furthermore, in the case of the polynucleotide of the present invention, it is usually 0.0001 mg to 100 mg, and preferably 0.001 mg to 10 mg, and this is preferably administered once in a few days to a few months. In the case of the TCR-like antibody of the present invention, it is usually 0.0001 mg to 2000 mg, and preferably 0.001 mg to 2000 mg, and this is preferably administered once in 1 week to 4 weeks. In the case of the genetically modified T cell or artificial CTL of the present invention, it is usually $1 \times 10^4$ to $1 \times 10^8$, and preferably $1 \times 10^5$ to $1 \times 10^7$, and this is preferably administered once in 1 day to 4 weeks. As an administration method, any known appropriate administration method such as intradermal administration, subcutaneous administration, intramuscular administration, or intravenous administration may be used. It is also possible to use an in vivo method in which the peptide or the nucleotide of the present invention is directly administered into the body as well as an ex vivo method in which a specific type of cells are collected from a person, CTLs or antigen-presenting cells are induced in vitro using the peptide or the polynucleotide of the present invention, and these cells are subsequently returned into the body.

One embodiment of the preventive/therapeutic method further includes, prior to the administration step, a step of selecting a subject who is HLA-A02-positive or HLA-A24-positive as a subject for the prevention/treatment. This embodiment may further include, prior to the selection step, a step of determining the HLA type of a subject. Determination of the HLA type of a subject may be carried out by any known method. Furthermore, one embodiment of the preventive/therapeutic method further includes, prior to the administration step, a step of selecting a subject having a FAM83B-positive cancer as a subject for the prevention/treatment. This embodiment may further include, prior to the selection step, a step of detecting a FAM83B-positive cancer in a subject. Detection of a FAM83B-positive cancer in a subject may employ the tumor detection method described in (10) above.

One embodiment of the preventive/therapeutic method further includes, prior to the administration step, a step of screening a subject who is HLA-A02-positive or HLA-A24-positive and has a FAM83B-positive cancer as a subject for the prevention/treatment. This embodiment further include, prior to the screening step, a step of determining the HLA type of a subject and a step of detecting a FAM83B-positive cancer in a subject. A subject who is HLA-A02-positive or HLA-A24-positive and has a FAM83B-positive cancer is a subject to whom a pharmaceutical composition containing the peptide and/or polynucleotide of the present invention as an active ingredient can be applied effectively. Therefore, the tumor-specific CTL-detecting agent or the tumor-detecting agent that can be used in the tumor detection method described in (11) above can be used as a diagnostic agent for a so-called companion diagnosis for screening a subject for treatment for whom a cancer treatment method using the pharmaceutical composition of the present invention is effective.

Furthermore, one embodiment of the preventive/therapeutic method may further include a step of assessing whether or not the treatment with the pharmaceutical composition of the present invention is effective in a patient to whom the pharmaceutical composition of the present invention has been administered, and screening a subject for treatment for whom the treatment with the pharmaceutical composition of the present invention is effective. As described in (11) above, a subject in which CTLs specific to the peptide of the present invention are induced when the pharmaceutical composition of the present invention is administered is a subject for whom the treatment with a pharmaceutical composition containing the peptide and/or polynucleotide of the present invention as an active ingredient is effective, that is, a subject to whom the pharmaceutical composition of the present application can be applied effectively, and induction of CTLs specific to the peptide of the present invention may be used as a surrogate marker for predicting or assessing the therapeutic effect of the pharmaceutical composition of the present invention. Therefore, a tumor-specific CTL-detecting agent that can be used in the tumor detection method described in (11) above may be used as a diagnostic agent for screening an effective subject for treatment in patients to whom the pharmaceutical composition of the present invention has been administered or as a diagnostic agent for predicting or assessing the therapeutic effect of the pharmaceutical composition of the present invention in a patient to whom the pharmaceutical composition has been administered by detecting CTLs as the surrogate marker.

(13) Method for screening cancer treatment drugs using cancer stem cells as target

In an embodiment in which the cancer stem cell-detecting is used, the amount of FAM83B expression product expressed in a detection target is thought to be correlated with the amount of cancer stem cells in the detection target. Therefore, it is possible by comparing the amounts of FAM83B expression product expressed before and after administering a candidate compound for the cancer treatment drug to a detection target to determine whether or not the candidate compound administered is useful as a cancer treatment drug targeting cancer stem cells.

The screening method includes steps (I) and (II), and optionally (III):

(I) a step of measuring a detected amount A of an expression product of the FAM83B gene in a subject before administering a candidate compound for a cancer treatment drug to the subject,
(II) a step of measuring a detected amount B of an expression product of the FAM83B gene in the subject after administering the candidate compound to the subject cell population, and
(III) a step of determination of the candidate compound as a cancer treatment drug candidate with cancer stem cells as a target when the detected amounts A and B are compared and the detected amount A is significantly larger than B.

A specific embodiment of the screening method includes steps (I) to (III) above. The step of measuring the amount detected in step (I) and (II) includes steps (d) and (e) in the detection (test, diagnosis) method.

The present invention is specifically explained below by reference to Examples.

[Examples]

Experimental Example 1: Detection and subcloning of SP fraction of human colon cancer cells

a) Preparation of reagents

5% fetal bovine serum (FCS (HyClone Laboratories))-supplemented DMEM (Sigma-Aldrich) medium was prepared as a medium and warmed at 37°C. Verapamil (Sigma-Aldrich) was adjusted to 50 mM and diluted to 5 mM using the 5% FCS-supplemented DMEM medium. Hoechst 33342 (Lonza) was adjusted to 250 μg/mL using the 5% FCS-supplemented DMEM medium. DNase I (Qiagen) was adjusted to 1 mg/mL using DDW and sterilized by filtration using a 0.2 μm filter.

b) Preparation of cells for flow cytometry (FACS)

A human colon cancer cell line (SW480 (ATCC)) was suspended in 4 mL of the 5% FCS-supplemented DMEM medium, and the number of cells was counted. Furthermore, the 5% FCS-supplemented DMEM medium was added so as to adjust the cell concentration to $10 \times 10^6$ cells/mL, thus giving a sample. Using part of the sample, dispensing was carried out; verapamil was not added to a main sample (verapamil(-) sample), and verapamil was added to a secondary sample so as to give a final concentration of 75 μM (verapamil(+) sample). Subsequently, the Hoechst 33342 solution was added to the verapamil(+) sample and the verapamil(-) sample so as to give a Hoechst 33342 final concentration of 5.0 μM.

The two samples were cultured while shaking at 37°C for 90 minutes and then cooled on ice. Centrifuging at 1500 rpm and 4°C was carried out for 5 minutes, and the supernatant was removed. A suspension in 5% FCS-supplemented 1× PBS was formed and transferred to an ice-cooled FACS tube. Centrifuging at 1500 rpm and 4°C was again carried out for 5 minutes, the supernatant was removed, and a suspension in 5% FCS-supplemented 1× PBS was formed. The same washing was repeated once, and a suspension in 2 mL of 2% FCS-supplemented 1× PBS with 2 mM EDTA was then formed. 2 μL of the DNase I solution was added and mixed, and a cell clump was then removed using a FACS filter (Beckton Dickinson (BD)). After 2 μL of 1 mg/mL propidium iodide (PI) (Sigma-Aldrich) was added, analysis was carried out using a BD FACS Aria II special edition (registered trademark) (BD) as a flow cytometer at a flow rate of 1000 to 2000 cells/sec.

c) Flow cytometry (FACS)

FACS operation was carried out in accordance with the instruction manual.

First, cells in the verapamil(-) sample were analyzed, and cells in a cell group (side population (SP)) having low emission intensity compared with a main cell group (main population (MP)) were detected (FIG. 1). In order to confirm that SP cells had low Hoechst 33342 dye stainability specific to an ABC transporter, the verapamil(+) sample was analyzed under the same conditions, and it was confirmed that SP cells disappeared (FIG. 1).

The SP cells were isolated, the cells were subjected to centrifuging at 4°C and 1500 rpm for 15 minutes, the supernatant was removed, and a suspension in 100 to 200 μL of 1× PBS was then formed.

d) Subcloning at single cell level

SW480-derived SP cells were detected in c) above, and the SP and MP cell fractions were each subjected to single cell sorting to give 1 cell/well in a 96 well plate (FIG. 2-1). Each well was previously charged with 1% penicillin/streptomycin-containing 10% FCS-supplemented DMEM medium.

After culturing for 2 to 3 weeks, the cell lines proliferating in the wells were defined as an SW480-SP clone cell line or an SW480-MP clone cell line. X and Y in 'SW480-SP-X' or 'SW480-MP-Y' denote clone number.

When the morphology was examined using a confocal microscope, the MP clone cell lines mainly proliferated as a single layer, and each cell showed a spindle shape. On the other hand, the SP clone cell lines showed a tendency for multi-layering, and each cell showed a circular to oval shape. Representative microscopic images of the SP clones and the MP clones obtained are shown in FIG. 2-2.

Experimental Example 2: Tumorigenicity experiment

In order to confirm the in vivo tumorigenicity of each of the SW480-SP and SW480-MP clone cell lines obtained in Experimental Example 1, the SP clone and the MP clone were each transplanted to a NOD/SCID immunodeficient mouse (Oriental Kobo) using three representative clones thereof.

Specifically, the same number of SP and MP clone cells were suspended in 100 μL of 1× PBS on ice and mixed with 100 μL of Matrigel (BD). 100 μL of the cell Matrigel mixed solution was injected under the dorsal skin of a NOD/SCID mouse (Oriental Kobo) so as to give 100, 1000, and 10000 SP and MP clone cells for each group of five animals, and tumor development was examined. The major diameter and the minor diameter of a tumor were measured, and the tumor volume was calculated using the equation (volume = major diameter × minor diameter^2/2). A tumor growth curve of a mouse into which 10000 cells had been transplanted is shown in FIG. 3.

From the results, in the 10000 cell-transplanted groups, 8 weeks after cell inoculation in the SW480-MP clone transplanted group tumor development could not be observed at all. On the other hand, in the SW480-SP clone transplanted group tumor development was observed in all mice, and the volume of the tumor formed was significantly higher compared with the SW480-MP clone group (FIG. 3). This agrees with the opinion that cancer stem cells are a significant factor in the development of a tumor, and cancer stem cells are concentrated in SP clone cells (Kondo T, Setoguchi T, Taga T. Persistence of a small subpopulation of cancer stem-like cells in the C6 glioma cell line. Proc Natl Acad Sci U S A. 20: 781-786, 2004).

Experimental Example 3: Identification of HLA-A24-binding natural peptide in human colon cancer SP cells

Elution and sequence analysis of an HLA-A24-binding natural peptide specifically presented only on the SP fraction cells of the SW480 human colon cancer cell line were carried out by the procedure below.

a) Cell line

SW480-MP and SW480-SP cell lines, which were the colon cancer cell line-derived clones, were cultured in 10% FCS and 1% penicillin/streptomycin (Gibco)-containing DMEM medium so as to give a cell count in the range of $1.5 \times 10^9$ to $1.8 \times 10^9$.

b) Antibody

An anti-HLA-A24 antibody (C7709A2)-producing hybridoma was donated by Dr. P. G. Coulie (de Duve Institute, Brussel). The hybridoma was cultured in an RPMI-1640 (Sigma-Aldrich) medium to which 10% FCS, 1% penicillin/streptomycin, 55 μM 2-mercapto ethanol (Gibco), 1 mM sodium pyruvate (Gibco), 2 mM L-glutamine (Sigma-Aldrich), and 20 mM HEPES (Gibco) had been added, and a concentrated antibody was obtained from the culture supernatant by a reverse osmosis method using a cellulose tube and polyethylene glycol (PEG-20000). 0.03% sodium azide and a protease inhibitor cocktail (Roche Diagnostics) were added to the concentrated antibody, and it was stored at 4°C.

c) Binding of antibody and beads

30 to 40 mL of the concentrated antibody and 3 mL of protein A Sepharose beads (GE Healthcare) were stirred at 4°C overnight so as to bind them, and then washed with 0.1 M boric acid and 0.2 M triethanolamine buffer (pH 8.2). The antibody and the beads were covalently bonded by stirring in a 20 mM dimethyl pimelimidate dihydrochloride-containing triethanolamine buffer (pH 8.3) at room temperature for 60 to 90 minutes.

d) Immunoprecipitation of HLA-A24-binding peptide

Cells (SW480-SP and SW480-MP) of Experimental Example 3a) were dissolved in a buffer containing 0.5% NP-40, 50 mM Tris HCl (pH 8), 150 mM sodium chloride, and a protease inhibitor. The cell solution was subjected to stepwise centrifuging (10 minutes at 2000 g, 30 minutes at 38000 g, 90 minutes at 100000 g), and the supernatant was collected. The collected supernatant was passed through a 0.5 mL protein A Sepharose suspension column to thus remove components that nonspecifically bound to protein A Sepharose, and then mixed with the antibody-binding protein A Sepharose beads prepared in Experimental Example 3c) to thus bind a complex of a natural peptide and an HLA-A24 molecule to the antibody beads by slowly stirring at 4°C overnight.

Subsequently, the antibody beads were washed stepwise with four types of buffer ([1] 0.005% NP-40, 50 mM Tris HCl (pH 8.0), 150 mM sodium chloride, 5 mM EDTA, and protease inhibitor; [2] 50 mM Tris HCl (pH 8.0) and 150 mM sodium chloride; [3] 50 mM Tris HCl (pH 8.0) and 450 mM sodium chloride; and [4] 50 mM Tris HCl (pH 8.0)), and the peptide and the HLA-A24 molecule bound to the antibody were then eluted by treatment with 10% acetic acid. Subsequently, only the target peptide was extracted using a 3 kDa cutoff filter (Millipore). This peptide-containing extract was concentrated, dried, and then redissolved using 0.1% formic acid as a solvent, thus giving a sample.

e) Sequence analysis of eluted peptide

The sample obtained in Experimental Example 3d) was fractionated using a nanoflow HPLC (Kya Technologies Corporation), spotted on a MALDI substrate, and then analyzed using a mass spectrometer (Applied Biosystems; MDS SCIEX 4800 MALDI TOF/TOF). Mass spectrometry analysis and peptide sequence analysis employed Applied Biosystems 4000 Series Explorer software (ver. 3.5.3), ProteinPilot 3.0 software (Applied Biosystems), and the ipi.HUMAN FASTA protein database (ver. 3.71). Among the peptide sequences obtained and among those specific to SW480-SP, the sequence and analytical spectrum of a peptide derived from the FAM83B gene, which is described in Experimental Example 4 and thereafter, are shown in FIG. 4.

f) Discussion

Identification of HLA-A24-binding peptides was possible by a method in which immunoprecipitation using an anti-HLA-A24 antibody and mass spectrometry analysis were combined. These are thought to be natural peptides presented on the surface of colon cancer cells. Furthermore, analysis was carried out using the same method for the MP fraction cells, and by comparing the two, a natural peptide with an amino acid sequence described in SEQ ID No: 3 was identified as a natural peptide that is specifically subjected to antigen presentation on the SP fraction cells. Identification of these natural peptides was the first identification of a peptide specifically subjected to antigen presentation on SP fraction cells of a cancer.

Experimental Example 4: Expression of gene encoding HLA-A24-binding natural peptide

a) SP-specific gene expression

In Experimental Example 3e), a plurality of HLA-A24-binding natural peptides specific to the SP fraction cells were identified. These peptides are thought to be largely classified into two groups. They are a group for which a gene encoding the peptide is specifically expressed in the SP fraction cells and a group for which a gene encoding the peptide is expressed in both the SP fraction cells and the MP fraction cells, but due to differences in protein expression level or peptide processing, it is not subjected to antigen presentation by HLA-A24 as a natural peptide in the MP.

When, in order to classify the natural peptides identified above for the purpose of the above classification, mRNAs were extracted from SW480-SP and SW480-MP, and gene expression was examined by RT-PCR, the FAM83B gene was identified as one of genes specifically expressing in the SP fraction cell. The results of gene expression analysis are shown in FIG. 5. Extraction and reverse transcription of mRNA respectively employed TRIzol (Invitrogen) and SuperScript (registered trademark) III Reverse Transcriptase (Invitrogen) in accordance with the product package inserts. The primer and conditions for the thermal cycler used in RT-PCR are shown in Table 1. RT-PCR products were subjected to electrophoresis at 100V for 25 minutes using 1.5% agarose gel.

[Table 1]

FAM83B PCR Primer

Fw: 5'-GACCGATTTGAGGGCTATGA-3'

Rv: 5'-ACATTCGTAGGGGTGTCTGG-3'

PCR conditions

| | | |
|---|---|---|
| 94°C | 2 min | |
| 94°C | 15 sec | 40 cycles |
| 60°C | 30 sec | 40 cycles |
| 72°C | 30 sec | 40 cycles |
| 72°C | 2 min | |
| 4°C | - | |

b) FAM83B gene expression in normal cells

The FAM83B identified in Experimental Example 4a) was subjected to examination of expression in human adult normal cells. A human adult normal tissue-derived mRNA panel was obtained from Clontech, and RT-PCR was carried out using this. The mRNA panel includes mRNAs derived from adult normal cells and tissues from the heart, brain, placenta, lung, liver, skeletal muscle, kidney, pancreas, spleen, thymus, prostate, testis, ovary, small intestine, large intestine, and peripheral blood mononuclear cells.

First, cDNA was synthesized from mRNA using SuperScript (registered trademark) III reverse transcription enzyme (Invitrogen) in accordance with the kit protocol. With regard to the cDNA thus synthesized, FAM83B cDNA was amplified by means of RT-PCR using a forward (Fw) primer and a reverse (Rv) primer (Table 1). As a control, GAPDH cDNA was amplified by the same method. The PCR conditions are shown in Table 1. The amplification product thus amplified was subjected to electrophoresis at 100V for 25 minutes using 1.5% agarose gel. The results are shown in FIG. 6.

c) FAM83B gene expression in cancer cell lines

FAM83B gene expression in four types of colon cancer cell lines (HT15, HCT116, HT29, Colo205), two types of breast cancer cell lines (MCF7, HMC1), two types of oral cancer cell lines (HSC3, HSC4), one type of lung cancer cell line (LHK2), one type of pancreatic cancer cell line (Panc1), and two types of melanoma cell lines (1102mel, LG2mel) was confirmed by the same method as in Experimental Example 4b). The results are shown in FIG. 7.

d) Discussion

Among the genes encoding proteins from which the eight types of HLA-A24-binding natural peptides obtained in Experimental Example 3e) were derived, only FAM83B showed an SP-specific expression pattern. The FAM83B gene has been reported as a novel oncogene but is not known for SP-specific gene expression. Furthermore, expression of the FAM83B gene was not observed in normal cells of any of the organs, whereas expression in many cancer cell lines was confirmed (FIG. 6 and FIG. 7). That is, the FAM83B gene and the peptide as a product thereof are thought to have ideal qualities as cancer treatment targets.

<u>Experimental Example 5: Induction of cytotoxic T cells (CTL)</u>

a) Separation of human peripheral blood mononuclear cells (PBMC)

Peripheral blood was collected using a heparin-containing 50 mL syringe from HLA-A24-positive colon cancer patients and HLA-A24-positive healthy controls who had given informed consent. The whole blood was layered in a 50 mL tube (Falcon) to which 13 mL of Lymphoprep (Nycomed) had been added, and subjected to centrifugation at 2000 rpm for 30 minutes. A PBMC layer precipitated on the Lymphoprep layer was recovered using a pipette and washed three times with PBS, thus giving human PBMC.

b) Separation of CD8 positive cells (CD8+) and CD8 negative cells (CD8-)

The PBMC thus separated was suspended in 10 mL of AIM-V culture medium (Life Technologies) and cultured in a 10 cm plastic dish at 37°C for about 2 hours. The 10 cm dish was gently shaken, floating cells were recovered together with the AIM-V culture medium, and centrifugation was carried out in a 15 mL tube at 1500 rpm for 5 minutes. A pellet thus obtained was suspended in 160 μL of 2 mM EDTA-containing 0.1% BSA-supplemented PBS, 40 μL of CD8 micro beads (Miltenyi Biotec) were added and mixed, culturing was then carried out at 4°C for 15 minutes, washing with 5 mL of 2 mM EDTA-containing 0.1% BSA-supplemented PBS was carried out, and centrifugation at 1500 rpm for 5 minutes was carried out. 1 mL of 2 mM EDTA-containing 0.1% BSA-supplemented PBS was added to and mixed with the pellet, a magnet-equipped column was loaded with the mixture, washing with 2 mM EDTA-containing 0.1% BSA-supplemented PBS was carried out five times, the column was then detached from the magnet, and the CD8+ cells were recovered. Cells that did not become attached to the column were defined as CD8- cells.

c) Stimulation of $CD8^+$ cells with synthetic peptide

The $CD8^-$ cells and the CD8+ cells were cultured in a 10% human AB serum (HS)-containing AIM-V culture medium. 1 mg/mL phytohemagglutinin (PHA) (WAKO chemicals) and 100 U/mL interleukin 2 (IL-2) (Takeda Chemical Industries, Ltd.) were added to some of the $CD8^-$ cells, and the mixture was cultured for 7 days, thus preparing PHA-blast cells. The PHA-blast cells were mixed with 20 μg/mL of a synthetic peptide with a FAM83B-derived sequence (SEQ ID No: 3) identified in Experimental Example 3e) and cultured at room temperature for 1 hour. The peptide pulsed PHA-blast cells were irradiated with 100 Gy using an irradiation machine (Softex), 10 mL of PBS was added, and centrifugation was then carried out at 1500 rpm for 5 minutes. A pellet was suspended in 1 mL of 10% HS-containing AMI-V, and the cell concentration was calculated. $2 \times 10^6$ $CD8^+$ cells were added to $4 \times 10^5$ PHA-blast cells, and the mixture was cultured in 1 mL of AIM-V containing 10%HS for 1 week at 37°C. On the 7th day, PHA-blast cells that had been peptide-pulsed in the same manner were irradiated with 100 Gy of radiation and added to the CD8+ cells. On the 8th day, 20 U/mL IL-2 was added to the CD8+ cells. Stimulation with PHA-blast cells was carried out in the same manner on the 14th day.

Experimental Example 6: Interferon (IFN)-γ ELISPOT assay

a) Preparation of ELISPOT plate

An experiment was carried out using a Human IFNγ ELISPOT set (BD). An ELISPOT plate was coated with anti-IFNy antibodies, which had been diluted by 200 times, and allowed to stand at 4°C overnight. The plate was cultured in 10% FCS-supplemented RPMI (Sigma-Aldrich) at room temperature for 2 hours and blocking was carried out, thus giving an ELISPOT plate.

b) Cell culturing

T2-A24 cells (donated by Dr. Kuzushima, Cancer Center, Aichi prefecture), which are of a cell line expressed by transferring the HLA-A2402 gene to human lymphoblastoid T2 cells, were pulsed with each peptide at a concentration of 20 μg/mL at room temperature for 1 hour. With regard to the peptide pulse groups there were three groups, that is, [1] no peptide pulse, [2] HIV peptide pulse, and [3] FAM83B peptide pulse. PBS was added subsequent to the peptide pulse, and centrifugation was carried out at 1500 rpm for 5 minutes. A cell pellet was suspended to give $5 \times 10^5$ cells/mL, and an ELISPOT plate was plated with $5 \times 10^4$ cells per well. CTLs were plated at $5 \times 10^4$ cells per well and cultured at 37°C overnight.

c) Detection of spot

The culture medium and the cells were removed from the ELISPOT plate that had been cultured overnight, and the ELISPOT plate was washed twice with Milli Q water and three times with wash buffer. Biotinylated detection antibody diluted by 250 times was added to each well, and culturing was carried out at room temperature for 2 hours. After washing three times with wash buffer, HRP-labeled streptavidin diluted by 100 times was added to each well, and culturing was carried out at room temperature for 1 hour. After washing three times with wash buffer and washing twice with PBS, a coloring reagent was added to each well, and a coloring reaction was carried out at room temperature for 15 to 30 minutes. After sufficient visible spot formation was detected, washing with Milli Q water was carried out, and the reaction was thus completed. A nitrocellulose film was dried and then subjected to detection and imaging by KS ELISPOT (ZEISS). As shown in FIG. 8, an IFNγ spot was detected in the FAM83B peptide pulse group.

Experimental Example 7: Cytotoxicity test

a) Calcein staining

T2-A24 cells and K562 HLA-class I-deficient leukemia cells (obtained from ATCC) were suspended in 10% FBS-supplemented RPMI at a cell concentration of $1 \times 10^6$ cells/mL. Calcein (obtained from Dojindo) was added so as to give a final concentration of 10 μg/mL, and culturing was carried out at 37°C for 30 minutes. Washing was carried out three times with 10 mL of 10% FBS-supplemented RPMI.

b) Coculturing with CTLs

T2-A24 cells that had been stained with calcein were pulsed with each peptide at a concentration of 20 μg/mL at room temperature for 1 hour. With regard to the peptide pulse group there were three groups, that is, [1] no peptide pulse, [2] HIV peptide pulse, and [3] FAM83B peptide pulse. Subsequent to the peptide pulse, washing was carried out twice with PBS. Each cell group was plated to each well at $1 \times 10^4$ cells/50 μL. The fluorescence intensity (0 hours) of the cells plated to each well was measured using a Terascan (Minerva Tech K.K.).

After the 0 hours measurement, numbers of effector cells (CTLs) were plated to each well so that the effector/target ratio (E/T ratio) became 3, 10, and 30. Human PBMC inactivated at 80°C was plated as a spontaneous release well. 2% NP-40-containing PBS was added in a maximum release well. After culturing at 37°C for 4 hours to 6 hours, the fluorescence intensity of each well was measured using a Terascan. The cytotoxicity was calculated using the equation below.

Cytotoxicity = (release amount of experimental group - spontaneous release amount of experimental group)/(maximum release amount of experimental group - spontaneous release amount of experimental group) × 100

As shown in FIG. 9, the CTLs showed high cytotoxicity toward [3] FAM83B peptide pulse group compared with [1] no peptide pulse group, [2] HIV peptide pulse group, and K562. This suggests that the CTLs showed specific cytotoxicity toward the FAM83B peptide.

Experimental Example 8: FAM83B-derived peptide having HLA-A*02:01 and HLA-A*24:02 binding motif

FAM83B-derived peptides (peptides described in SEQ ID No: 3 to 35, 37 to 41, 43 to 60, and 65 to 85) for which binding to HLA-A*02:01 and/or HLA-A*24:02 is predicted were extracted using BIMAS (http://www-bimas.cit.nih.gov/molbio/hla_bind/), SYFPEITHI (http://www.syfpeithi.de/), and IEDB (MHC-I processing predictions; http://www.iedb.org/), etc. which are programs for predicting binding between an MHC and a peptide. These peptides were chemically synthesized by the Fmoc method. The peptides thus synthesized are shown in Tables 2-1 and 2-2. Start denotes the amino acid position in FAM83B (SEQ ID No: 2) of the N terminal amino acid of the synthesized peptide, and End denotes the amino acid position in FAM83B (SEQ ID No: 2) of the C terminal amino acid of the synthesized peptide. Length denotes the number of amino acids of the synthesized peptide.

[Table 2-1]

| Peptide | | | | | |
|---|---|---|---|---|---|
| Name | Sequence number | Start | End | Length | Sequence |
| SF9 | 3 | 988 | 996 | 9 | SYQPNENKF |
| F23_10 | 4 | 23 | 32 | 10 | HYKEWYRVAI |
| F27_10 | 5 | 27 | 36 | 10 | WYRVAIDILI |
| F107_10 | 6 | 107 | 116 | 10 | GWPYVMPGLL |
| F124_10 | 7 | 124 | 133 | 10 | LFHPPRAHLL |
| F141_10 | 8 | 141 | 150 | 10 | KMIKEARKVI |
| F176_10 | 9 | 176 | 185 | 10 | VYILLDESNF |
| F214_10 | 10 | 214 | 223 | 10 | DYLSKTGAKF |
| F222_10 | 11 | 222 | 231 | 10 | KFHGKMEQKF |
| F245_11 | 12 | 245 | 255 | 11 | SYMWSFEKAHL |
| F268_9 | 13 | 268 | 276 | 9 | SFDEEFRTL |
| F276_11 | 14 | 276 | 286 | 11 | LYARSCVPSSF |
| F305_9 | 15 | 305 | 313 | 9 | TYQHSVSSL |
| F335_11 | 16 | 335 | 345 | 11 | SYFKNRGIYTL |
| F357_10 | 17 | 357 | 366 | 10 | GYKPHFVPNF |
| F398_9 | 18 | 398 | 406 | 9 | PYLLLNRAL |
| F466_11 | 19 | 466 | 476 | 11 | SFNGTDNHIRF |
| F475_10 | 20 | 475 | 484 | 10 | RFLQQRMPTL |
| F667_9 | 21 | 667 | 675 | 9 | LFDNSKANL |
| F667_9 | 21 | 667 | 675 | 9 | LFDNSKANL |
| F766_10 | 22 | 766 | 775 | 10 | SFLKKGSQKL |

(continued)

| Peptide | | | | | |
|---|---|---|---|---|---|
| Name | Sequence number | Start | End | Length | Sequence |
| F766_10 | 22 | 766 | 775 | 10 | SFLKKGSQKL |
| F939_9 | 23 | 939 | 947 | 9 | PFCKIESSI |
| F971_9 | 24 | 971 | 979 | 9 | SYGRSSPLL |
| F995_9 | 25 | 995 | 1003 | 9 | KFRGFMQKF |
| F998_10 | 26 | 998 | 1007 | 10 | GFMQKFGNFI |
| F39_10 | 27 | 39 | 48 | 10 | GLEAYQEFLV |
| F46_11 | 28 | 46 | 56 | 11 | FLVQERVSDFL |
| F55_11 | 29 | 55 | 65 | 11 | FLAEEEINYIL |
| F63_9 | 30 | 63 | 71 | 9 | YILKNVQKV |
| F86_10 | 31 | 86 | 95 | 10 | TLSSGTYWPV |
| F103_9 | 32 | 103 | 111 | 9 | NLDLGWPYV |
| F114_11 | 33 | 114 | 124 | 11 | GLLGGTHIDLL |
| F123_11 | 34 | 123 | 133 | 11 | LLFHPPRAHLL |
| F131_9 | 35 | 131 | 139 | 9 | HLLTIKETI |
| F151_10 | 37 | 151 | 160 | 10 | ALVMDIFTDV |
| F179_11 | 38 | 179 | 189 | 11 | LLDESNFNHFL |
| F190_10 | 39 | 190 | 199 | 10 | NMTEKQGCSV |
| F201_10 | 40 | 201 | 210 | 10 | RLRNIRVRTV |

[Table 2-2]

| Peptide | | | | | |
|---|---|---|---|---|---|
| Name | Sequence number | Start | End | Length | Sequence |
| F226_9 | 41 | 226 | 234 | 9 | KMEQKFLLV |
| F321_9 | 43 | 321 | 329 | 9 | NLFGRQDKI |
| F344_10 | 44 | 344 | 353 | 10 | TLNEHDKYNI |
| F447_10 | 45 | 447 | 456 | 10 | RLAQRKTTNL |
| F455_9 | 46 | 455 | 463 | 9 | NLADRNSNV |
| F476_9 | 47 | 476 | 484 | 9 | FLQQRMPTL |
| F491_11 | 48 | 491 | 501 | 11 | FLRNWRIESYL |
| F594_9 | 49 | 594 | 602 | 9 | PLPESIPKL |
| F613_10 | 50 | 613 | 622 | 10 | TLQVPENHSV |
| F658_10 | 51 | 658 | 667 | 10 | NLLKRRSFPL |
| F666_10 | 52 | 666 | 675 | 10 | PLFDNSKANL |
| F709_9 | 53 | 709 | 717 | 9 | SMHNVTHNL |
| F716_9 | 54 | 716 | 724 | 9 | NLEEDEEEV |
| F767_9 | 55 | 767 | 775 | 9 | FLKKGSQKL |

(continued)

| Peptide | | | | | |
|---|---|---|---|---|---|
| Name | Sequence number | Start | End | Length | Sequence |
| F780_10 | 56 | 780 | 789 | 10 | SLTPDKKENL |
| F799_10 | 57 | 799 | 808 | 10 | RLCSSSDTLV |
| F999_9 | 58 | 999 | 1007 | 9 | FMQKFGNFI |
| F238_10 | 59 | 238 | 247 | 10 | KVMYGSYSYM |
| F256_11 | 60 | 256 | 266 | 11 | SMVQIITGQLV |
| F62_10 | 65 | 62 | 71 | 10 | NYILKNVQKV |
| F148_9 | 66 | 148 | 156 | 9 | KVIALVMDI |
| F239_12 | 67 | 239 | 250 | 12 | VMYGSYSYMWSF |
| F305_12 | 68 | 305 | 316 | 12 | TYQHSVSSLASV |
| F38_11 | 69 | 38 | 48 | 11 | HGLEAYQEFLV |
| F593_10 | 70 | 593 | 602 | 10 | KPLPESIPKL |
| F55_10 | 71 | 55 | 64 | 10 | FLAEEEINYI |
| F231_10 | 72 | 231 | 240 | 10 | FLLVDCQKVM |
| F257_10 | 73 | 257 | 266 | 10 | MVQIITGQLV |
| F18_10 | 74 | 18 | 27 | 10 | NYIEPHYKEW |
| F47_10 | 75 | 47 | 56 | 10 | LVQERVSDFL |
| F148_10 | 76 | 148 | 157 | 10 | KVIALVMDIF |
| F149_10 | 77 | 149 | 158 | 10 | VIALVMDIFT |
| F153_11 | 78 | 153 | 163 | 11 | VMDIFTDVDIF |
| F238_13 | 79 | 238 | 250 | 13 | KVMYGSYSYMWSF |
| F239_10 | 80 | 239 | 248 | 10 | VMYGSYSYMW |
| F123_10 | 81 | 123 | 132 | 10 | LLFHPPRAHL |
| F708_10 | 82 | 708 | 717 | 10 | KSMHNVTHNL |
| F736_9 | 83 | 736 | 744 | 9 | KSVSIAALL |
| F932_9 | 84 | 932 | 940 | 9 | RVYSRFEPF |
| F980_10 | 85 | 980 | 989 | 10 | NYNTGVYRSY |

Example 9: Evaluation of binding of FAM83B-derived peptide to HLA-A*02:01 or HLA-A*24:02

Evaluation of the binding of a FAM83B-derived peptide to each HLA molecule was carried out by an MHC class I expression stabilization test. In this test, T2-A24 cells, which are a human lymphoblastoid cell line, were used. T2 cells are deficient in the transporter associated with antigen processing (TAP), which is involved in the transport of a peptide from the cytoplasm to the endoplasmic reticulum. It is known that an MHC class I molecule (HLA-A*02:01 and HLA-A*24:02) has an unstable structure in a state in which a peptide is not bound (empty MHC class I). T2 cells can usually only express a low level of empty MHC class I molecules on the cell surface. However, when a peptide that can bind to the MHC class I molecule is added, the empty MHC class I molecule binds to the peptide and can be present on the cell surface in a stable manner. Therefore, the cell surface MHC class I expression level depends on the MHC class I binding affinity of a peptide.

The T2-A24 cells were subcultured at 37°C under 5% $CO_2$. With regard to peptides, a synthesized FAM83B-derived peptide, Melan A A27L peptide (amino acid sequence: ELAGIGILTV; SEQ ID No: 61) as an HLA-A02-positive control, $HIV_{584-592}$ peptide (amino acid sequence: RYLRDQQLL; SEQ ID No: 62) as an HLA-A24-positive control, MAGE-1161-169 peptide (amino acid sequence: EADPTGHSY; SEQ ID No: 63) as an HLA-A02-negative control, and $VSV_{52-59}$ peptide (amino acid sequence: RGYVYQGL; SEQ ID No: 64) as an HLA-A24-negative control were each evaluated in terms of binding at a concentration of 100 μg/mL. These peptides were dissolved in DMSO and further diluted by 200 times with RPMI 1640 medium. A cell suspension and the peptide solution were mixed and cultured under conditions of 5% $CO_2$ and 26°C for 16 to 18 hours. Coculturing was carried out at a temperature of 37°C for a further 3 hours, the supernatant was then removed by centrifuging, and cells were isolated. The isolated cells were washed with 3% FBS-containing PBS, an anti-HLA-A02 antibody (clone: BB7.2; Medical & Biological Laboratories Co., Ltd.) or anti-HLA-A24 antibody (clone: 17A10; Medical & Biological Laboratories Co., Ltd.) fluorescently-labeled with FITC was added, and the mixture was allowed to stand at room temperature for 30 minutes. Subsequently, the cells were washed with 3% FBS-containing PBS, 4% paraformaldehyde phosphate buffer was added, and the mixture was allowed to stand at room temperature for 10 minutes to thus fix the cells. The fixed cells were subjected to measurement of FITC fluorescence intensity by a flow cytometer (FACScan). The mean fluorescence intensity (MFI) was calculated as a solvent ratio.

The results of the HLA-binding test are shown in Table 3. As shown in Table 3, the MFI of peptides described in SEQ ID No: 4, SEQ ID No: 12, SEQ ID No: 20, SEQ ID Nos: 29 to 33, SEQ ID No: 35, SEQ ID No: 37, SEQ ID No: 38, SEQ ID No: 47, SEQ ID No: 57, SEQ ID No: 60, and SEQ ID Nos: 65 to 73 with respect to HLA-A*02:01 was at least 1.5, the MFI of peptides described in SEQ ID Nos: 3 to 6, SEQ ID No: 9, SEQ ID No: 12, SEQ ID Nos: 14 to 16, SEQ ID No: 19, SEQ ID No: 20, SEQ ID No: 23, SEQ ID No: 24, SEQ ID No: 28, SEQ ID No: 35, SEQ ID No: 47, SEQ ID No: 49, SEQ ID Nos: 65 to 70, and SEQ ID Nos: 74 to 80 with respect to HLA-A*24:02 was at least 1.5, and the MFI of peptides described in SEQ ID No: 4, SEQ ID No: 12, SEQ ID No: 20, SEQ ID No: 35, SEQ ID No: 47, and SEQ ID Nos: 65 to 70 with respect to both HLA-A*02:01 and HLA-A*24:02 was at least 1.5.

[Table 3]

| Peptide | | HLA-A*02:01 | | HLA-A*24:02 | | Evaluation results | |
|---|---|---|---|---|---|---|---|
| Name | Sequence number | Positive control | Negative control | Positive control | Negative control | HLA-A0201 | HLA-A2402 |
| SF9 | 3 | 3.3 | 1.0 | 2.7 | 1.0 | 1.0 | 2.3 |
| F23_10 | 4 | 3.5 | 1.1 | 3.8 | 1.0 | 2.0 | 3.0 |
| F27_10 | 5 | 3.5 | 1.1 | 3.8 | 1.0 | 1.2 | 3.2 |
| F107_10 | 6 | 3.7 | 1.0 | 2.3 | 1.1 | 1.0 | 2.7 |
| F176_10 | 9 | 3.5 | 1.1 | 3.8 | 1.0 | 1.2 | 2.7 |
| F245_11 | 12 | 3.6 | 1.0 | 2.4 | 1.0 | 1.5 | 2.9 |
| F276_11 | 14 | 3.5 | 1.1 | 3.8 | 1.0 | 1.2 | 3.4 |
| F305_9 | 15 | 3.5 | 1.1 | 3.8 | 1.0 | 1.1 | 2.4 |
| F335_11 | 16 | 3.5 | 1.1 | 3.8 | 1.0 | 1.0 | 2.3 |
| F466_11 | 19 | 3.7 | 1.0 | 2.3 | 1.1 | 1.0 | 1.7 |
| F475_10 | 20 | 3.6 | 1.0 | 2.4 | 1.0 | 1.9 | 3.0 |
| F939_9 | 23 | 3.7 | 1.0 | 2.3 | 1.1 | 0.9 | 2.7 |
| F971_9 | 24 | 3.5 | 1.1 | 3.8 | 1.0 | 1.1 | 1.8 |
| F46_11 | 28 | 3.3 | 1.0 | 2.7 | 1.0 | 1.4 | 1.6 |
| F55_11 | 29 | 3.7 | 1.0 | 2.7 | 1.1 | 3.6 | 1.0 |
| F63_9 | 30 | 3.7 | 1.0 | 3.0 | 1.0 | 3.3 | 1.0 |
| F86_10 | 31 | 3.7 | 1.0 | 2.7 | 1.1 | 3.0 | 1.0 |
| F103_9 | 32 | 3.7 | 1.0 | 2.7 | 1.1 | 2.6 | 1.1 |
| F114_11 | 33 | 3.3 | 1.0 | 2.7 | 1.0 | 2.9 | 1.1 |
| F131_9 | 35 | 3.7 | 1.0 | 2.7 | 1.1 | 1.5 | 2.4 |
| F151_10 | 37 | 3.7 | 1.0 | 2.7 | 1.1 | 3.4 | 1.0 |
| F179_11 | 38 | 3.3 | 1.0 | 2.7 | 1.0 | 1.6 | 1.1 |

(continued)

| Peptide | | HLA-A*02:01 | | HLA-A*24:02 | | Evaluation results | |
|---|---|---|---|---|---|---|---|
| Name | Sequence number | Positive control | Negative control | Positive control | Negative control | HLA-A0201 | HLA-A2402 |
| F476_9 | 47 | 3.7 | 1.0 | 3.0 | 1.0 | 2.0 | 2.6 |
| F594_9 | 49 | 3.7 | 1.0 | 3.0 | 1.0 | 1.4 | 3.8 |
| F799_10 | 57 | 3.7 | 1.0 | 2.3 | 1.1 | 2.1 | 1.2 |
| F256_11 | 60 | 3.3 | 1.0 | 2.7 | 1.0 | 3.2 | 1.1 |
| F62_10 | 65 | 3.6 | 1.0 | 2.4 | 1.0 | 2.9 | 1.6 |
| F148_9 | 66 | 3.7 | 1.0 | 3.0 | 1.0 | 2.4 | 1.6 |
| F239_12 | 67 | 3.5 | 1.1 | 3.8 | 1.0 | 1.6 | 4.0 |
| F305_12 | 68 | 3.3 | 1.0 | 2.7 | 1.0 | 2.0 | 3.1 |
| F38_11 | 69 | 3.3 | 1.0 | 2.7 | 1.0 | 2.5 | 3.1 |
| F593_10 | 70 | 3.6 | 1.0 | 2.4 | 1.0 | 1.9 | 3.5 |
| F55_10 | 71 | 3.7 | 1.0 | 3.0 | 1.0 | 3.5 | 1.2 |
| F231_10 | 72 | 3.7 | 1.0 | 2.7 | 1.1 | 2.0 | 1.0 |
| F257_10 | 73 | 3.7 | 1.0 | 3.0 | 1.0 | 1.9 | 1.1 |
| F18_10 | 74 | 3.5 | 1.1 | 3.8 | 1.0 | 1.0 | 3.2 |
| F47_10 | 75 | 3.7 | 1.0 | 3.0 | 1.0 | 1.1 | 1.5 |
| F148_10 | 76 | 3.7 | 1.0 | 3.0 | 1.0 | 1.1 | 2.6 |
| F149_10 | 77 | 3.7 | 1.0 | 3.0 | 1.0 | 1.3 | 2.1 |
| F153_11 | 78 | 3.3 | 1.0 | 2.7 | 1.0 | 0.9 | 2.0 |
| F238_13 | 79 | 3.3 | 1.0 | 2.7 | 1.0 | 1.2 | 5.1 |
| F239_10 | 80 | 3.5 | 1.1 | 3.8 | 1.0 | 1.1 | 2.2 |

Example 10: Evaluation of in vivo CTL inducibility using HLA-A*02:01 transgenic mouse and HLA-A*24:02 transgenic mouse

The CTL inducibility of a FAM83B-derived peptide that had an MFI with respect to HLA-A*02:01 or HLA-A*24:02 of at least 1.5 in Example 9 was evaluated by an in vivo CTL induction test using an HLA-A*02:01 transgenic mouse or an HLA-A*24:02 transgenic mouse.

An HLA-A*02:01 transgenic mouse (C57BL/6CrHLA-A2.1DR1) is a mouse that is deficient in mouse MHC and expresses HLA-A*02:01 and HLA-DRB1*01:01, which are human MHCs, and the use of this mouse enables a peptide that can induce CTLs in humans to be selected. Furthermore, an HLA-A*24:02 transgenic mouse is a mouse that expresses HLA-A*24:02, which is a human MHC, and the use of this mouse enables a peptide that can induce CTLs in a human to be selected. Whether or not each peptide has an activity in inducing CTLs was determined by whether or not T cells that can react with the administered peptide are induced by administering the peptide to the mouse.

Specifically, it was carried as follows. First, the peptide was dissolved in dimethyl sulfoxide at 80 mg/mL, then diluted with water for injection, and mixed with an equal part of incomplete Freund's adjuvant (IFA or ISA51VG), thus forming an emulsion. The peptide thus emulsified was administered to the mouse tail base intradermally at two locations at a dose of 250 $\mu$g/location. One week after that, the mouse was euthanized with $CO_2$ gas, the spleen was removed, and spleen cells were prepared. For measurement of IFN$\gamma$ production, an IFN$\gamma$ ELISPOT assay kit (Becton, Dickinson and Company) was used. On the day before preparing the spleen cells, an ELISPOT plate was treated with an anti-IFNy antibody, and on the day it was blocked with 10% FBS-containing RPMI 1640 medium. The spleen cells prepared were plated on the blocked ELISPOT plate at 0.25 to 1.0 $\times$ $10^6$ cells/well. The administered FAM83B-derived peptide was dissolved in DMSO at 40 mg/mL and further diluted with 10% RPMI 1640 medium to 20 $\mu$g/mL. The diluted peptide was added at 50 $\mu$L/well to the spleen cells derived from the animal to which the peptide had been administered. In vitro peptide restimulation was applied by culturing the spleen cells to which the peptide was added under 5% $CO_2$ at 37°C for 16 to 18 hours. After culturing, the supernatant was removed, and the ELISPOT plate was subjected to color development in accordance with the included protocol. The number of color developed spots was measured by

KS-ELISPOT.

The results of the IFNγ ELISPOT assay are shown in FIG. 10 to FIG. 19.

It can be seen from the results of this test that, by confirming IFNγ production specific to the peptide in the HLA-A*02:01 transgenic mouse-derived spleen cells, the FAM83B-derived peptides represented by SEQ ID Nos: 4, 20, 29 to 33, 35, 37, 38, 47, 57, 60, 65 to 68, and 71 to 73 had CTL inducibility. Furthermore, it can be seen that, by confirming IFNγ production specific to the peptide in the HLA-A*24:02 transgenic mouse-derived spleen cells, the FAM83B-derived peptides represented by SEQ ID Nos: 4, 6, 9, 12, 14, 15, 20, 23, 24, 47, 65 to 68, 74, 75, and 77 to 80 had CTL inducibility. Therefore, it was shown that each FAM83B-derived peptide described in SEQ ID Nos: 4, 20, 47, and 65 to 68 had CTL inducibility in both HLA-A02 type and HLA-A24 type subjects.

Example 11: HLA-A24 tetramer assay

a) Separation of human peripheral blood mononuclear cells (PBMC)

Peripheral blood was collected using a heparin-containing 50 mL syringe from HLA-A24-positive cancer patients who had given informed consent. The whole blood was layered in a Leucosep lymphocyte separation tube 50 ML (Greiner) to which 15 mL of Lymphoprep (Axis-Shield PoC AS) had been added, and subjected to centrifugation at 1000 g for 30 minutes. A PBMC layer precipitated on the Lymphoprep layer was recovered using a pipette and washed three times with PBS, thus giving human PBMCs.

b) MLPC

The PBMCs separated as above were suspended in 2 mL of AIM-V culture medium (Life Technologies) and cultured on a 24 well plate at 37°C for about 1 hour. Precipitation of lymphocyte was confirmed and 1 mL of the culture medium was discarded. 40 μg/mL of FAM83B (F476_9: SEQ ID No: 47) peptide was added, and the mixture was pipetted and then allowed to stand at room temperature for 30 minutes. 1 mL of the culture medium to which 50 U/mL interleukin 2 (IL-2) had been added was added to AIM-V containing 10% human AB serum (HS) warmed at 37°C, and the mixture was pipetted and then cultured at 37°C for 7 days.

c) Tetramer assay

The PBMCs that had been cultured for 7 days were recovered in a 15 mL tube (BD), an amount of 1× PBS that was twice the amount of the culture medium was added, and the mixture was centrifuged at 1500 rpm and room temperature for 5 minutes. The supernatant was discarded, 2 mL of a solution formed by diluting hemolysis Lysing Buffer (BD) by 10 times was added, and the mixture was pipetted and allowed to stand at room temperature for 15 minutes. Centrifuging was carried out at 1500 rpm and room temperature for 5 minutes, after the supernatant was removed 2 mL of 1× PBS was added again, and centrifuging was carried out again at 1500 rpm and room temperature for 5 minutes. The supernatant was discarded, 1 mL of 1× PBS to which 2% FBS (Thermo) had been added was added, and cell counting was carried out. 100 μL each of cells adjusted to give $10^5$ to $10^6$ cells/100 μL were poured into a total of six 5 mL FACS tubes (BD) so that there were four tubes for equipment adjustment, one tube for a negative control, and one tube for FAM83B tetramer. 5 μL each of HIV-PE and HIV-FITC tetramer (MBL) were added to the negative control tube, and 5 μL each of HIV-FITC and FAM83B (F476_9)-PE tetramer (MBL) were added to the FAM83B tetramer tube and incubated on ice for 1 hour while protecting from light. 3 μL each of CD8-PC5 (Beckman Coulter) was added to each of the tubes. Furthermore, with regard to the tubes for equipment adjustment, 3 μL each of CD8-FITC (Beckman Coulter), CD8-PE (BD), and CD8-PC5 were added to each tube. After the CD8 was added, incubation was carried out on ice for 1 hour while protecting from light. After incubation, 1 mL of 2% FBS-supplemented PBS was added to each tube, and centrifugation was carried out at 1500 rpm and 4°C for 5 minutes. This washing procedure was repeated once more, and 500 μL of a 1% formalin (Wako Pure Chemical Industries Ltd.)-containing PBS solution was added. The samples were analyzed using a FACS Calibur (BD).

The results are shown in FIG. 20. A significantly larger amount of FAM83B-PE tetramer positive cells were observed in both PBMCs harvested from patient A and PBMCs harvested from patient B compared with the number of HIV-PE tetramer positive cells, which was the negative control (patient A: 1.66%, patient B: 2.36%). This shows that CTLs were induced by adding F476_9 peptide to each of the patient-derived PBMCs and incubating, and the CTLs could be detected by the FAM83B-PE tetramer.

[Industrial Applicability]

In accordance with the present disclosure, a method for identifying a natural peptide that is actually subjected to antigen presentation on a cell is provided. Use of such a method contributes to the development of a highly effective cancer vaccine in which CTLs induced by the peptide vaccine reliably kill cancer cells. Furthermore, since it can be determined from a natural peptide specific to a cancer stem cell identified using such a method that FAM83B is specifically expressed in a cancer stem cell, it becomes possible to identify a cancer stem cell using FAM83B as a marker. Moreover, a gene-derived natural antigen peptide is useful as an agent for the prevention and/or treatment of a cancer having a large effect even with a small amount. The present invention provides a FAM83B-derived tumor antigen peptide having activity in inducing CTLs, etc. The peptide of the present invention is useful as an agent for the prevention and/or treatment of a cancer.
[Table of Sequences] PCT2674DN_ST25.txt

## Claims

1. FAM83B peptides consisting of amino acid sequences SEQ ID Nos: 3, 4, 6, 9, 12, 14, 15, 20, 23, 24, 29-33, 35, 37, 38, 47, 57, 60, 65-68, 71-75 and 77-80.

2. An HLA multimer comprising an HLA and any one of the FAM83B peptides according to Claim 1.

3. A T cell receptor-like antibody that recognizes a complex of an HLA and any one of the FAM83B peptides according to Claim 1.

4. A chimeric antigen receptor that recognizes a complex of an HLA and any one of the FAM83B peptides according to Claim 1.

5. A genetically modified T cell **characterized in that** the genetic modification is due to the introduction of the chimeric antigen receptor according to Claim 4 into a T cell.

6. An artificial CTL comprising a T cell receptor that recognizes a complex of an HLA and any one of the FAM83B peptides according to Claim 1.

7. A polynucleotide encoding at least one of the FAM83B peptides according to Claim 1.

8. A pharmaceutical composition for use in the prevention and/or treatment of a cancer comprising as an active ingredient any of (a) to (e) below:

(a) at least one of the FAM83B peptides according to Claim 1,
(b) the T cell receptor-like antibody according to Claim 3,
(c) the genetically modified T cell according to Claim 5,
(d) the artificial CTL according to Claim 6, and
(e) the polynucleotide according to Claim 7.

9. The pharmaceutical composition for use according to Claim 8; the pharmaceutical composition comprising as the active ingredient any of (a) to (e), and further comprising an adjuvant.

## Patentansprüche

1. FAM83B-Peptide, bestehend aus den Aminosäuresequenzen SEQ ID Nr.: 3, 4, 6, 9, 12, 14, 15, 20, 23, 24, 29-33, 35, 37, 38, 47, 57, 60, 65-68, 71-75 und 77-80.

2. HLA-Multimer, umfassend ein HLA und eines der FAM83B-Peptide nach Anspruch 1.

3. T-Zell-Rezeptor-ähnlicher Antikörper, der einen Komplex aus einem HLA und einem der FAM83B-Peptide nach Anspruch 1 erkennt.

4. Chimärer Antigenrezeptor, der einen Komplex aus einem HLA und einem der FAM83B-Peptide nach Anspruch 1 erkennt.

5. Genetisch veränderte T-Zelle, **dadurch gekennzeichnet, dass** die genetische Veränderung auf die Einführung des chimären Antigenrezeptors nach Anspruch 4 in eine T-Zelle zurückzuführen ist.

6. Künstliche CTL, umfassend einen T-Zell-Rezeptor, der einen Komplex aus einem HLA und einem der FAM83B-Peptide nach Anspruch 1 erkennt.

7. Polynukleotid, das wenigstens eines der FAM83B-Peptide nach Anspruch 1 codiert.

8. Pharmazeutische Zusammensetzung zur Verwendung bei der Vorbeugung und/oder Behandlung eines Krebses, umfassend als Wirkstoff eines von dem untenstehenden (a) bis (e):

   (a) wenigstens eines der FAM83B-Peptide nach Anspruch 1,
   (b) den T-Zell-Rezeptor-ähnlichen Antikörper T-Zelle nach Anspruch 3,
   (c) die genetisch veränderte T-Zelle nach Anspruch 5,
   (d) die künstliche CTL nach Anspruch 6, und
   (e) das Polynukleotid nach Anspruch 7.

9. Pharmazeutische Zusammensetzung zur Verwendung nach Anspruch 8; pharmazeutische Zusammensetzung, die als Wirkstoff eines von (a) bis (e) umfasst und ferner ein Adjuvans umfasst.

## Revendications

1. Peptides FAM83B constitués des séquences d'acides aminés SEQ ID N° : 3, 4, 6, 9, 12, 14, 15, 20, 23, 24, 29 à 33, 35, 37, 38, 47, 57, 60, 65 à 68, 71 à 75 et 77 à 80.

2. Multimère de HLA comprenant un HLA et l'un quelconque des peptides FAM83B selon la revendication 1.

3. Anticorps de type récepteur des lymphocytes T qui reconnaît un complexe d'un HLA et de l'un quelconque des peptides FAM83B selon la revendication 1.

4. Récepteur d'antigène chimérique qui reconnaît un complexe d'un HLA et de l'un quelconque des peptides FAM83B selon la revendication 1.

5. Lymphocyte T génétiquement modifié **caractérisé en ce que** la modification génétique est due à l'introduction du récepteur d'antigène chimérique selon la revendication 4 dans un lymphocyte T.

6. CTL artificiel comprenant un récepteur de lymphocyte T qui reconnaît un complexe d'un HLA et de l'un quelconque des peptides FAM83B selon la revendication 1.

7. Polynucléotide codant pour au moins l'un des peptides FAM83B selon la revendication 1.

8. Composition pharmaceutique pour une utilisation dans la prévention et/ou le traitement d'un cancer comprenant en tant que principe actif l'un quelconque de (a) à (e) ci-dessous :

   (a) au moins l'un des peptides FAM83B selon la revendication 1,
   (b) l'anticorps de type récepteur des lymphocytes T selon la revendication 3,
   (c) le lymphocyte T génétiquement modifié selon la revendication 5,
   (d) le CTL artificiel selon la revendication 6, et
   (e) le polynucléotide selon la revendication 7.

9. Composition pharmaceutique pour une utilisation selon la revendication 8 ; la composition pharmaceutique comprenant en tant que principe actif l'un quelconque de (a) à (e), et comprenant en outre un adjuvant.

FIG. 1

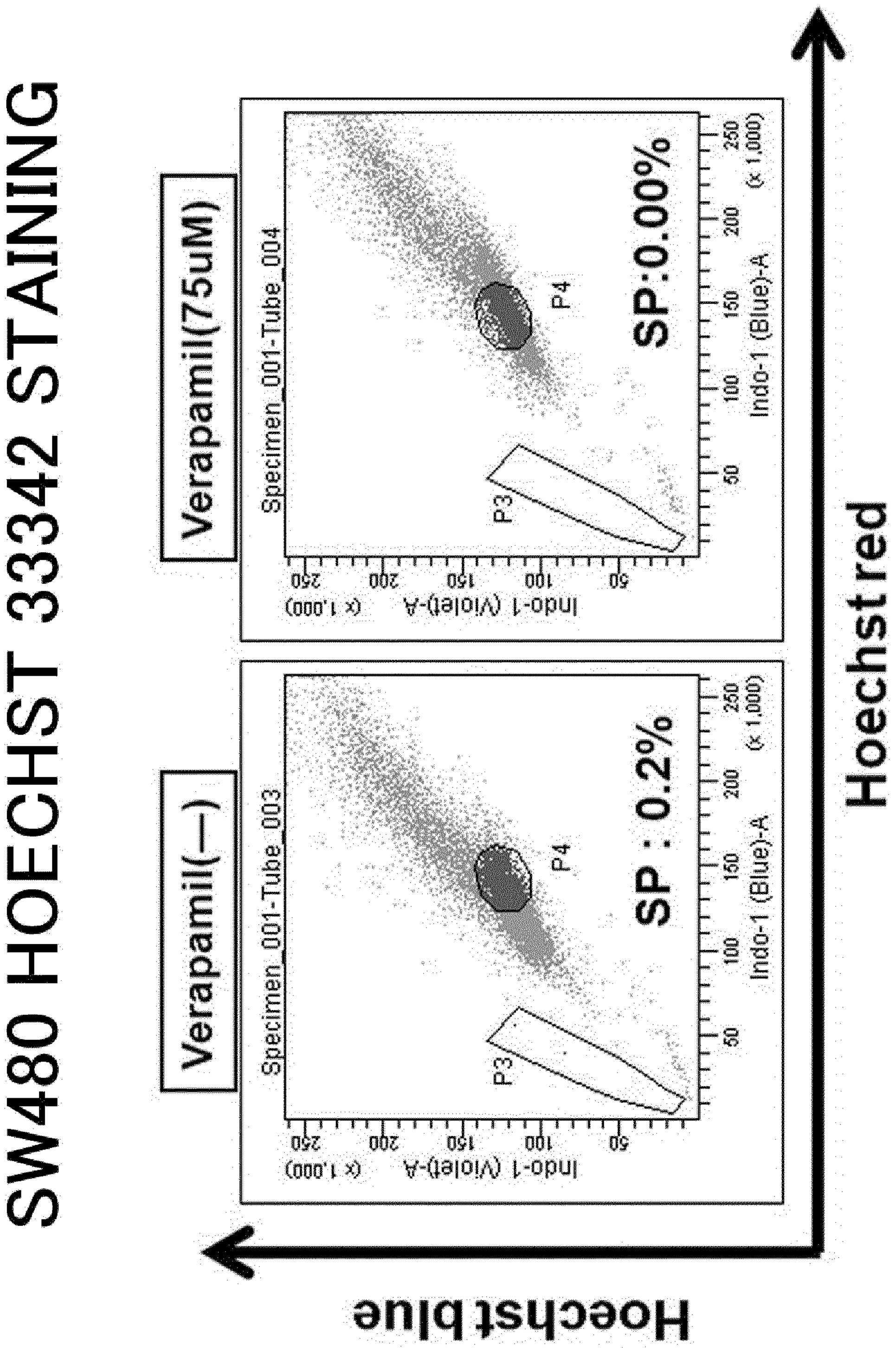
SW480 HOECHST 33342 STAINING
Verapamil(—)
Verapamil(75uM)
Specimen_001-Tube_003
Specimen_001-Tube_004
P3
P4
SP : 0.2%
SP:0.00%
Indo-1 (Violet)-A
(x 1,000)
Indo-1 (Blue)-A
(x 1,000)
Hoechst red
Hoechst blue

FIG. 2-1

SW480-SP AND -MP CELL CLONING

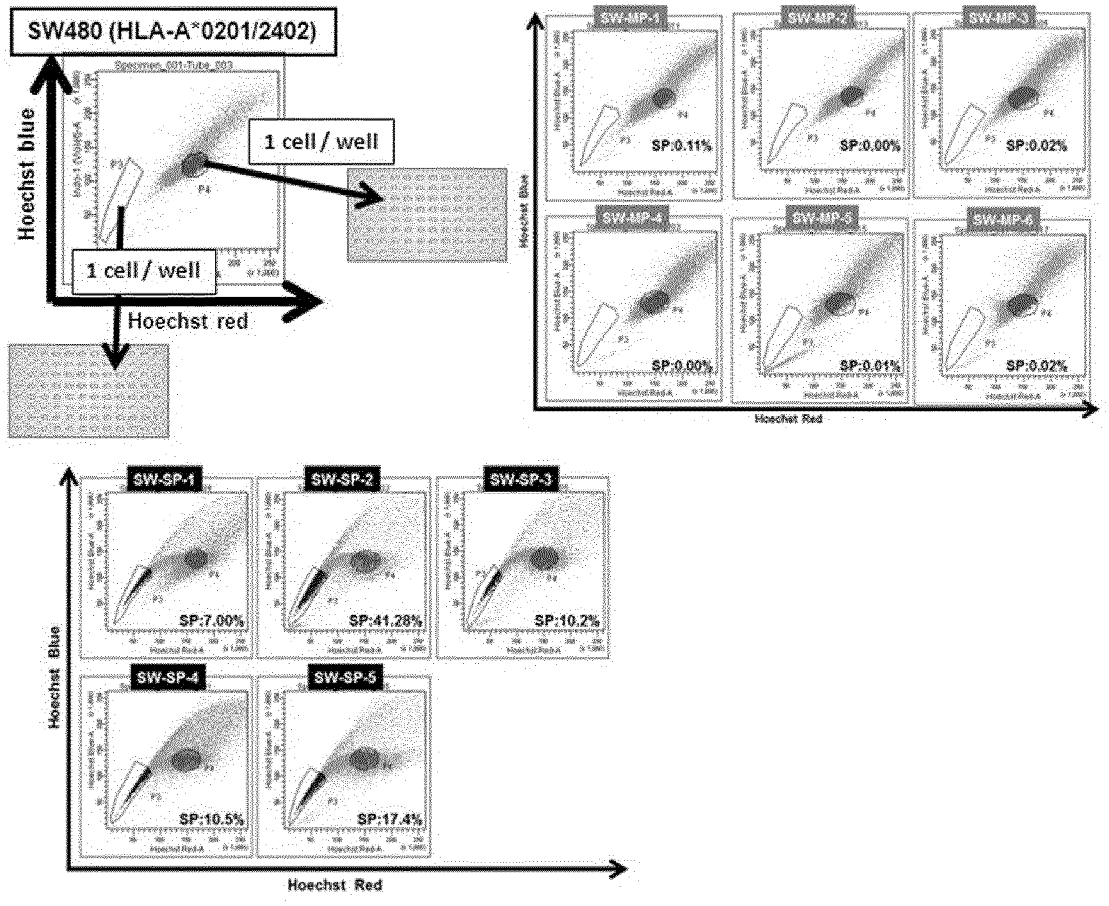


FIG. 2-2

SW480-SP AND -MP CELL SPHERE-FORMING ABILITY

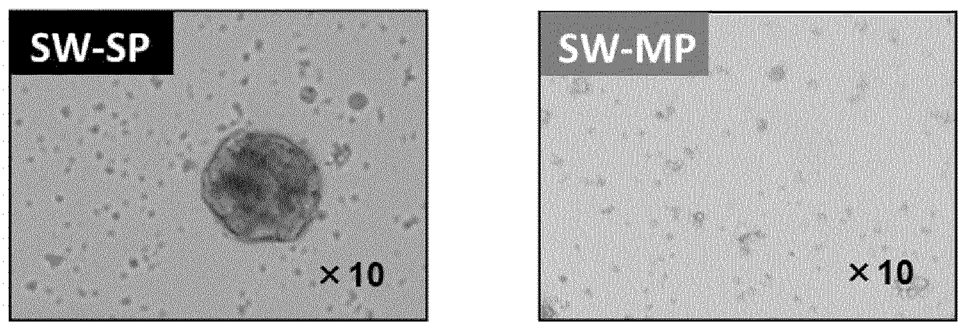

FIG. 3

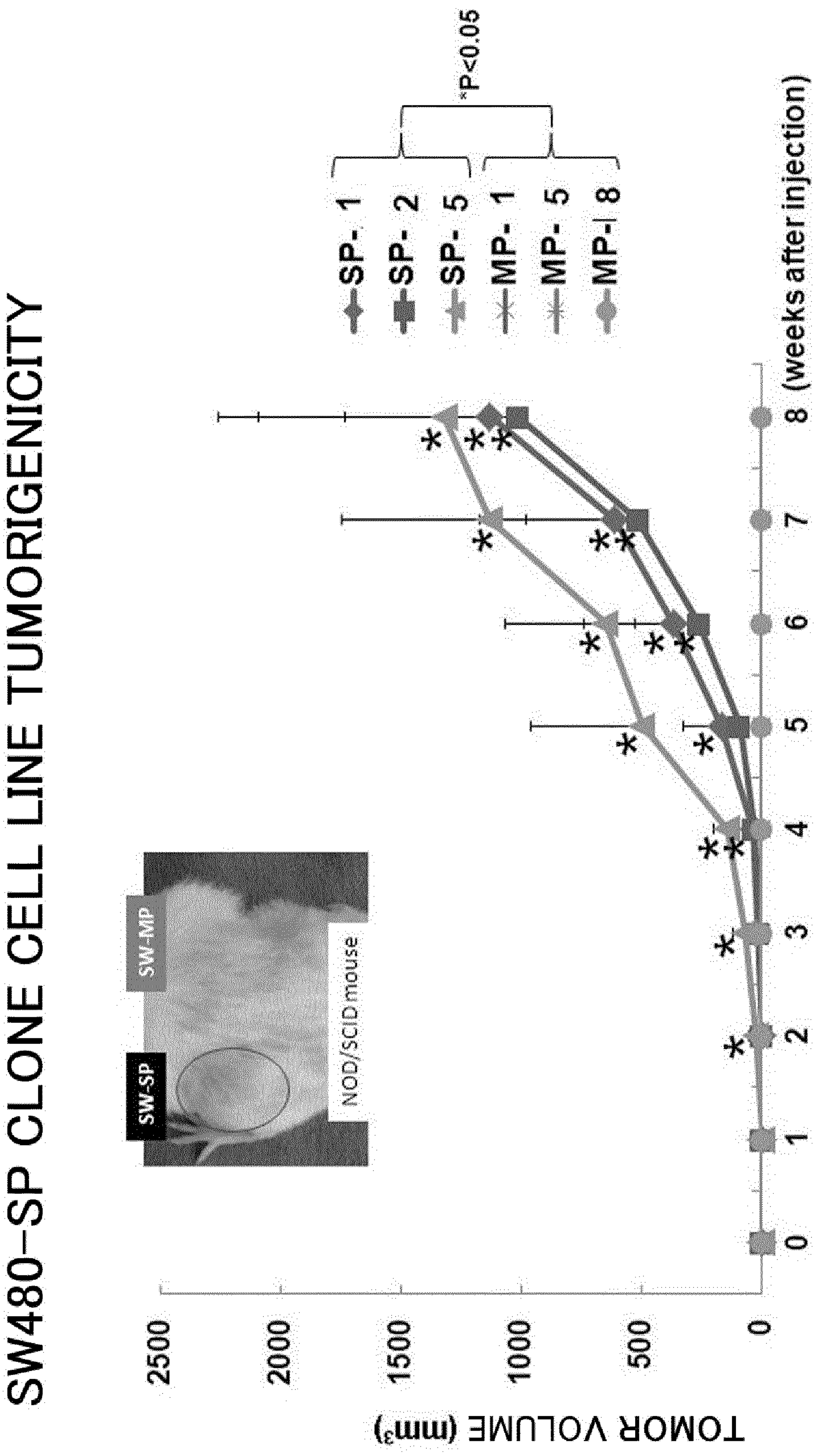
SW480–SP CLONE CELL LINE TUMORIGENICITY
SW-SP
SW-MP
NOD/SCID mouse
TOMOR VOLUME (mm³)
2500
2000
1500
1000
500
0
0
1
2
3
4
5
6
7
8
(weeks after injection)
SP- 1
SP- 2
SP- 5
MP- 1
MP- 5
MP- 8
*P<0.05

FIG. 4

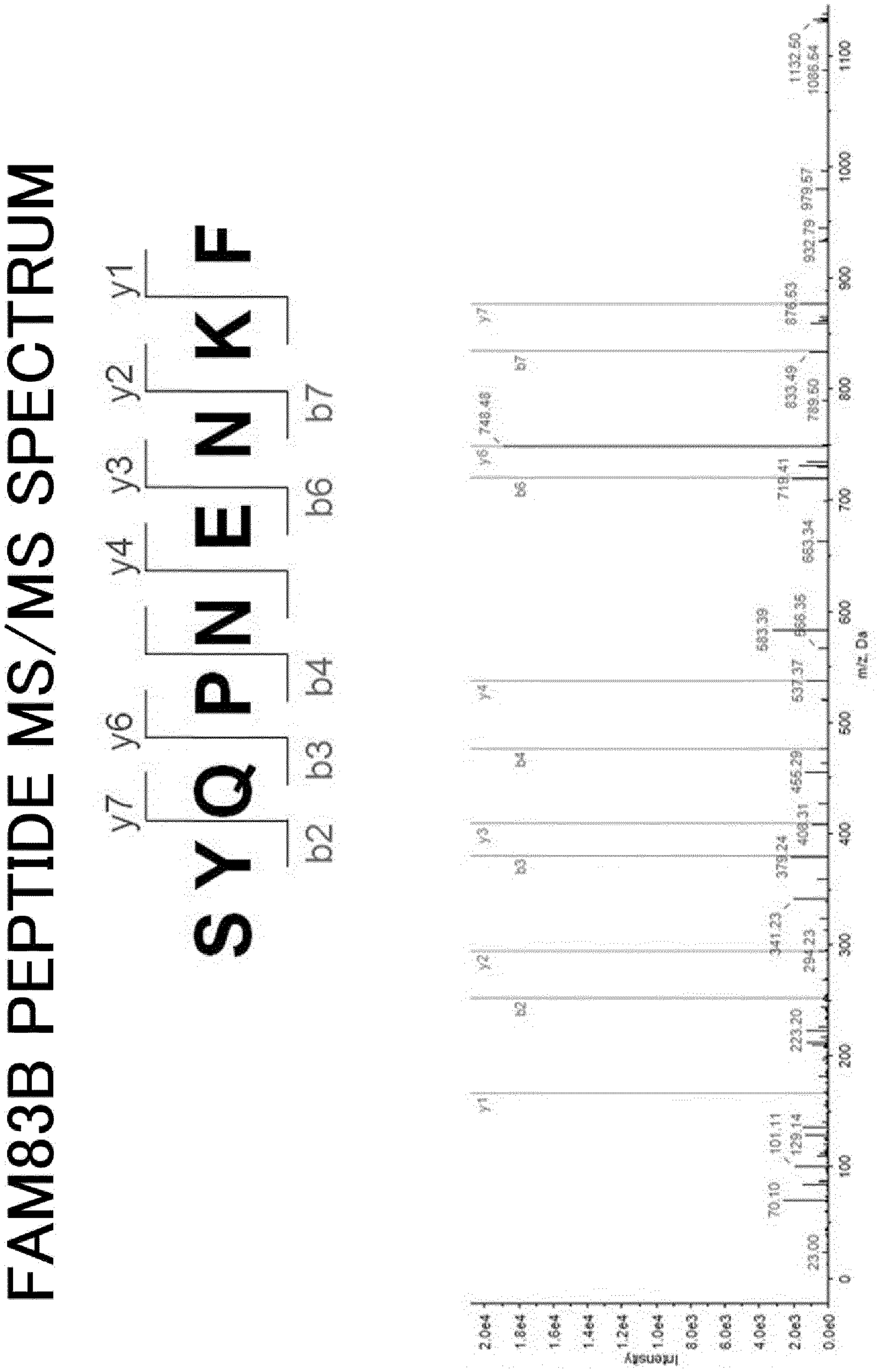
FAM83B PEPTIDE MS/MS SPECTRUM
S Y Q P N E N K F
y7 y6 y4 y3 y2 y1
b2 b3 b4 b6 b7
Intensity
m/z, Da

FIG. 5

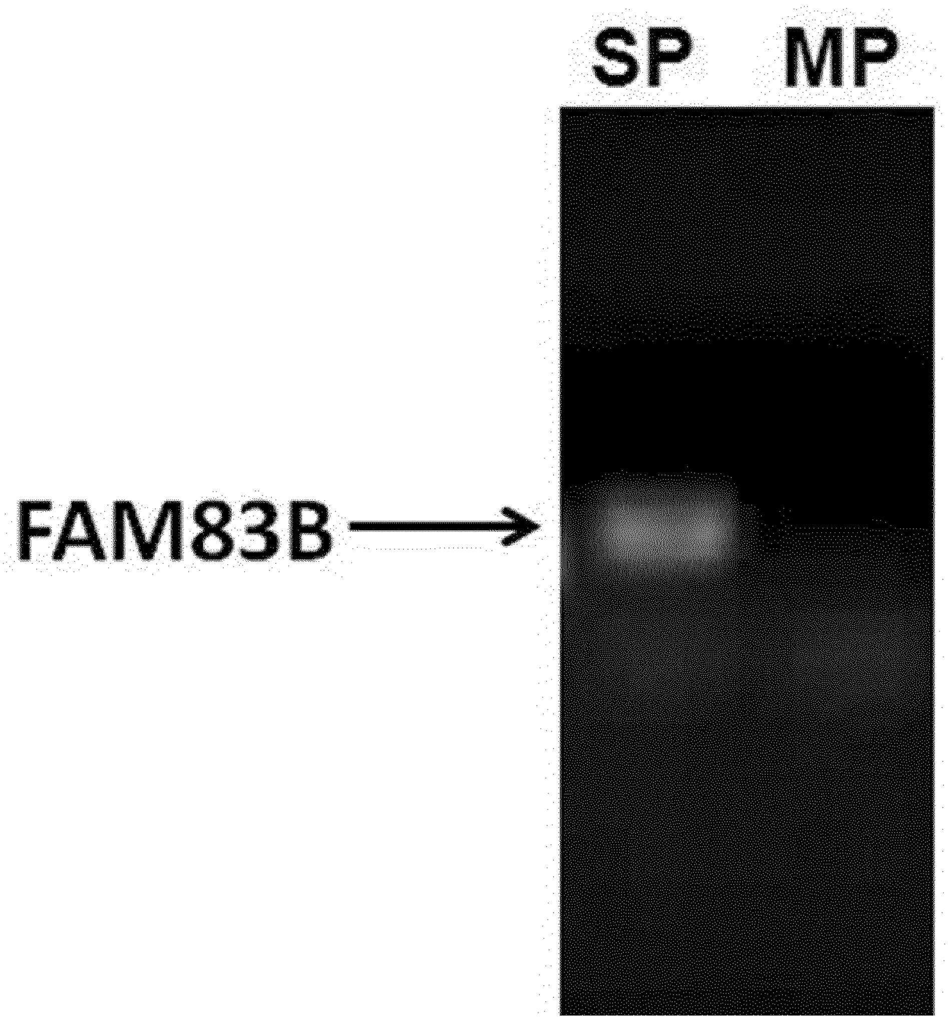

FIG. 6

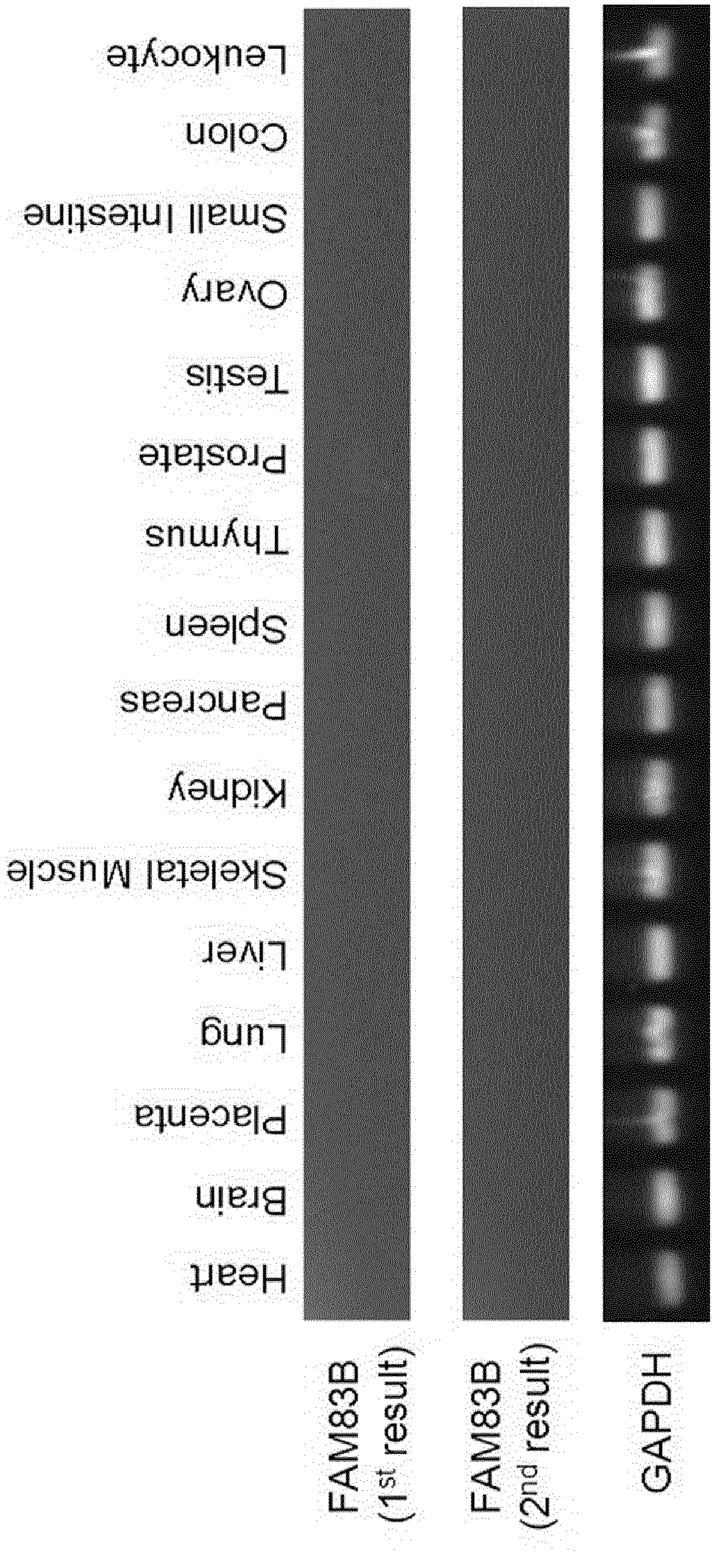
Leukocyte
Colon
Small Intestine
Ovary
Testis
Prostate
Thymus
Spleen
Pancreas
Kidney
Skeletal Muscle
Liver
Lung
Placenta
Brain
Heart
FAM83B (1st result)
FAM83B (2nd result)
GAPDH

FIG. 7

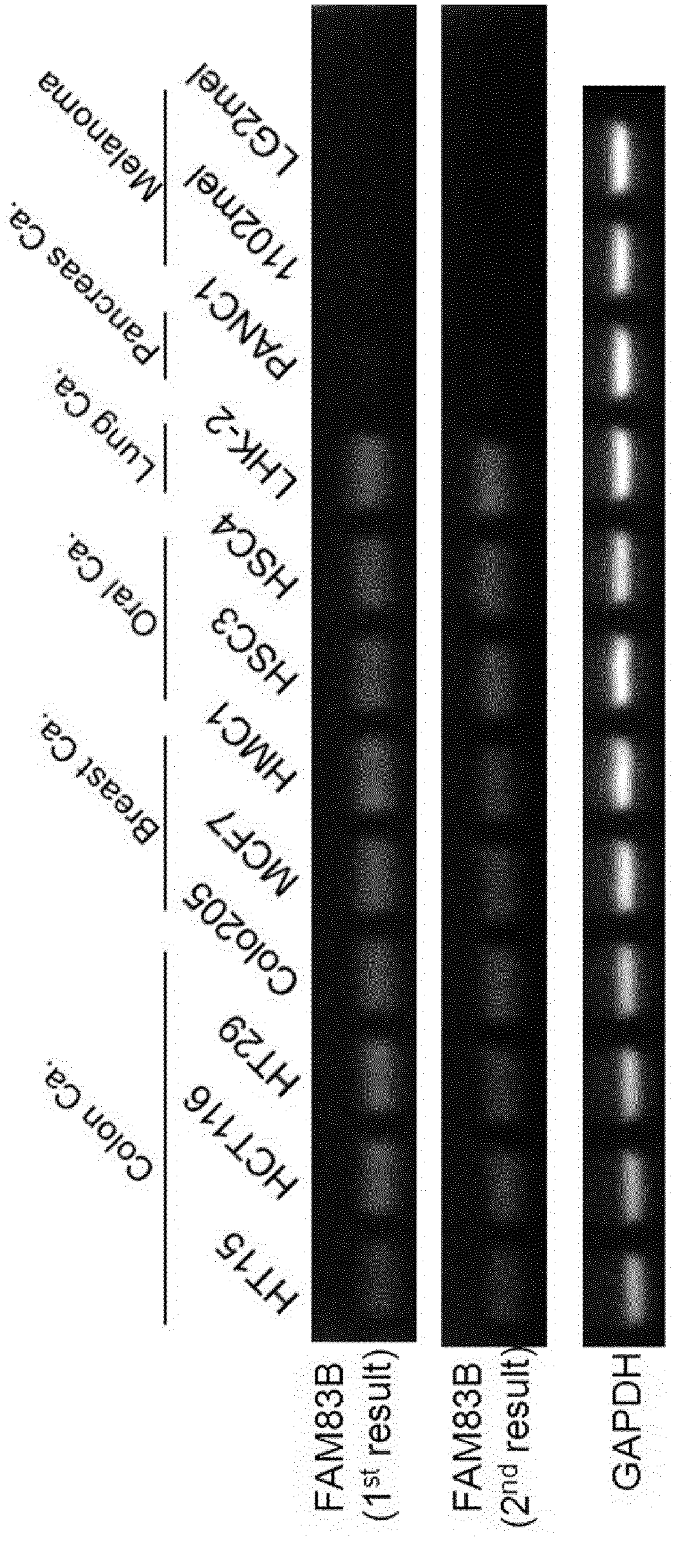
Melanoma
Pancreas Ca.
Lung Ca.
Oral Ca.
Breast Ca.
Colon Ca.
LG2mel
1102mel
PANC1
LHK-2
HSC4
HSC3
HMC1
MCF7
Colo205
HT29
HCT116
HT15
FAM83B (1st result)
FAM83B (2nd result)
GAPDH

FIG. 8

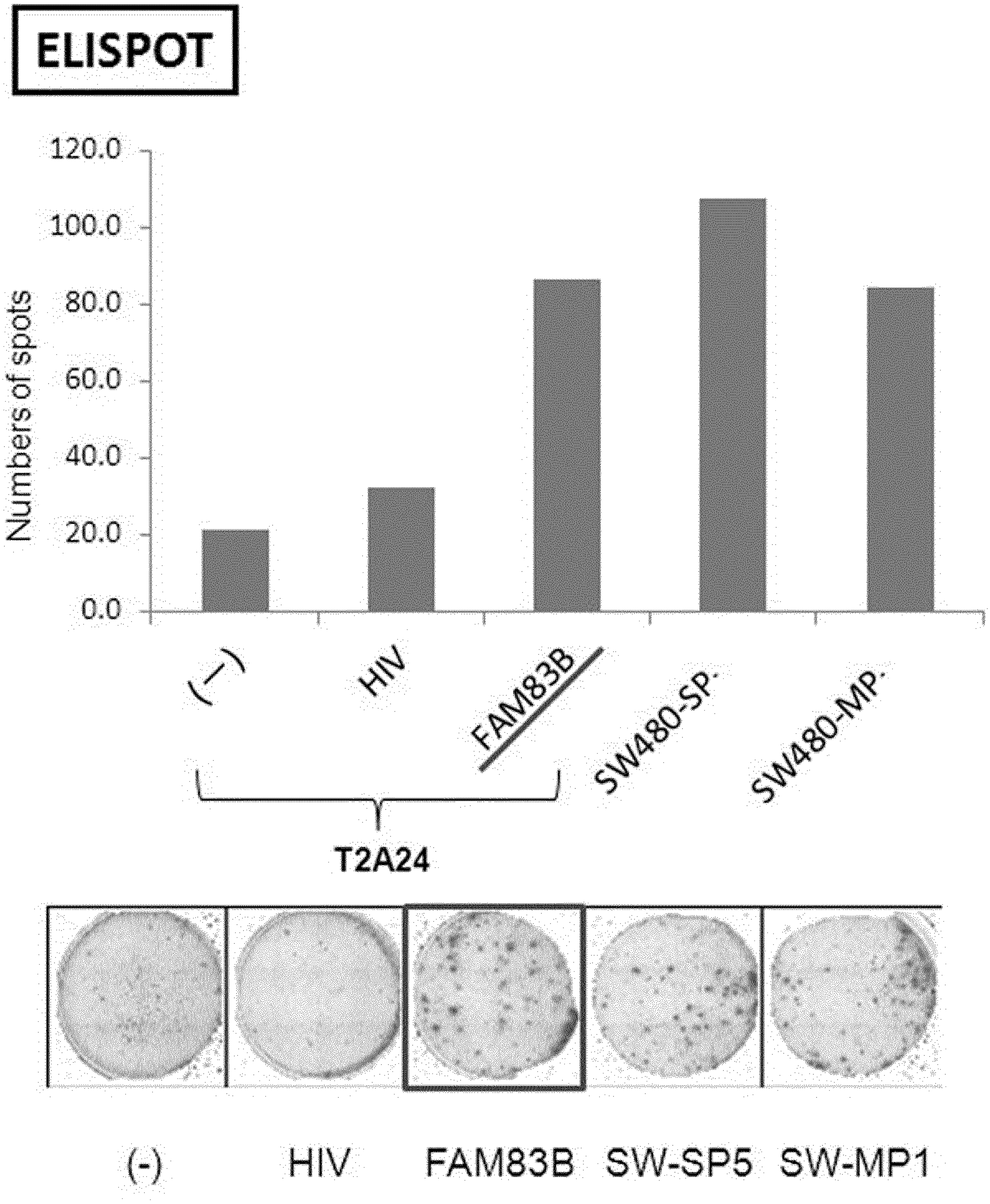
ELISPOT
120.0
100.0
80.0
60.0
40.0
20.0
0.0
Numbers of spots
(−)
HIV
FAM83B
SW480-SP.
SW480-MP.
T2A24
(-)
HIV
FAM83B
SW-SP5
SW-MP1

FIG. 9

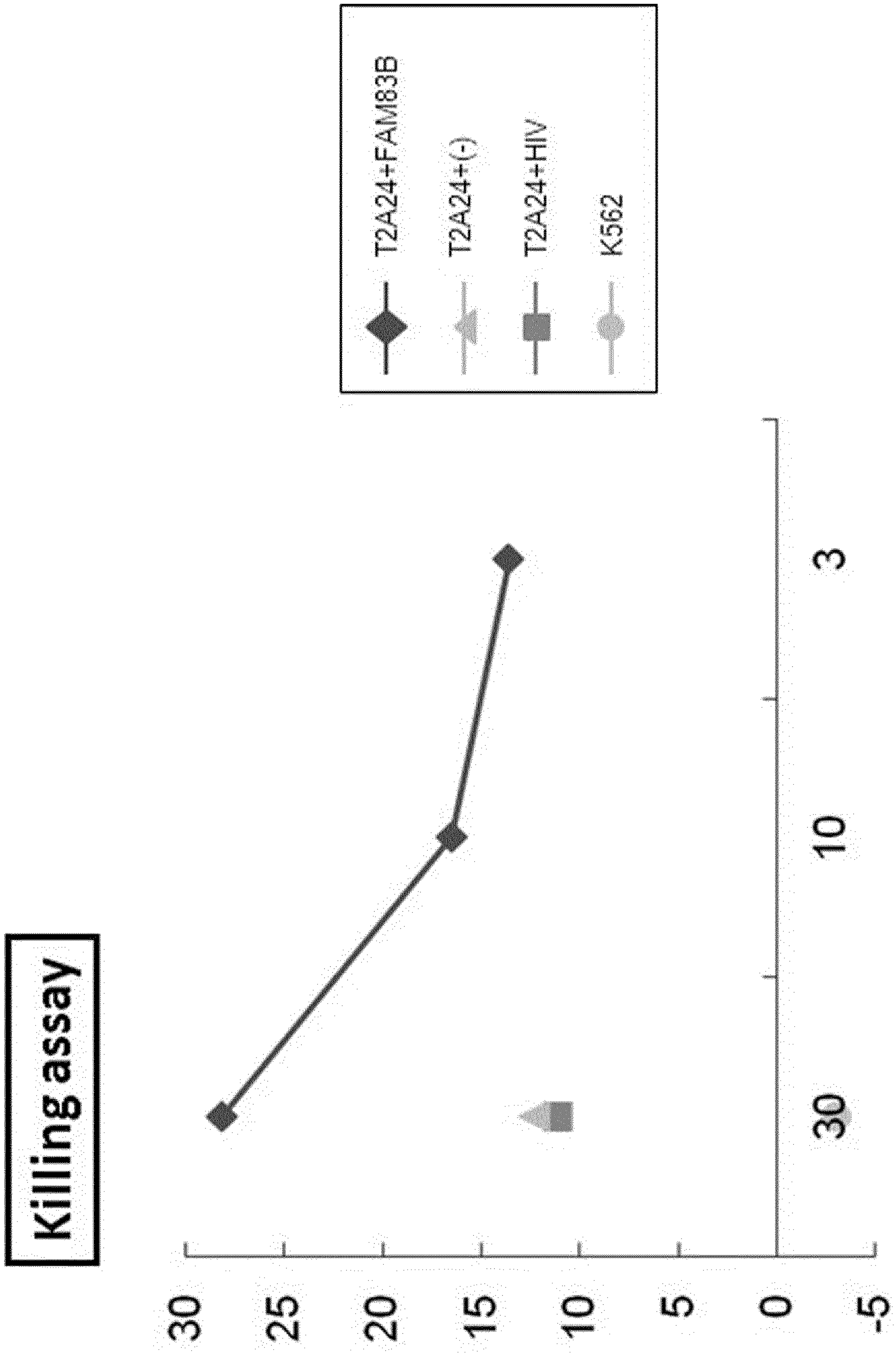
Killing assay
T2A24+FAM83B
T2A24+(-)
T2A24+HIV
K562
30
25
20
15
10
5
0
-5
30
10
3

FIG. 10

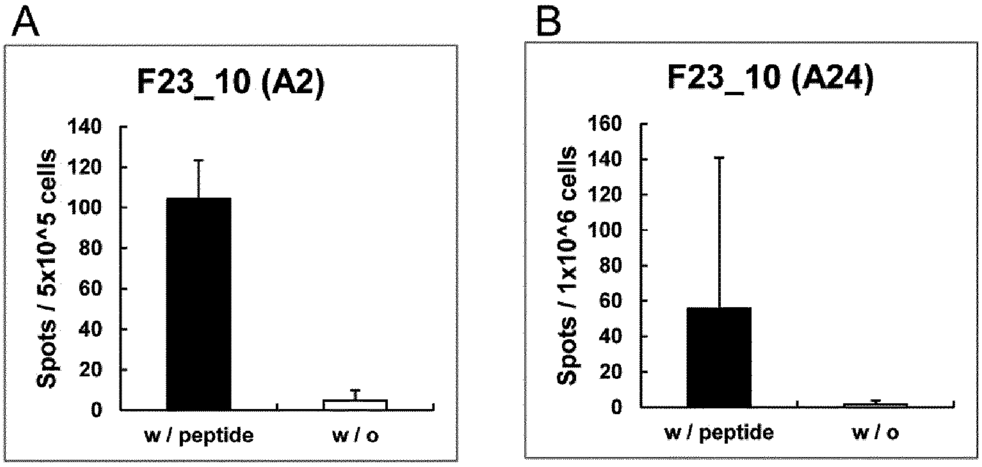


FIG. 11

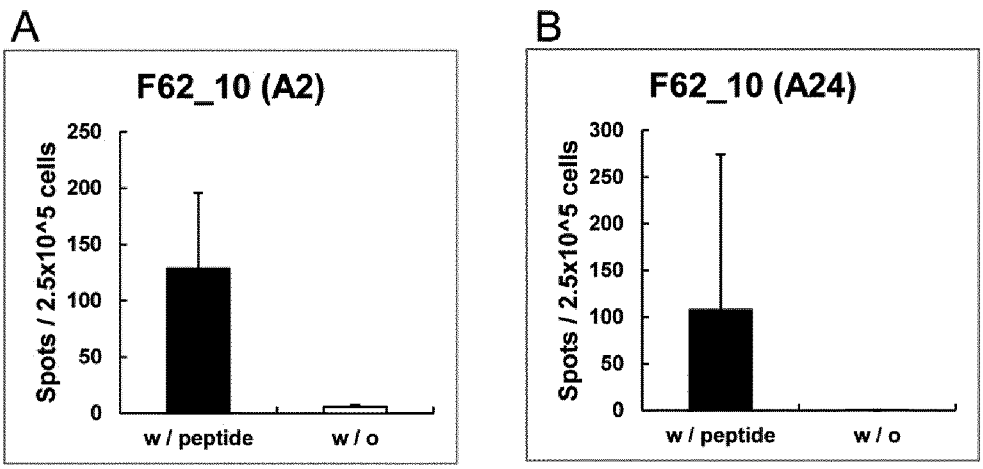


FIG. 12

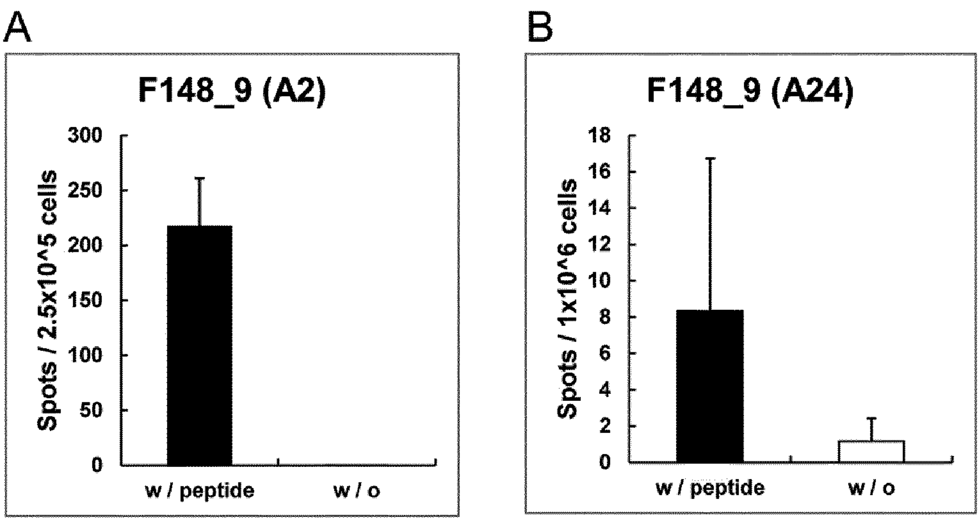

FIG. 13

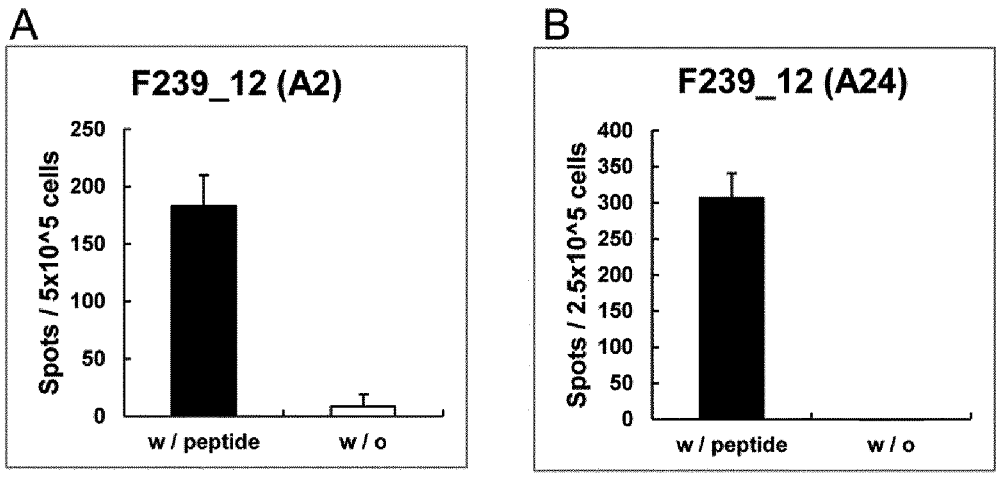


FIG. 14

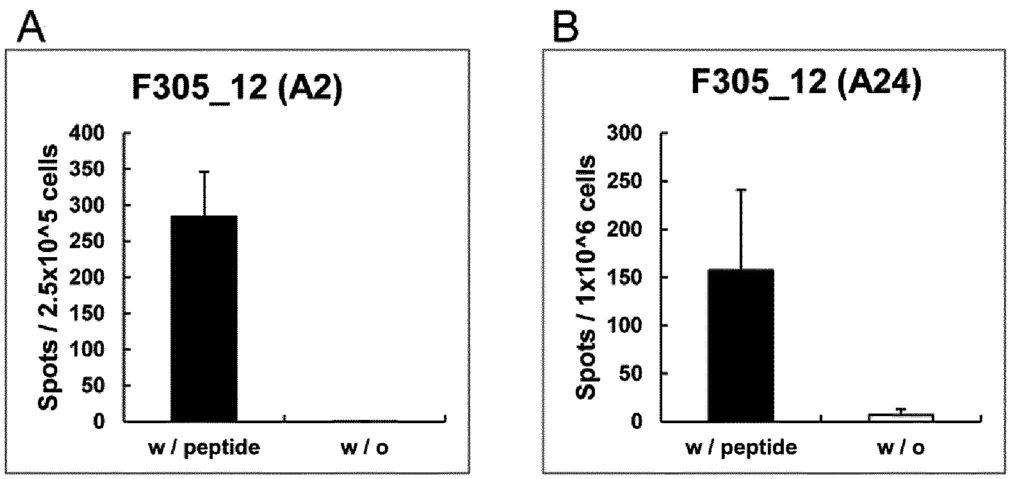


FIG. 15

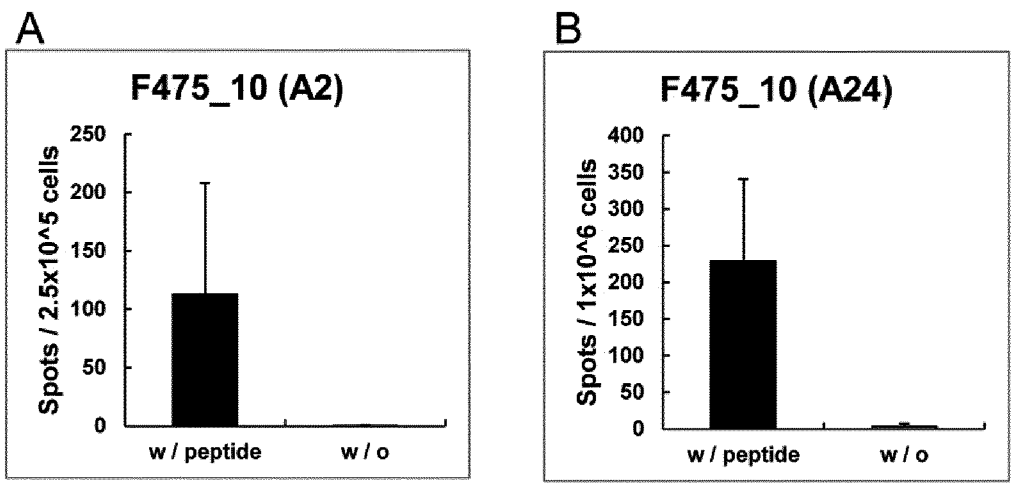

FIG. 16

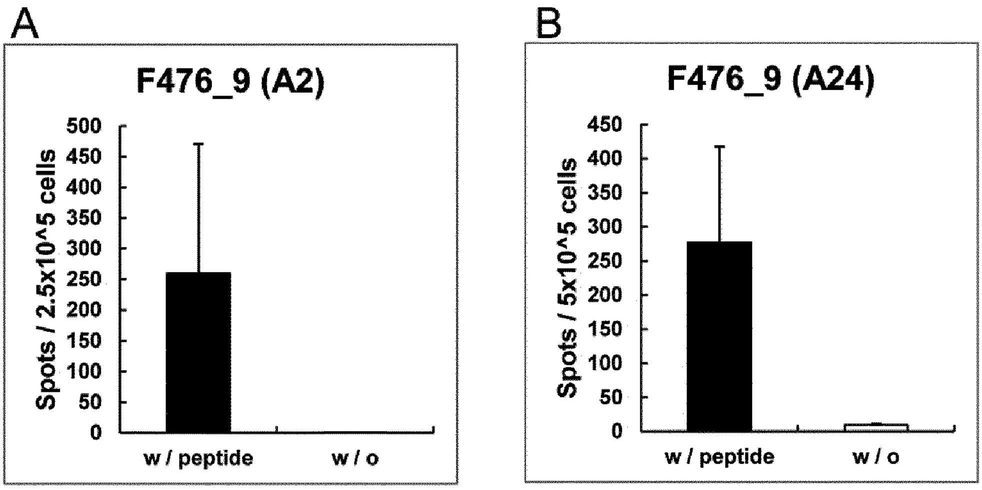


FIG. 17

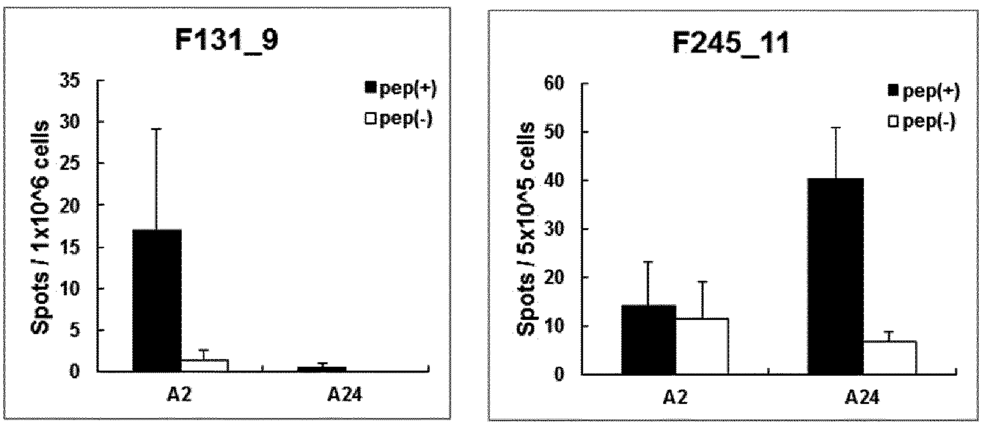


FIG. 18

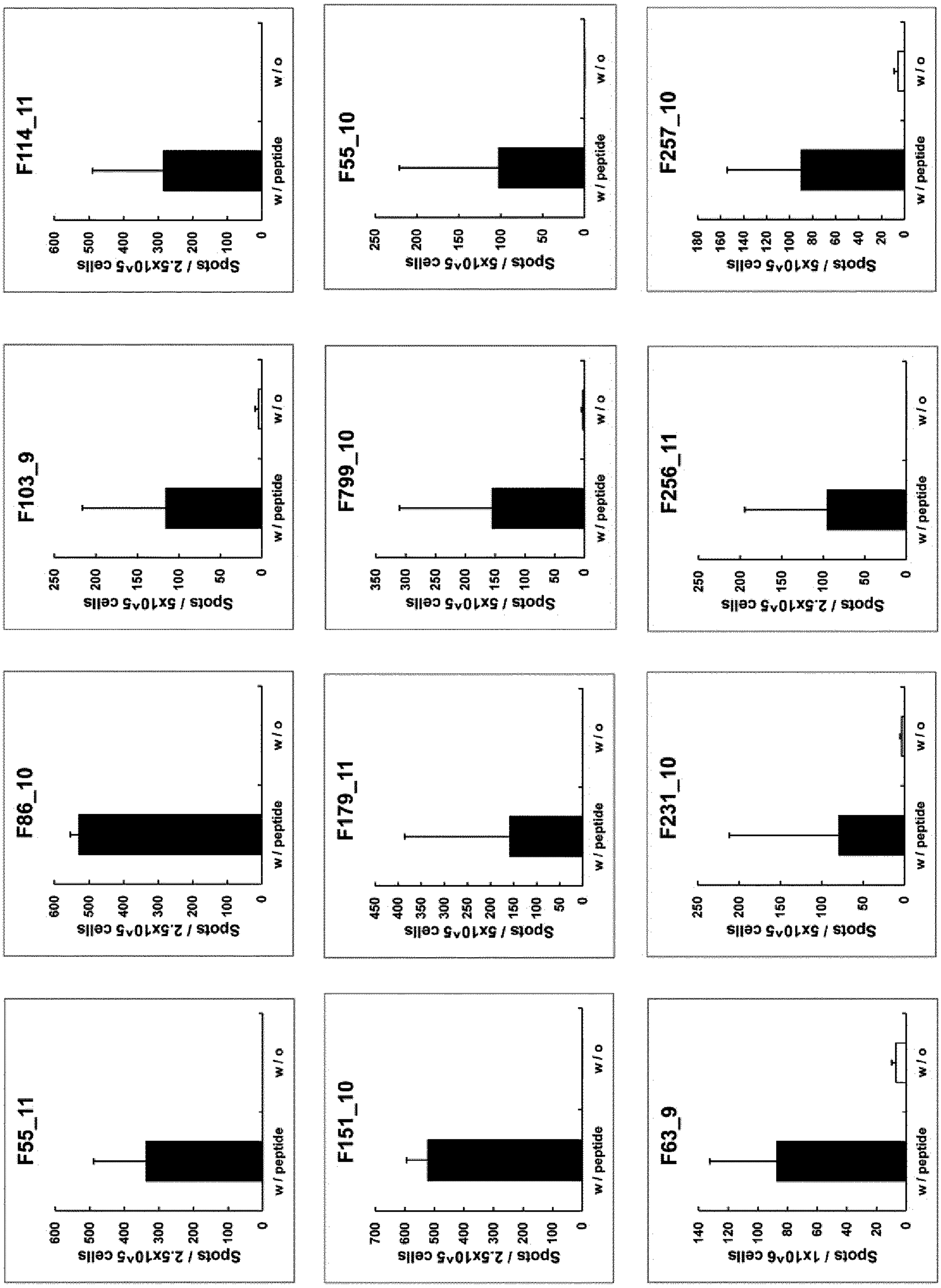
F55_11
Spots / 2.5x10^5 cells
w / peptide
w / o
F86_10
Spots / 2.5x10^5 cells
w / peptide
w / o
F103_9
Spots / 5x10^5 cells
w / peptide
w / o
F114_11
Spots / 2.5x10^5 cells
w / peptide
w / o
F151_10
Spots / 2.5x10^5 cells
w / peptide
w / o
F179_11
Spots / 5x10^5 cells
w / peptide
w / o
F799_10
Spots / 5x10^5 cells
w / peptide
w / o
F55_10
Spots / 5x10^5 cells
w / peptide
w / o
F63_9
Spots / 1x10^6 cells
w / peptide
w / o
F231_10
Spots / 5x10^5 cells
w / peptide
w / o
F256_11
Spots / 2.5x10^5 cells
w / peptide
w / o
F257_10
Spots / 5x10^5 cells
w / peptide
w / o

FIG. 19

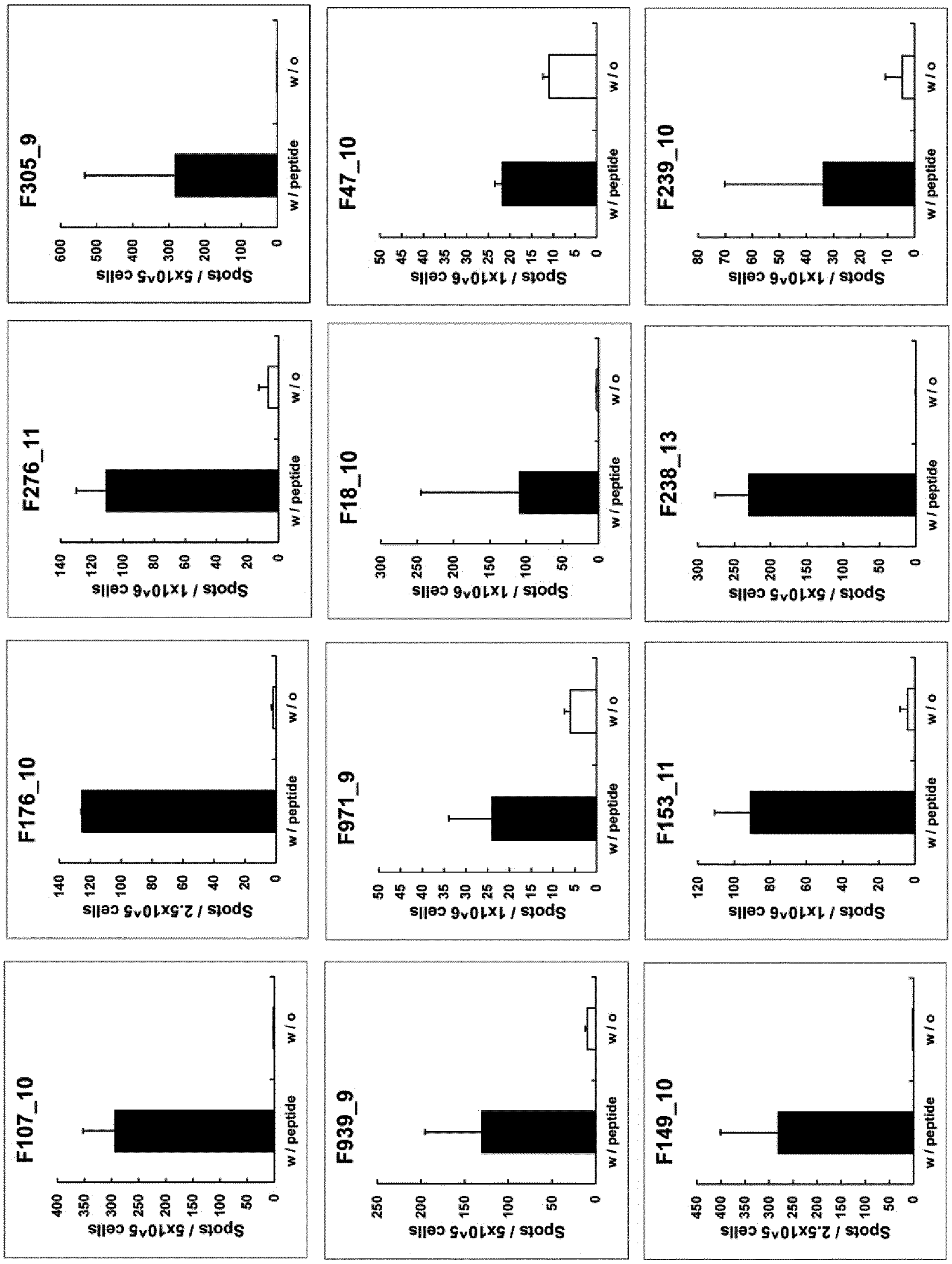
F305_9
Spots / 5x10^5 cells
w / peptide
w / o
F276_11
Spots / 1x10^6 cells
F176_10
Spots / 2.5x10^5 cells
F107_10
Spots / 5x10^5 cells
F47_10
Spots / 1x10^6 cells
F18_10
Spots / 1x10^6 cells
F971_9
Spots / 1x10^6 cells
F939_9
Spots / 5x10^5 cells
F239_10
Spots / 1x10^6 cells
F238_13
Spots / 5x10^5 cells
F153_11
Spots / 1x10^6 cells
F149_10
Spots / 2.5x10^5 cells

FIG. 20-1

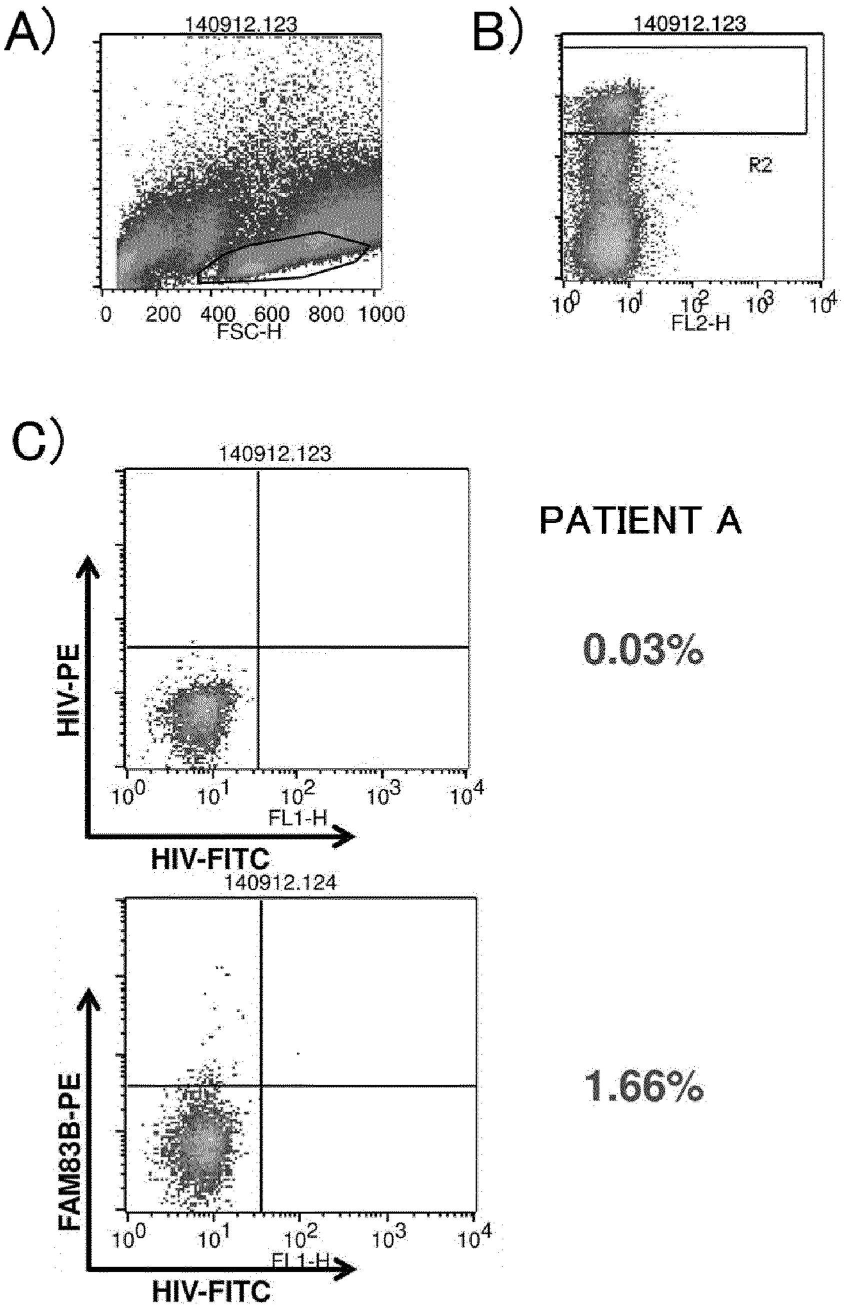
A)
140912.123
0
200
400
600
800
1000
FSC-H
B)
140912.123
R2
$10^0$
$10^1$
$10^2$
$10^3$
$10^4$
FL2-H
C)
140912.123
HIV-PE
$10^0$
$10^1$
$10^2$
$10^3$
$10^4$
FL1-H
HIV-FITC
PATIENT A
0.03%
140912.124
FAM83B-PE
$10^0$
$10^1$
$10^2$
$10^3$
$10^4$
FL1-H
HIV-FITC
1.66%

FIG. 20-2

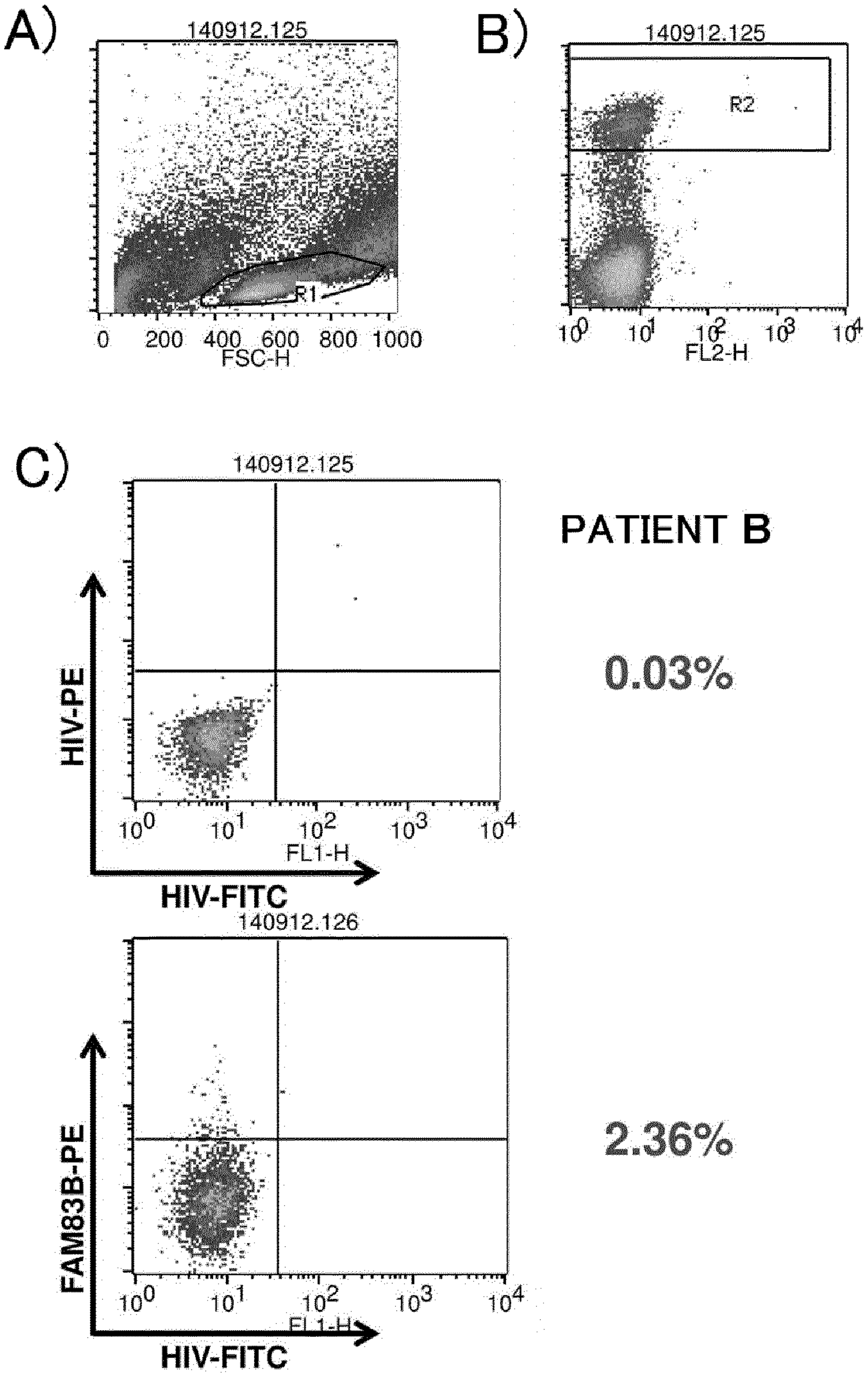
A)
140912.125
R1
0
200
400
600
800
1000
FSC-H
B)
140912.125
R2
10^0
10^1
10^2
10^3
10^4
FL2-H
C)
140912.125
HIV-PE
10^0
10^1
10^2
10^3
10^4
FL1-H
HIV-FITC
140912.126
FAM83B-PE
10^0
10^1
10^2
10^3
10^4
FL1-H
HIV-FITC
PATIENT B
0.03%
2.36%

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010050268 A
- WO 0247474 A
- WO 2010050190 A
- JP 2013208645 A


### Non-patent literature cited in the description


- **R. CIPRIANO et al.** *J. Clin. Invest.,* 2012, vol. 122 (9), 3197-3210
- **M. HILGER et al.** *J. Proteome Res.,* 2012, vol. 11, 982-994
- **R. CIPRIANO et al.** *Oncotarget,* 2013, vol. 4, 728-738
- **R. CIPRIANO et al.** *Oncogene,* 2013, 1-9
- **S. GRANT et al.** *J. Clin. Invest.,* 2012, vol. 122 (9), 3048-3051
- *J Proteome Res.,* 2012, vol. 11, 982-94
- *J Clin Invest.,* 2012, vol. 122, 3197-210
- **PEPTIDE SYNTHESIS.** *Interscience,* 1966
- The Proteins. Academic Press Inc, 1976, vol. 2
- Peptide Synthesis. Maruzen Co., Ltd, 1975
- Basics and Experiments of Peptide Synthesis. Maruzen Co., Ltd, 1985
- Development of Pharmaceuticals Seq. Peptide Synthesis, Hirokawa Shoten Co, 1991, vol. 14
- *Int J. Cancer,* 2002, vol. 100, 565-570
- *Cancer Sci,* vol. 103, 150-153
- *Angew. Chem. Int. Ed.,* 2008, vol. 47, 7551-7556
- **T. MANIATIS et al.** Molecular Cloning. CSH Laboratory, 1983
- **D M. GLOVER.** DNA Cloning. IRL PRESS, 1985
- *J. Immunol.,* 1995, vol. 154, 2257
- *Int. J. Cancer,* 1994, vol. 58, 317
- *Int. J. Cancer,* 1987, vol. 39, 390-396
- *N. Eng. J. Med,* 1995, vol. 333, 1038-1044
- *Journal of Immunology,* 1998, vol. 161, 3186-3194
- *Clin Infect Dis.,* 2000, 266-70
- **NIKKEI.** *Science,* April 1994, 20-45
- *Gekkan Yakuji,* 1994, vol. 36, 23-48
- *Experimental Medicine Special Edition,* 1994, vol. 12 (15
- *Nikkei Science,* April 1994, 20-45
- *Gekkan Yakuji,* 1994, vol. 36 (1), 23-48
- *Journal of Immunology,* 1999, vol. 162, 3915-3925
- *Cancer Immunol. Immunother.,* 1998, vol. 46 (82
- *J. Immunol.,* 1997, vol. 158, 1796
- *Cancer Res.,* 1999, vol. 59, 1184
- *DNA, Cancer Res.,* 1996, vol. 56, 5672
- *J. Immunol.,* 1998, vol. 161, 5607
- *RNA, J. Exp. Med.,* 1996, vol. 184, 465
- *J. Natl. Cancer. Inst.,* 1994, vol. 86, 1159
- *J. Exp. Med.,* 1997, vol. 185, 453
- **OCHI et al.** *Blood,* 11 August 2011, vol. 118 (6), 1495-503
- *Science,* 1998, vol. 279, 2103-2106
- *Science,* 1996, vol. 274, 94-96
- *Eur J Immunol.,* 2004, vol. 34, 2919-29
- *Nat Rev Immunol.,* 2012, vol. 12, 269-81
- *Science,* 1996, vol. 274, 94
- **KONDO T ; SETOGUCHI T ; TAGA T.** Persistence of a small subpopulation of cancer stem-like cells in the C6 glioma cell line. *Proc Natl Acad Sci U S A.,* 2004, vol. 20, 781-786

SEQUENCE LISTING

<110> Sumitomo Dainippon Pharma Co. Ltd. Sapporo Medical University

<120> Tumor antigenic peptide

<130> PCT2674DN

<150> JP2013-208645
<151> 2013-10-03

<160> 85

<170> PatentIn version 3.5

<210> 1
<211> 6269
<212> DNA
<213> Homo sapiens

<400> 1

aaaccttcgg cggccggcgc tgtgcggcgg gcgcggttgc gcgcggcttg gggcaaatac 60
ttctcaccac tgcatgaatg gacatttgaa agtgccatag ccaaacactt gcaagcatgg 120
agacctcatc aatgctttcc tcattgaatg atgagtgtaa atctgacaac tacattgagc 180
ctcactacaa ggaatggtat cgagtagcca ttgatattct gattgaacac gggttagaag 240
cataccaaga atttcttgtc caggaacgag tttcagactt tcttgctgag gaagaaatta 300
attatatttt gaaaaatgtc cagaaagttg cacaaagcac agcacatggt actgatgatt 360
cctgtgatga taccttatct tcagggacct actggcctgt tgagtctgat gtggaagctc 420
caaatcttga cttaggctgg ccatatgtga tgcccggact cttagggggc acccatatag 480
atctcctttt tcatccacca agagcacatc tacttacgat aaaagaaact attcggaaga 540
tgataaaaga agcaagaaag gtcattgctt tagtgatgga tatatttaca gatgtggaca 600
ttttcaaaga aatcgttgag gcatcaactc gaggagtatc tgtttacatt ctgcttgatg 660
agtccaattt taatcatttt ctaaatatga ctgagaaaca aggttgttca gttcagcgtc 720
tcaggaatat tcgagtgcga acagtaaaag gccaagatta tctttcaaaa acaggggcaa 780
aattccatgg aaaaatggaa cagaaatttt tgttagttga ctgccagaaa gtgatgtacg 840
gttcttacag ttatatgtgg tcatttgaga aagctcacct cagcatggtt cagataatta 900
caggacaact tgttgagtcc tttgatgaag aatttagaac tctctatgcc agatcctgtg 960
tccctagttc atttgctcag gaagaatcag caagggtgaa gcatggaaaa gccctctggg 1020
aaaatggcac ttaccagcat tcggtgtctt cattagcatc tgtttccagc cagagaaacc 1080
tttttggtag acaagacaag attcataaac tagattccag ttacttcaaa aacagaggga 1140
tatatacttt aaatgaacat gacaaatata acataagaag tcacggatac aaacctcatt 1200
ttgttcctaa ctttaatggt ccaaacgcaa tacgtcagtt tcaacccaat cagataaatg 1260

aaaattggaa aaggcatagt tatgctgggg aacagccaga aacagtgcca tacctcctgc 1320
ttaatagggc tctgaataga accaataatc cacctggtaa ttggaaaaag ccatctgata 1380
gtctcagtgt ggcgtcctca tcacgggaag gctatgtaag ccaccacaac acacctgccc 1440
agagttttgc caatcggctt gcgcagagaa aaacaacaaa tcttgcagac aggaattcaa 1500
atgttcggag gtcttttaat gggacagata accatatccg ctttttgcaa caacgaatgc 1560
caacccttga acataccaca aagtcattcc tacgtaactg gagaattgaa tcctacttaa 1620
atgatcattc agaagctaca ccggactcaa atggatcagc tttaggtgac cgatttgagg 1680
gctatgataa tcctgagaat ttgaaggcca atgcccttta tactcattct cggcttcgtt 1740
cctctttagt atttaaaccc actttacctg agcaaaagga agttaacagt tgtacaactg 1800
gctcctcaaa ttcaactatc attggttctc agggaagtga gacacctaaa gaggtcccag 1860
acacccctac gaatgtacag catttgacag acaaaccctt gccagaatca atccccaagc 1920
tcccattgca gtcagaggca ccaaaaatgc acaccttgca ggttcctgaa aaccactcag 1980
tagccttaaa ccaaactaca aatggccata ctgaatcaaa taactatata tataaaacct 2040
tgggtgtaaa taagcagaca gaaaatctaa agaatcaaca gactgagaat ctacttaaaa 2100
ggcgaagttt cccgttattt gacaactcaa aagccaactt agatcctgga aatagtaagc 2160
attatgtata tagtacactt accaggaatc gagttagaca accagaaaag cccaaagaag 2220
atttgctgaa aagttctaaa agcatgcaca atgtgactca taacttggag gaggatgagg 2280
aggaagttac caagagaaac tctccaagtg gcactactac caaatcagtt tccattgctg 2340
ctttacttga tgtgaataaa gaggaatcta acaaagaact tgcttcaaag aaggaagtta 2400
agggttcccc aagtttttg aaaaaggggt ctcagaagtt aaggtcatta cttagcctta 2460
ccccagataa gaaagaaaat ctatccaaaa ataaagcacc tgccttttat agattgtgta 2520
gtagctctga cacattagtt tctgagggtg aagaaaatca aaaaccaaag aaatcagaca 2580
caaaagttga ttcatctcct agaagaaagc attcttcctc atcgaattct caaggcagca 2640
tccacaagag taaggaagat gtaacagtta gcccatctca agagataaat gctccaccag 2700
atgaaaataa aagaacacct tctccaggtc cagttgaaag caagttcttg gaaagggcag 2760
gagatgcctc tgccccaaga tttaacactg aacagatcca ataccgagat tcaagggaga 2820
ttaatgcagt tgttacccct gaaagaagac ctacttcttc tccaaggcca acgtccagtg 2880
agcttctacg atctcattca actgatcggc gtgtttacag tcgttttgag ccgttttgta 2940
agattgagag ctctattcag ccaacaagca acatgccaaa taccagtata aatcgcccag 3000
aaataaaatc tgcgactatg ggcaacagtt atggcaggtc tagtccattg cttaattaca 3060
acactggtgt ttatcgctca tatcaaccca atgagaacaa gtttcgagga tttatgcaaa 3120

agtttggaaa ctttatacac aaaaataaat agctattaaa atgcaaaatg aatgaggcta 3180
tcaatatttg tccaaagaaa attgtggaca gtctttgtaa catgccaata gattttccta 3240
aggacagaat tatgggtatg atgtatatgt tcaccagtgt cctagtataa agttattttt 3300
ctgtgtgata aagttagggt ctgctgtaga tagaatttct ctgtaaacac attatttgta 3360
agtggtaatg gtaaaaataa tagatgtatt taaatcattt tctttagact gaagatttta 3420
agtctcactt agaaattttt gtggacgatg tatggtgtat ggtgtatggt gtatggattt 3480
taaccatttc cttttaaaaa ggtcatacta ccctcagtaa ccctttataa catgtgttta 3540
tagtgttttc atatcttcaa aaatataagc aaatgggaca aagccctttt ttaagattaa 3600
cttgaagttc tacgggataa cttgttatat tttattaata ttattttttc tgtggatcat 3660
tgtacagctg tttgggcaac agcagtacct tttacatttt ttattttta ttttttttc 3720
tgcctgaagt gtttgcaaag tatcagcctc atgtagtata atgacattcg taggaattta 3780
ttttcctcag cctttgaaaa cggtctggga tgtgcttctt tctacactgg acccagggag 3840
ctgtcccct cttacccata ggctgctgat tttttatagt cattccttac ttcacattta 3900
ggacaaacca gtaggaattt agaaaatctg aatgattccc cctccttttt ctattgtata 3960
aaagctttta gaaatgtaaa tttctgccta aatttttgtt agattggcat gacaatgcta 4020
aggggtcttg cttgcgaaaa ttcttgctct ttttttttt cttttgagat ggagtctcgc 4080
tctgtcaccc aggctagagt gcagtggcgc aatcttggct cactgcaacc tctgcctccc 4140
aagttcaaac gattctcctg cctcagcctc ctgagtagct gggattacag gtgcgcacca 4200
ccacacccgg ctaatttttg tattttcaga agagatggga tttcaccatg ttggccagtc 4260
tagtctcaaa ctcctgacct cgtgatcctc ccgcctcggc ctcccagagt gctgggatta 4320
caggtcttgc tcttctttaa gttgtgccaa atatgatatg actgcatagt gtttttgtaa 4380
gaataccatt gggaaaatga gaagttttac tgggctagtt cttccatgat ttgaggatga 4440
tattcagatt ataagatacc ctttgtcttt cttacagtaa tgaagtataa gctccatatc 4500
tgttccagag actttaactt gactttgctt cattcacaaa gaacagagtt caagaagcag 4560
tgcatcctgt gagaagtgtg aagtgtttgt acatcacttt aaatatatta cttaatatat 4620
tctaagttgc tgtgtggagc agtatactgt tgttttaaaa atgcaagatt cagacaaatt 4680
ttaatattgt ctcattaaaa taatttaaat aatggtataa aatatctata tcattaaaat 4740
aattttccat agtgtttaga aaccatgaaa aagaaaacat agcaggagaa aatatgacag 4800
gaaaagaaaa cctaacaaag cccagaaccc acagttaacc aaactagact gactttgtta 4860
ttacccattc tttgttagta ttggataggg aattaggata atgagccatt aggcagttct 4920
gaaatggaaa gccctgaaat ttagtgcaat gtaactttaa aactgaaatt atataaagag 4980
tagaagttta atcatgttta attacaacca aaagcctgtt gccttttgt acagaaatct 5040

ccttaatttc aggacatgta gatagcttat agaaacatcc ttttaaatag tttgtgagct 5100

cttcctcttc agtggaattg aggaatacag aatgctttat ttcatcatcc cctgacaggt 5160

gacttaggct ttgcacagca gatttatttt tctctgcgtt ttttaaaaat tgtttttctt 5220

gtatcttttt tcttcaaaaa tatctatatt tgagaatatg tacataacaa ctttccaaag 5280

tctctgtggc caaaacctct ccagggataa gactgagcaa gaatataata cttcaaaaaa 5340

tgtacagcta ctgtttaagt tttaaacaga caccatcaca gtttgtggat gaaatagttt 5400

taagccatat actttctgtc tttttttccc catattaata ttgggggcg gataatatca 5460

ctttgatgta cattgatatt aaagtttggt aatgcagctt ttactgtcta catggtactg 5520

tacattagtt tttaagcaga aacacaagaa aaatgggtat aatttcaaag tagttcttgg 5580

cagatggcta gagaatactg caagtgaccc tgtatcccga atacacagat atccctctat 5640

tacaagtttg ggattagcca taattctgac atggtgtgtt caggtatggg tatatgttgt 5700

cagtctacac ttgtggaagc aaatatcttg tttaatcaag atgatgtcta gtgtcaccta 5760

aataatgcaa aaagtttaat tctggatgaa ttcagcttta ctcaagatcc acatattcaa 5820

gtatcattcc acagatattc acagaacaca gaaatatctg tgttctgcct tatgcctgtg 5880

gctaagggat gcaaagtaga attgctttac attgactata tatgtgacat gtacgttgct 5940

gtttttttta aaataacttt atcatgatat tcaggtagat cctgggttct agaatattta 6000

aaacaaaagg ataaaatgat aaaccaaaga gtcaacttgt taacttttct tttttaagag 6060

atgggttttc actagtatgc ccagtctgga ctccaagtcc tgggctcaaa cgatcctcca 6120

gcctcaggtt cctgagtagc tcaacatttt atgtatgtac gatattataa agaaatattc 6180

ttccaaatga acttttgttt ttagatcaat aaatgaataa taaataattt tgtacaaaca 6240

tcaaaaaaaa aaaaaaaaaa aaaaaaaaa 6269

<210> 2
<211> 1011
<212> PRT
<213> Homo sapiens

<400> 2

Met Glu Thr Ser Ser Met Leu Ser Ser Leu Asn Asp Glu Cys Lys Ser
1               5                   10                  15

Asp Asn Tyr Ile Glu Pro His Tyr Lys Glu Trp Tyr Arg Val Ala Ile
            20                  25                  30

Asp Ile Leu Ile Glu His Gly Leu Glu Ala Tyr Gln Glu Phe Leu Val
        35                  40                  45

Gln Glu Arg Val Ser Asp Phe Leu Ala Glu Glu Glu Ile Asn Tyr Ile
50 55 60

Leu Lys Asn Val Gln Lys Val Ala Gln Ser Thr Ala His Gly Thr Asp
65 70 75 80

Asp Ser Cys Asp Asp Thr Leu Ser Ser Gly Thr Tyr Trp Pro Val Glu
85 90 95

Ser Asp Val Glu Ala Pro Asn Leu Asp Leu Gly Trp Pro Tyr Val Met
100 105 110

Pro Gly Leu Leu Gly Gly Thr His Ile Asp Leu Leu Phe His Pro Pro
115 120 125

Arg Ala His Leu Leu Thr Ile Lys Glu Thr Ile Arg Lys Met Ile Lys
130 135 140

Glu Ala Arg Lys Val Ile Ala Leu Val Met Asp Ile Phe Thr Asp Val
145 150 155 160

Asp Ile Phe Lys Glu Ile Val Glu Ala Ser Thr Arg Gly Val Ser Val
165 170 175

Tyr Ile Leu Leu Asp Glu Ser Asn Phe Asn His Phe Leu Asn Met Thr
180 185 190

Glu Lys Gln Gly Cys Ser Val Gln Arg Leu Arg Asn Ile Arg Val Arg
195 200 205

Thr Val Lys Gly Gln Asp Tyr Leu Ser Lys Thr Gly Ala Lys Phe His
210 215 220

Gly Lys Met Glu Gln Lys Phe Leu Leu Val Asp Cys Gln Lys Val Met
225 230 235 240

Tyr Gly Ser Tyr Ser Tyr Met Trp Ser Phe Glu Lys Ala His Leu Ser
245 250 255

Met Val Gln Ile Ile Thr Gly Gln Leu Val Glu Ser Phe Asp Glu Glu
260 265 270

Phe Arg Thr Leu Tyr Ala Arg Ser Cys Val Pro Ser Ser Phe Ala Gln
275 280 285

Glu Glu Ser Ala Arg Val Lys His Gly Lys Ala Leu Trp Glu Asn Gly
290 295 300

```
Thr Tyr Gln His Ser Val Ser Ser Leu Ala Ser Val Ser Ser Gln Arg
305                 310                 315                 320

Asn Leu Phe Gly Arg Gln Asp Lys Ile His Lys Leu Asp Ser Ser Tyr
                325                 330                 335

Phe Lys Asn Arg Gly Ile Tyr Thr Leu Asn Glu His Asp Lys Tyr Asn
            340                 345                 350

Ile Arg Ser His Gly Tyr Lys Pro His Phe Val Pro Asn Phe Asn Gly
        355                 360                 365

Pro Asn Ala Ile Arg Gln Phe Gln Pro Asn Gln Ile Asn Glu Asn Trp
    370                 375                 380

Lys Arg His Ser Tyr Ala Gly Glu Gln Pro Glu Thr Val Pro Tyr Leu
385                 390                 395                 400

Leu Leu Asn Arg Ala Leu Asn Arg Thr Asn Asn Pro Pro Gly Asn Trp
                405                 410                 415

Lys Lys Pro Ser Asp Ser Leu Ser Val Ala Ser Ser Ser Arg Glu Gly
            420                 425                 430

Tyr Val Ser His His Asn Thr Pro Ala Gln Ser Phe Ala Asn Arg Leu
        435                 440                 445

Ala Gln Arg Lys Thr Thr Asn Leu Ala Asp Arg Asn Ser Asn Val Arg
    450                 455                 460

Arg Ser Phe Asn Gly Thr Asp Asn His Ile Arg Phe Leu Gln Gln Arg
465                 470                 475                 480

Met Pro Thr Leu Glu His Thr Thr Lys Ser Phe Leu Arg Asn Trp Arg
                485                 490                 495

Ile Glu Ser Tyr Leu Asn Asp His Ser Glu Ala Thr Pro Asp Ser Asn
            500                 505                 510

Gly Ser Ala Leu Gly Asp Arg Phe Glu Gly Tyr Asp Asn Pro Glu Asn
        515                 520                 525

Leu Lys Ala Asn Ala Leu Tyr Thr His Ser Arg Leu Arg Ser Ser Leu
    530                 535                 540

Val Phe Lys Pro Thr Leu Pro Glu Gln Lys Glu Val Asn Ser Cys Thr
545                 550                 555                 560
```

Thr Gly Ser Ser Asn Ser Thr Ile Ile Gly Ser Gln Gly Ser Glu Thr
565 570 575

Pro Lys Glu Val Pro Asp Thr Pro Thr Asn Val Gln His Leu Thr Asp
580 585 590

Lys Pro Leu Pro Glu Ser Ile Pro Lys Leu Pro Leu Gln Ser Glu Ala
595 600 605

Pro Lys Met His Thr Leu Gln Val Pro Glu Asn His Ser Val Ala Leu
610 615 620

Asn Gln Thr Thr Asn Gly His Thr Glu Ser Asn Asn Tyr Ile Tyr Lys
625 630 635 640

Thr Leu Gly Val Asn Lys Gln Thr Glu Asn Leu Lys Asn Gln Gln Thr
645 650 655

Glu Asn Leu Leu Lys Arg Arg Ser Phe Pro Leu Phe Asp Asn Ser Lys
660 665 670

Ala Asn Leu Asp Pro Gly Asn Ser Lys His Tyr Val Tyr Ser Thr Leu
675 680 685

Thr Arg Asn Arg Val Arg Gln Pro Glu Lys Pro Lys Glu Asp Leu Leu
690 695 700

Lys Ser Ser Lys Ser Met His Asn Val Thr His Asn Leu Glu Glu Asp
705 710 715 720

Glu Glu Glu Val Thr Lys Arg Asn Ser Pro Ser Gly Thr Thr Thr Lys
725 730 735

Ser Val Ser Ile Ala Ala Leu Leu Asp Val Asn Lys Glu Glu Ser Asn
740 745 750

Lys Glu Leu Ala Ser Lys Lys Glu Val Lys Gly Ser Pro Ser Phe Leu
755 760 765

Lys Lys Gly Ser Gln Lys Leu Arg Ser Leu Leu Ser Leu Thr Pro Asp
770 775 780

Lys Lys Glu Asn Leu Ser Lys Asn Lys Ala Pro Ala Phe Tyr Arg Leu
785 790 795 800

Cys Ser Ser Ser Asp Thr Leu Val Ser Glu Gly Glu Glu Asn Gln Lys

```
                805                 810                 815

Pro Lys Lys Ser Asp Thr Lys Val Asp Ser Ser Pro Arg Arg Lys His
            820                 825                 830

Ser Ser Ser Ser Asn Ser Gln Gly Ser Ile His Lys Ser Lys Glu Asp
        835                 840                 845

Val Thr Val Ser Pro Ser Gln Glu Ile Asn Ala Pro Pro Asp Glu Asn
    850                 855                 860

Lys Arg Thr Pro Ser Pro Gly Pro Val Glu Ser Lys Phe Leu Glu Arg
865                 870                 875                 880

Ala Gly Asp Ala Ser Ala Pro Arg Phe Asn Thr Glu Gln Ile Gln Tyr
                885                 890                 895

Arg Asp Ser Arg Glu Ile Asn Ala Val Val Thr Pro Glu Arg Arg Pro
            900                 905                 910

Thr Ser Ser Pro Arg Pro Thr Ser Ser Glu Leu Leu Arg Ser His Ser
        915                 920                 925

Thr Asp Arg Arg Val Tyr Ser Arg Phe Glu Pro Phe Cys Lys Ile Glu
    930                 935                 940

Ser Ser Ile Gln Pro Thr Ser Asn Met Pro Asn Thr Ser Ile Asn Arg
945                 950                 955                 960

Pro Glu Ile Lys Ser Ala Thr Met Gly Asn Ser Tyr Gly Arg Ser Ser
                965                 970                 975

Pro Leu Leu Asn Tyr Asn Thr Gly Val Tyr Arg Ser Tyr Gln Pro Asn
            980                 985                 990

Glu Asn Lys Phe Arg Gly Phe Met  Gln Lys Phe Gly Asn  Phe Ile His
        995                 1000                1005

Lys Asn  Lys
    1010
```

<210> 3
<211> 9
<212> **PRT**
<213> **Artificial** Sequence

<220>
<223> synthetic peptide

<400> 3

Ser Tyr Gln Pro Asn Glu Asn Lys Phe
1 5

<210> 4
<211> 10
<212> **PRT**
<213> **Artificial** Sequence

<220>
<223> synthetic peptide

<400> 4

His Tyr Lys Glu Trp Tyr Arg Val Ala Ile
1 5 10

<210> 5
<211> 10
<212> **PRT**
<213> **Artificial** Sequence

<220>
<223> synthetic peptide

<400> 5

Trp Tyr Arg Val Ala Ile Asp Ile Leu Ile
1 5 10

<210> 6
<211> 10
<212> **PRT**
<213> **Artificial** Sequence

<220>
<223> synthetic peptide

<400> 6

Gly Trp Pro Tyr Val Met Pro Gly Leu Leu
1 5 10

<210> 7
<211> 10
<212> **PRT**
<213> **Artificial** Sequence

<220>
<223> synthetic peptide

<400> 7

Leu Phe His Pro Pro Arg Ala His Leu Leu
1 5 10

<210> 8

<211> 10
<212> **PRT**
<213> **Artificial** Sequence

<220>
<223> synthetic peptide

<400> 8

```
Lys Met Ile Lys Glu Ala Arg Lys Val Ile
1               5                   10
```

<210> 9
<211> 10
<212> **PRT**
<213> **Artificial** Sequence

<220>
<223> synthetic peptide

<400> 9

```
Val Tyr Ile Leu Leu Asp Glu Ser Asn Phe
1               5                   10
```

<210> 10
<211> 10
<212> **PRT**
<213> **Artificial** Sequence

<220>
<223> synthetic peptide

<400> 10

```
Asp Tyr Leu Ser Lys Thr Gly Ala Lys Phe
1               5                   10
```

<210> 11
<211> 10
<212> **PRT**
<213> **Artificial** Sequence

<220>
<223> synthetic peptide

<400> 11

```
Lys Phe His Gly Lys Met Glu Gln Lys Phe
1               5                   10
```

<210> 12
<211> 11
<212> **PRT**
<213> **Artificial** Sequence

<220>
<223> synthetic peptide

<400> 12

Ser Tyr Met Trp Ser Phe Glu Lys Ala His Leu
1 5 10

<210> 13
<211> 9
<212> PRT
<213> Artificial Sequence

<220>
<223> synthetic peptide

<400> 13

Ser Phe Asp Glu Glu Phe Arg Thr Leu
1 5

<210> 14
<211> 11
<212> **PRT**
<213> **Artificial** Sequence

<220>
<223> synthetic peptide

<400> 14

Leu Tyr Ala Arg Ser Cys Val Pro Ser Ser Phe
1 5 10

<210> 15
<211> 9
<212> **PRT**
<213> **Artificial** Sequence

<220>
<223> synthetic peptide

<400> 15

Thr Tyr Gln His Ser Val Ser Ser Leu
1 5

<210> 16
<211> 11
<212> **PRT**
<213> **Artificial** Sequence

<220>
<223> synthetic peptide

<400> 16

Ser Tyr Phe Lys Asn Arg Gly Ile Tyr Thr Leu
1 5 10

<210> 17

<211> 10
<212> PRT
<213> Artificial Sequence

<220>
<223> synthetic peptide

<400> 17

Gly Tyr Lys Pro His Phe Val Pro Asn Phe
1 5 10

<210> 18
<211> 9
<212> PRT
<213> Artificial Sequence

<220>
<223> synthetic peptide

<400> 18

Pro Tyr Leu Leu Leu Asn Arg Ala Leu
1 5

<210> 19
<211> 11
<212> PRT
<213> Artificial Sequence

<220>
<223> synthetic peptide

<400> 19

Ser Phe Asn Gly Thr Asp Asn His Ile Arg Phe
1 5 10

<210> 20
<211> 10
<212> PRT
<213> Artificial Sequence

<220>
<223> synthetic peptide

<400> 20

Arg Phe Leu Gln Gln Arg Met Pro Thr Leu
1 5 10

<210> 21
<211> 9
<212> PRT
<213> Artificial Sequence

<220>
<223> synthetic peptide

<400> 21

Leu Phe Asp Asn Ser Lys Ala Asn Leu
1 5

<210> 22
<211> 10
<212> PRT
<213> Artificial Sequence

<220>
<223> synthetic peptide

<400> 22

Ser Phe Leu Lys Lys Gly Ser Gln Lys Leu
1 5 10

<210> 23
<211> 9
<212> PRT
<213> Artificial Sequence

<220>
<223> synthetic peptide

<400> 23

Pro Phe Cys Lys Ile Glu Ser Ser Ile
1 5

<210> 24
<211> 9
<212> PRT
<213> Artificial Sequence

<220>
<223> synthetic peptide

<400> 24

Ser Tyr Gly Arg Ser Ser Pro Leu Leu
1 5

<210> 25
<211> 9
<212> PRT
<213> Artificial Sequence

<220>
<223> synthetic peptide

<400> 25

Lys Phe Arg Gly Phe Met Gln Lys Phe
1 5

<210> 26

<211> 10
<212> PRT
<213> Artificial Sequence

<220>
<223> synthetic peptide

<400> 26

```
Gly Phe Met Gln Lys Phe Gly Asn Phe Ile
1               5                   10
```

<210> 27
<211> 10
<212> PRT
<213> Artificial Sequence

<220>
<223> synthetic peptide

<400> 27

```
Gly Leu Glu Ala Tyr Gln Glu Phe Leu Val
1               5                   10
```

<210> 28
<211> 11
<212> PRT
<213> Artificial Sequence

<220>
<223> synthetic peptide

<400> 28

```
Phe Leu Val Gln Glu Arg Val Ser Asp Phe Leu
1               5                   10
```

<210> 29
<211> 11
<212> PRT
<213> Artificial Sequence

<220>
<223> synthetic peptide

<400> 29

```
Phe Leu Ala Glu Glu Glu Ile Asn Tyr Ile Leu
1               5                   10
```

<210> 30
<211> 9
<212> PRT
<213> Artificial Sequence

<220>
<223> synthetic peptide

<400> 30

Tyr Ile Leu Lys Asn Val Gln Lys Val
1 5

<210> 31
<211> 10
<212> PRT
<213> Artificial Sequence

<220>
<223> synthetic peptide

<400> 31

Thr Leu Ser Ser Gly Thr Tyr Trp Pro Val
1 5 10

<210> 32
<211> 9
<212> PRT
<213> Artificial Sequence

<220>
<223> synthetic peptide

<400> 32

Asn Leu Asp Leu Gly Trp Pro Tyr Val
1 5

<210> 33
<211> 11
<212> PRT
<213> Artificial Sequence

<220>
<223> synthetic peptide

<400> 33

Gly Leu Leu Gly Gly Thr His Ile Asp Leu Leu
1 5 10

<210> 34
<211> 11
<212> PRT
<213> Artificial Sequence

<220>
<223> synthetic peptide

<400> 34

Leu Leu Phe His Pro Pro Arg Ala His Leu Leu
1 5 10

<210> 35

<211> 9
<212> PRT
<213> Artificial Sequence

<220>
<223> synthetic peptide

<400> 35

His Leu Leu Thr Ile Lys Glu Thr Ile
1 5

<210> 36
<211> 9
<212> PRT
<213> Artificial Sequence

<220>
<223> synthetic peptide

<400> 36

Lys Met Ile Lys Glu Ala Arg Lys Val
1 5

<210> 37
<211> 10
<212> PRT
<213> Artificial Sequence

<220>
<223> synthetic peptide

<400> 37

Ala Leu Val Met Asp Ile Phe Thr Asp Val
1 5 10

<210> 38
<211> 11
<212> PRT
<213> Artificial Sequence

<220>
<223> synthetic peptide

<400> 38

Leu Leu Asp Glu Ser Asn Phe Asn His Phe Leu
1 5 10

<210> 39
<211> 10
<212> PRT
<213> Artificial Sequence

<220>
<223> synthetic peptide

<400> 39

Asn Met Thr Glu Lys Gln Gly Cys Ser Val
1 5 10

<210> 40
<211> 10
<212> PRT
<213> Artificial Sequence

<220>
<223> synthetic peptide

<400> 40

Arg Leu Arg Asn Ile Arg Val Arg Thr Val
1 5 10

<210> 41
<211> 9
<212> PRT
<213> Artificial Sequence

<220>
<223> synthetic peptide

<400> 41

Lys Met Glu Gln Lys Phe Leu Leu Val
1 5

<210> 42
<211> 10
<212> PRT
<213> Artificial Sequence

<220>
<223> synthetic peptide

<400> 42

Tyr Met Trp Ser Phe Glu Lys Ala His Leu
1 5 10

<210> 43
<211> 9
<212> PRT
<213> Artificial Sequence

<220>
<223> synthetic peptide

<400> 43

Asn Leu Phe Gly Arg Gln Asp Lys Ile
1 5

<210> 44

<211> 10
<212> PRT
<213> Artificial Sequence

<220>
<223> synthetic peptide

<400> 44

```
Thr Leu Asn Glu His Asp Lys Tyr Asn Ile
1               5                   10
```

<210> 45
<211> 10
<212> PRT
<213> Artificial Sequence

<220>
<223> synthetic peptide

<400> 45

```
Arg Leu Ala Gln Arg Lys Thr Thr Asn Leu
1               5                   10
```

<210> 46
<211> 9
<212> PRT
<213> Artificial Sequence

<220>
<223> synthetic peptide

<400> 46

```
Asn Leu Ala Asp Arg Asn Ser Asn Val
1               5
```

<210> 47
<211> 9
<212> PRT
<213> Artificial Sequence

<220>
<223> synthetic peptide

<400> 47

```
Phe Leu Gln Gln Arg Met Pro Thr Leu
1               5
```

<210> 48
<211> 11
<212> PRT
<213> Artificial Sequence

<220>
<223> synthetic peptide

<400> 48

Phe Leu Arg Asn Trp Arg Ile Glu Ser Tyr Leu
1 5 10

<210> 49
<211> 9
<212> PRT
<213> Artificial Sequence

<220>
<223> synthetic peptide

<400> 49

Pro Leu Pro Glu Ser Ile Pro Lys Leu
1 5

<210> 50
<211> 10
<212> PRT
<213> Artificial Sequence

<220>
<223> synthetic peptide

<400> 50

Thr Leu Gln Val Pro Glu Asn His Ser Val
1 5 10

<210> 51
<211> 10
<212> PRT
<213> Artificial Sequence

<220>
<223> synthetic peptide

<400> 51

Asn Leu Leu Lys Arg Arg Ser Phe Pro Leu
1 5 10

<210> 52
<211> 10
<212> PRT
<213> Artificial Sequence

<220>
<223> synthetic peptide

<400> 52

Pro Leu Phe Asp Asn Ser Lys Ala Asn Leu
1 5 10

<210> 53

<211> 9
<212> PRT
<213> Artificial Sequence

<220>
<223> synthetic peptide

<400> 53

```
Ser Met His Asn Val Thr His Asn Leu
1               5
```

<210> 54
<211> 9
<212> PRT
<213> Artificial Sequence

<220>
<223> synthetic peptide

<400> 54

```
Asn Leu Glu Glu Asp Glu Glu Glu Val
1               5
```

<210> 55
<211> 9
<212> PRT
<213> Artificial Sequence

<220>
<223> synthetic peptide

<400> 55

```
Phe Leu Lys Lys Gly Ser Gln Lys Leu
1               5
```

<210> 56
<211> 10
<212> PRT
<213> Artificial Sequence

<220>
<223> synthetic peptide

<400> 56

```
Ser Leu Thr Pro Asp Lys Lys Glu Asn Leu
1               5                   10
```

<210> 57
<211> 10
<212> PRT
<213> Artificial Sequence

<220>
<223> synthetic peptide

<400> 57

Arg Leu Cys Ser Ser Ser Asp Thr Leu Val
1               5                   10

<210> 58
<211> 9
<212> PRT
<213> Artificial Sequence

<220>
<223> synthetic peptide

<400> 58

Phe Met Gln Lys Phe Gly Asn Phe Ile
1               5

<210> 59
<211> 10
<212> PRT
<213> Artificial Sequence

<220>
<223> synthetic peptide

<400> 59

Lys Val Met Tyr Gly Ser Tyr Ser Tyr Met
1               5                   10

<210> 60
<211> 11
<212> PRT
<213> Artificial Sequence

<220>
<223> synthetic peptide

<400> 60

Ser Met Val Gln Ile Ile Thr Gly Gln Leu Val
1               5                   10

<210> 61
<211> 10
<212> PRT
<213> Artificial Sequence

<220>
<223> synthetic peptide

<400> 61

Glu Leu Ala Gly Ile Gly Ile Leu Thr Val
1               5                   10

<210> 62

<211> 9
<212> PRT
<213> Artificial Sequence

<220>
<223> synthetic peptide

<400> 62

Arg Tyr Leu Arg Asp Gln Gln Leu Leu
1 5

<210> 63
<211> 9
<212> PRT
<213> Artificial Sequence

<220>
<223> synthetic peptide

<400> 63

Glu Ala Asp Pro Thr Gly His Ser Tyr
1 5

<210> 64
<211> 8
<212> PRT
<213> Artificial Sequence

<220>
<223> synthetic peptide

<400> 64

Arg Gly Tyr Val Tyr Gln Gly Leu
1 5

<210> 65
<211> 10
<212> PRT
<213> Artificial Sequence

<220>
<223> synthetic peptide

<400> 65

Asn Tyr Ile Leu Lys Asn Val Gln Lys Val
1 5 10

<210> 66
<211> 9
<212> PRT
<213> Artificial Sequence

<220>
<223> synthetic peptide

<400> 66

Lys Val Ile Ala Leu Val Met Asp Ile
1 5

<210> 67
<211> 12
<212> PRT
<213> Artificial Sequence

<220>
<223> synthetic peptide

<400> 67

Val Met Tyr Gly Ser Tyr Ser Tyr Met Trp Ser Phe
1 5 10

<210> 68
<211> 12
<212> PRT
<213> Artificial Sequence

<220>
<223> synthetic peptide

<400> 68

Thr Tyr Gln His Ser Val Ser Ser Leu Ala Ser Val
1 5 10

<210> 69
<211> 11
<212> PRT
<213> Artificial Sequence

<220>
<223> synthetic peptide

<400> 69

His Gly Leu Glu Ala Tyr Gln Glu Phe Leu Val
1 5 10

<210> 70
<211> 10
<212> PRT
<213> Artificial Sequence

<220>
<223> synthetic peptide

<400> 70

Lys Pro Leu Pro Glu Ser Ile Pro Lys Leu
1 5 10

<210> 71

<211> 10
<212> PRT
<213> Artificial Sequence

<220>
<223> synthetic peptide

<400> 71

Phe Leu Ala Glu Glu Glu Ile Asn Tyr Ile
1               5                   10

<210> 72
<211> 10
<212> PRT
<213> Artificial Sequence

<220>
<223> synthetic peptide

<400> 72

Phe Leu Leu Val Asp Cys Gln Lys Val Met
1               5                   10

<210> 73
<211> 10
<212> PRT
<213> Artificial Sequence

<220>
<223> synthetic peptide

<400> 73

Met Val Gln Ile Ile Thr Gly Gln Leu Val
1               5                   10

<210> 74
<211> 10
<212> PRT
<213> Artificial Sequence

<220>
<223> synthetic peptide

<400> 74

Asn Tyr Ile Glu Pro His Tyr Lys Glu Trp
1               5                   10

<210> 75
<211> 10
<212> PRT
<213> Artificial Sequence

<220>
<223> synthetic peptide

<400> 75

```
Leu Val Gln Glu Arg Val Ser Asp Phe Leu
1               5                   10
```

<210> 76
<211> 10
<212> PRT
<213> Artificial Sequence

<220>
<223> synthetic peptide

<400> 76

```
Lys Val Ile Ala Leu Val Met Asp Ile Phe
1               5                   10
```

<210> 77
<211> 10
<212> PRT
<213> Artificial Sequence

<220>
<223> synthetic peptide

<400> 77

```
Val Ile Ala Leu Val Met Asp Ile Phe Thr
1               5                   10
```

<210> 78
<211> 11
<212> PRT
<213> Artificial Sequence

<220>
<223> synthetic peptide

<400> 78

```
Val Met Asp Ile Phe Thr Asp Val Asp Ile Phe
1               5                   10
```

<210> 79
<211> 13
<212> PRT
<213> Artificial Sequence

<220>
<223> synthetic peptide

<400> 79

```
Lys Val Met Tyr Gly Ser Tyr Ser Tyr Met Trp Ser Phe
1               5                   10
```

<210> 80

<211> 10
<212> PRT
<213> Artificial Sequence

<220>
<223> synthetic peptide

<400> 80

```
Val Met Tyr Gly Ser Tyr Ser Tyr Met Trp
1               5                   10
```

<210> 81
<211> 10
<212> PRT
<213> Artificial Sequence

<220>
<223> synthetic peptide

<400> 81

```
Leu Leu Phe His Pro Pro Arg Ala His Leu
1               5                   10
```

<210> 82
<211> 10
<212> PRT
<213> Artificial Sequence

<220>
<223> synthetic peptide

<400> 82

```
Lys Ser Met His Asn Val Thr His Asn Leu
1               5                   10
```

<210> 83
<211> 9
<212> PRT
<213> Artificial Sequence

<220>
<223> synthetic peptide

<400> 83

```
Lys Ser Val Ser Ile Ala Ala Leu Leu
1               5
```

<210> 84
<211> 9
<212> PRT
<213> Artificial Sequence

<220>
<223> synthetic peptide

<400> 84

Arg Val Tyr Ser Arg Phe Glu Pro Phe
1 5

<210> 85
<211> 10
<212> PRT
<213> Artificial Sequence

<220>
<223> synthetic peptide

<400> 85

Asn Tyr Asn Thr Gly Val Tyr Arg Ser Tyr
1 5 10